(19) Europäisches Patentamt
European Patent Office
Office européen des brevets

(11) Veröffentlichungsnummer: **0 451 790 A1**

# (12) EUROPÄISCHE PATENTANMELDUNG

(21) Anmeldenummer: **91105614.1**

(22) Anmeldetag: **09.04.91**

(51) Int. Cl.[5]: **C07D 261/08**, C07D 261/18, C07D 261/04, C07F 9/30, C07F 9/32, C07F 9/38, C07F 9/40, A61K 31/42

(30) Priorität: **12.04.90 DE 4011880**

(43) Veröffentlichungstag der Anmeldung:
**16.10.91 Patentblatt 91/42**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Schwab, Wilfried, Dr.**
**Auf den Erlen 1d**
**W-6200 Wiesbaden(DE)**
Erfinder: **Anagnostopulos, Hiristo**
**Forststrasse 40a**
**W-6200 Wiesbaden(DE)**
Erfinder: **Porsche-Wiebking, Elena, Dr.**
**Adolfallee 16**
**W-6200 Wiesbaden(DE)**
Erfinder: **Grome, John, Dr.**
**Fritz-Reuter-Strasse 1**
**W-6200 Wiesbaden(DE)**

(54) **3,5-disubstituierte 2-Isoxazoline und Isoxazole, Verfahren zu deren Herstellung, diese enthaltende Mittel und ihre Verwendung.**

(57) 3,5-disubstituierte 2-Isoxazoline und Isoxazole, Verfahren zu deren Herstellung, diese enthaltende Mittel und ihre Verwendung

Es werden neue 3,5-disubstituierte 2-Isoxazoline und Isoxazole sowie neue Arzneimittel, die sich insbesondere zur Prophylaxe und/oder Behandlung von pathologischen, neurodegenerativen Erkrankungen bei Mensch und Tier eignen, beschrieben. Ferner werden Verfahren zur Herstellung dieser 3,5-disubstituierten 2-Isoxazoline und Isoxazole gezeigt.

EP 0 451 790 A1

3,5-disubstituierte 2-Isoxazoline und Isoxazole, Verfahren zu deren Herstellung, diese enthaltende Mittel und ihre Verwendung

Die vorliegende Erfindung betrifft neue 3,5-disubstituierte 2-Isoxazoline und Isoxazole sowie neue Arzneimittel, die sich insbesondere zur Prophylaxe und/oder Behandlung von pathologischen neurodegenerativen Erkrankungen bei Mensch und Tier eignen.

Aus der deutschen Offenlegungsschrift 37 36 113 (HOE 87/F 316) sind phosphorhaltige 3,5-disubstituierte 2-Isoxazoline und Isoxazole bekannt. Muscimol sowie deren Analoga sind z.B. in J. Med. Chem. 27 (1984), 585-591 und J. Med. Chem. 28 (1985), 668-679 beschrieben.

Eine große Zahl neuropathologischer Situationen sind durch eine Degeneration sowie den Verlust von Neuronen charakterisiert. Dies gilt insbesondere für neurodegenerative Krankheitsbilder, wie Schlaganfall, temporäre Hirnischämie (TIA), zerebraler Infarkt mit nur teilweise reversiblen Symptomen (PRIND), Gehirnlähmung, zerebrale Hypoglycämie, ischämische Ereignisse während Herzstillstand oder chirurgischer Eingriffe im Herz-Lungen-Bereich, anoxische Zustände, zum Beispiel nach Ertrinken, Intoxikation oder Rückenmarksverletzungen, perinatale Asphyxie, altersbedingte neurodegenerative Veränderungen, Alzheimer-Demenz (SDAT), Schmerz, Übersekretion von Wachstumshormon und Luteinisierungshormon, Schizophrenie, Epilepsie, Huntington-Chorea sowie andere chronische neurodegenerative Erkrankungen.

Weiterhin ist bekannt, daß es im Säugetiergehirn zahlreiche excitatorische synaptische Rezeptoren gibt, die von natürlich vorkommendem L-Glutamin und L-Asparaginsäure aktiviert werden. Diese Aminosäuren werden zur Beendigung eines Neuronenreizes durch ein hoch affines Transportsystem in die präsynaptischen Vesikel aufgenommen. Dieser Transportmechanismus ist $Na^+$-abhängig. Notwendigerweise ist die Aufnahme der Aminosäuren L-Gln und L-Asp in die präsynaptischen Vesikel zur Erhaltung der intrazellulären $Na^+$-Konzentration unter anderem auch von ATP abhängig (Erecinska, Biochem. Pharmacol. 36 (1987), 3547-3555). Eine Ansammlung exzitatorischer Aminosäuren in dem synaptischen Spalt, wie z.B. nach hypoxischen oder ischämischen Zuständen, kann zu andauernden Nervenimpulsen, zu pathologischen Veränderungen und letztlich zum irreversiben Untergang der Neuronen führen. Aus der Literatur gibt es Hinweise, daß Präparate, die direkt die Aminosäureaufnahme in den synaptischen Vesikeln verbessern, die Freisetzung reduzieren, oder indirekt den Rücktransport begünstigen, sowie auch Substanzen, die die Rezeptoraktivierung antagonisieren, neuroprotektiv wirken (Gill et al., Neuroscience, 25 (1988) 847-855; Jarvis et al., Synapse, 2 (1988) 577-584; Kaneko et al., Arzneimittel-Forschung, 39 (1989) 445-450; Silverstein et al., J. Neurochem., 47 (1986) 1614-1619; Weiss et al., Brain Res., 380 (1986) 186-190).
Auch andere Aminosäuren wie z.B. N-Methyl-D-Asparaginsäure (NMDA), Kain-, und Quisqualinsäure sind als potente exzitatorische Neurotransmitter im Zentralnervensystem bekannt. Sie wurden für die Charakterisierung der Glutamatrezeptor-Subtypen verwendet. Diese exzitatorischen Rezeptoren, lokalisiert auf den sogenannten glutamatergen Neuronen, sind in zwei Gruppen klassifiziert: NMDA- und nicht-NMDA-Rezeptoren. Nach dem heutigen Kenntnisstand sind die NMDA-Rezeptoren nach ischämischen Ereignissen, wie zum Beispiel Schlaganfall maßgeblich an dem Neuronenuntergang beteiligt (Rothman et al., TINS, 10 (1987) 299-302).

Der Erfindung liegt daher die Aufgabe zugrunde, neue, potent "Glutamat-Antagonisten" zu finden, die, frei von unerwünschten Nebenwirkungen, den durch Hypoxie und/oder Ischämie bedingten Zelluntergang reduzieren können.

Klinische Erfolge mit zerebroprotektiv wirksamen NMDA-Rezeptorantagonisten sind jedoch wegen unerwünschter Nebenwirkungen, wie zum Beispiel der teilweise ausgeprägten motorischen Dyskoordination, psychotischer Effekte, sowie sonstiger, zum Teil toxischer Nebenwirkungen weitgehend ausgeblieben (Handelmann et al., Eur. J. Pharmacol., 140 (1987) 69-73; Morris et al., Nature, 319 (1986) 774-776). Zur Zeit ist keine bekannte Substanz auf dem Markt, die klinisch getestet ist und die bei guter Verträglichkeit die Wirkung bei neurodegenerativen Erkrankungen bewiesen hat. Somit besteht ein dringendes Bedürfnis nach neuen, chemisch definierten und gut verträglichen zerebroprotektiv wirksamen Substanzen, die zur Prophylaxe und/oder Nachbehandlung der oben geschilderten pathologischen Zustände, insbesondere des Schlaganfalls, geeignet sind.

Überraschend wurde nun gefunden, daß durch Einführen bestimmter, sich insbesondere von Alkylcarbonsäuren ableitenden Gruppen in die 3- oder 5-Position, sowie gegebenenfalls substituierter basischer Gruppen in die 5- oder 3-Position von 2-Isoxazolinen und Isoxazolen Verbindungen erhalten werden, die aufgrund ihrer biochemischen und pharmakologischen Eigenschaften die oben aufgeführten Anforderungen erfüllen, und sich demnach zur Prophylaxe und/oder Therapie von Erkrankungen eignen, die mit zerebralen neurodegenerativen Veränderungen einhergehen. Die Verbindungen besitzen gegenüber literaturbekannten Substanzen, wie zum Beispiel Muscimol [5-(Aminomethyl)-3(2H)-isoxazolon], MK-801 [(+)-5-Methyl-10,11-dihydro-5H-dibenzo[a,d]cyclohepten-5-10-imin] und CPP [3-(+)-2-Carboxypiperazin-4-yl-propyl-1-phosphonsäure] (Lust et al., Metabolic Brain Disease, 3 (1988) 287-292, Rod et al., Can. J. Neurol. Sci., 16

(1989) 340-344., Meldrum et al., "Pharmacology of Cerebral Ischaemia" ed. J. Krieglstein, CRC Press, 1989, 157-163) hervorragende Verträglichkeit, sowie starke Wirkung in biochemischen "in vitro"- und pathologischen Tiermodellen, die sich am Beispiel der

Stimulation der hoch affinen Aufnahme von 3H-Aspartat in synaptischen Vesikelpräparationen aus Rattenhirn, Hemmung der Freisetzung von 3H-Acetylcholin aus Striatum-Schnitten von Ratten,

Hemmung der 3H-MK801-Bindung an Membranen aus Rattengehirn, Inhibierung der NMDA-induzierten Krämpfe an der Maus, neuroprotektiven Wirkung nach bilateraler Okklusion der Arteria Carotis am Mongolischen Gerbil, und

Reduktion des Infarktvolumens nach photochemisch induzierter fokaler Ischämie an der Ratte belegen läßt.

Gegenstand dieser Erfindung sind somit neue 3,5-disubstituierte 2-Isoxazoline und Isoxazole der allgemeinen Formeln Ia, Ib und Ic

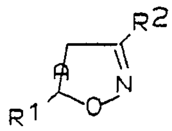 (Ia) 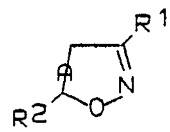 (Ib) 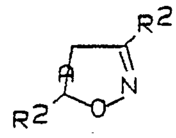 (Ic)

in welcher

$R^1$ für 2-, 3-, 4-Pyridyl oder einen Rest der Formel II

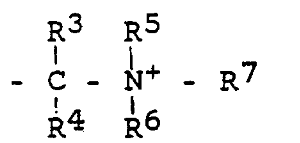 (II)

steht, wobei

$R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_6$-Alkyl; $C_3$-$C_6$-Cycloalkyl; $C_6$-$C_{12}$-Aryl-$C_1$-$C_4$-Alkyl; Carbamimidoyl; $C_1$-$C_6$-Alkylcarbonyl, $C_1$-$C_4$-Alkenylcarbonyl, $C_1$-$C_6$-Alkyloxycarbonyl, $C_6$-$C_{12}$-Aryl-$C_1$-$C_4$-Alkylcarbonyl, $C_6$-$C_{10}$-Aryl-$C_1$-$C_4$-Alkyloxycarbonyl, $C_6$-$C_{10}$-Arylcarbonyl, den Rest einer natürlich vorkommenden $\alpha$-Aminosäure oder $\gamma$-Aminobuttersäure (Gaba), die durch $C_1$-$C_6$-Alkyl, Hydroxy, Halogen, Amino oder Nitro substituiert sein können; oder

$R^6$ und $R^7$ zusammen mit dem sie verknüpfenden Stickstoffatom einen fünf- bis siebengliedrigen Heterozyklus bilden, wobei ein Kohlenstoffatom durch ein Schwefel-, Sauerstoff- oder Stickstoffatom ersetzt sein kann, bedeuten; oder

$R^5$, $R^6$, $R^7$ unabhängig voneinander $C_1$-$C_4$-Alkyl oder $C_3$-$C_6$-Cycloalkyl bedeuten, oder $R^5$ $C_1$-$C_4$-Alkyl bedeutet und $R^6$ und $R^7$ zusammen mit dem sie verknüpfenden Stickstoffatom einen fünf- bis siebengliedrigen Heterozyklus bilden, oder $R^5$, $R^6$ und $R^7$ zusammen mit dem sie verknüpfenden Stickstoffatom einen sechs-bis zwölfgliedrigen bicyclischen Heterozyklus bilden;

$R^2$ für einen Rest der Formel III

$-(CH_2)_n-X$ (III)

steht, wobei

n 0 oder eine ganze Zahl von 1 bis 4 bedeutet;

X für Hydroxy; $C_1$-$C_4$-Alkyloxy; Carboxy; Halogenformyl; Formyl; Oxyimino; $C_1$-$C_{12}$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden $\alpha$-Aminosäure, $\gamma$-Aminobuttersäure oder einem Dipeptid peptidisch verknüpft ist, oder Aminocarbonyl bedeutet, wobei Amino durch $C_1$-$C_6$-Alkyl mono- oder disubstituiert oder durch Phenyl-$C_1$-$C_6$-Alkyl monosubstituiert sein kann, oder beide

Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann;
oder X für eine Gruppe der Formel IV

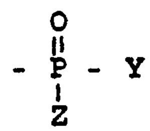 (IV)

steht, wobei

Y und Z unabhängig voneinander Hydroxy, $C_1$-$C_4$-Alkyl oder $C_1$-$C_4$-Alkyloxy bedeuten; und

A für eine C,C-Einfach- oder eine C,C-Doppelbindung steht;

mit der Maßgabe, daß $R^1$ nicht 2-, 3- oder 4-Pyridyl bedeutet, falls $R^2$ für eine Gruppe der Formel IV steht und $n \neq 0$ ist, falls X für Hydroxy oder Methyloxy steht, sowie ihre gegebenenfalls stereoisomeren Formen und ihre physiologisch verträgliche- Salze

Falls im Einzelfall nicht anders angegeben, kann Alkyl geradkettig oder verzweigt sein. Entsprechendes gilt für davon abgeleitete Reste wie Alkyloxy, Alkylcarbonyl, Alkyloxycarbonyl oder Arylalkyl.

$C_6$-$C_{12}$-Aryl bedeutet vorzugsweise Phenyl, Naphthyl oder Biphenyl, insbesondere Phenyl. Entsprechend sind davon abgeleitete Reste zu formulieren, wie Arylcarbonyl oder Arylalkyl.

Halogen steht für Fluor, Chlor, Brom oder Jod, vorzugsweise für Chlor.

Fünf- bis siebengliedrige Heterozyklen im Sinne der vorliegenden Erfindung sind beispielsweise Pyrrol, Pyridin, Azepin, Thiazol, Isothiazol, Oxazol, Isoxazol, Pyrazol, Imidazol, Thiazin, 1,2-Oxazin, 1,3-Oxazin, Morpholin, Pyridazin, Pyrimidin, Pyrazin, 1,2-Thiazepin, 1,3-Thiazepin, 1,4-Thiazepin, 1,2-Oxazepin, 1,3-Oxazepin, 1,4-Oxazepin, 1,2-Diazepin, 1,3-Diazepin, 1,4-Diazepin sowie deren teilweise oder ganz gesättigten Varianten. Insbesondere seien genannt Pyrrolidin, Piperidin, Morpholin, Piperazin oder Pyridin.

Natürlich vorkommende α-Aminosäuren, wie beispielsweise Ala, Arg, Cys, Gly, His, Ile, Leu, Lys, Met, Phe, Pro, Ser, Thr, Trp, Tyr, Val, Asp, Asn, Glu und Gln sind z.B. in Ann. Rev. Biochem. 38 (1969) 137-158 und FEBS Letters 64 (1976) 29-35 beschrieben. Dipeptide im Sinne der vorliegenden Erfindung enthalten als Bausteine natürlich vorkommende α-Aminosäuren sowie γ-Aminobuttersäure.

Bevorzugt sind Verbindungen der Formel Ia und Ib worin $R^1$ für 2-, 3-, 4-Pyridyl oder für einen Rest der Formel II

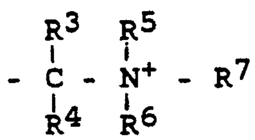 (II)

steht, wobei $R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_4$-Alkyl; Phenyl-$C_1$-$C_2$-Alkyl;

$R^6$ und $R^7$ zusammen mit dem sie verknüpfenden Stickstoffatom einen fünf- bis siebengliedrigen Heterozyklus bilden, wobei ein Kohlenstoffatom durch ein Schwefel-, Sauerstoff-oder Stickstoffatom ersetzt sein kann, bedeuten;

$R^6$ Wasserstoff und $R^7$ Carbamimidoyl; $C_1$-$C_6$-Alkylcarbonyl, $C_1$-$C_4$-Alkenylcarbonyl, $C_1$-$C_6$-Alkyloxycarbonyl, Phenyl-$C_1$-$C_4$-Alkylcarbonyl, Benzyloxycarbonyl, Benzoyl, den Rest einer natürlich vorkommenden α-Aminosäure oder γ-Aminobuttersäure, die durch $C_1$-$C_4$-Alkyl, Hydroxy, Halogen, Amino oder Nitro substituiert sein können;

$R^5$, $R^6$, $R^7$ unabhängig voneinander $C_1$-$C_4$-Alkyl oder $C_3$-$C_6$-Cycloalkyl bedeuten;

$R^2$ für einen Rest der Formel III

$-(CH_2)_n-X$ (III)

steht wobei

n 0 oder eine ganze Zahl von 1 bis 3 beduetet;
X für Hydroxy; $C_1$-$C_4$-Alkyloxy; Carboxy; $C_1$-$C_4$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl steht, das mit einer natürlich vorkommenden $\alpha$-Aminosäure, $\gamma$-Aminobuttersäure oder einem Dipeptid peptidisch verknüpft ist; Aminocarbonyl bedeutet, wobei Amino durch $C_1$-$C_4$-Alkyl mono- oder disubstituiert oder durch Phenyl-$C_1$-$C_4$-Alkyl monosubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann;
oder X für eine Gruppe der Formel IV

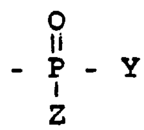

(IV)

steht, wobei
Y und Z unabhängig voneinander Hydroxy, $C_1$-$C_4$-Alkyl oder $C_1$-$C_4$-Alkyloxy bedeuten; und
A für eine C,C-Einfach- oder eine C,C-Doppelbindung steht;
mit der Maßgabe, daß $R^1$ nicht 2-, 3-, oder 4- Pyridyl bedeutet, falls $R^2$ für eine Gruppe der Formel IV steht.

Insbesondere seien Verbindungen der Formel Ia genannt, worin $R^1$ für 2-Pyridyl oder für einen Rest der Formel II

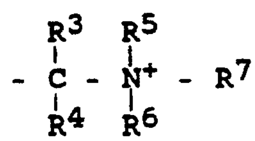

(II)

steht, wobei $R^3$ und $R^4$ unabhängig voneinander Wasserstoff oder $C_1$-$C_4$-Alkyl;
$R^5$ ein freies Elektronenpaar oder Wasserstoff;
$R^6$ und $R^7$ unabhängig voneinander Wasserstoff; $C_1$-$C_4$-Alkyl; Phenyl-$C_1$-$C_2$-Alkyl;
$R^6$ und $R^7$ zusammen mit dem sie verknüpfenden Stickstoffatom einen fünf- bis siebengliedrigen gesättigten Heterozyklus bilden, wobei ein Kohlenstoffatom durch ein Schwefel-, Sauerstoff-oder Stickstoffatom ersetzt sein kann, bedeuten;
$R^6$ Wasserstoff und $R^7$ Carbamimidoyl, $C_1$-$C_6$-Alkylcarbonyl oder den Rest einer natürlich vorkommenden $\alpha$-Aminosäure oder $\gamma$-Aminobuttersäure bedeuten;
$R^5$, $R^6$, $R^7$ unabhängig voneinander $C_1$-$C_4$-Alkyl bedeuten;
$R^2$ für einen Rest der Formel III

$$-(CH_2)_n-X \quad (III)$$

steht wobei
n 0, 1 oder 2 bedeutet;
X für Hydroxy; $C_1$-$C_4$-Alkyloxy; Carboxy; Halogenformyl; $C_1$-$C_4$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl steht, das mit einer natürlich vorkommenden $\alpha$-Aminosäure oder $\gamma$-Aminobuttersäure peptidisch verknüpft ist; Aminocarbonyl bedeutet, wobei Amino durch $C_1$-$C_4$-Alkyl mono- oder disubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann;
oder X für eine Gruppe der Formel IV

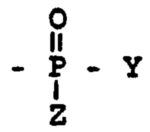 (IV)

steht, wobei

Y und Z unabhängig voneinander Hydroxy, $C_1$-$C_4$-Alkyl oder $C_1$-$C_4$-Alkyloxy bedeuten; und

A für eine C,C-Einfach- oder eine C,C-Doppelbindung steht;

mit der Maßgabe, daß $R^1$ nicht 2-Pyridyl bedeutet, falls $R^2$ für eine Gruppe der Formel IV steht und A ≠ einer C,C-Doppelbindung ist, falls X = OH und n = 0 sind; sowie Verbindungen der Formel Ia worin

$R^1$ für 2-Pyridyl oder für einen Rest der Formel II

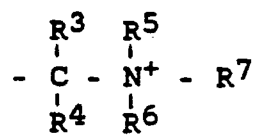 (II)

steht, wobei

$R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_4$-Alkyl; Benzyl;

$R^6$ und $R^7$ zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff- oder Stickstoffatom ersetzt sein kann;

$R^6$ Wasserstoff und $R^7$ Carbamimidoyl; $C_1$-$C_6$-Alkylcarbonyl, den Rest einer natürlich vorkommenden $\alpha$-Aminosäure;

$R^5$, $R^6$, $R^7$ unabhängig voneinander $C_1$-$C_4$-Alkyl bedeuten;

$R^2$ für einen Rest der Formel III

$-(CH_2)_n-X$ (III)

steht, wobei

n 0, 1 oder 2 bedeutet;

X für Hydroxy; Carboxy; $C_1$-$C_4$-Alkyloxycarbonyl; Benzyloxycarbonyl; Cyclohexyloxycarbonyl, das ein-oder mehrfach durch $C_1$-$C_6$-Alkyl substituiert sein kann; Carbonyl, das mit einer natürlich vorkommenden $\alpha$-Aminosäure peptidisch verknüpft ist; Aminocarbonyl, wobei beide Amino-Reste zusammen mit dem sie verknüpfenden Stickstoffatom einen fünf- bis sechsgliedrigen Heterozyklus bilden, wobei ein Kohlenstoffatom durch ein Sauerstoff- oder Stickstoffatom ersetzt sein kann;
oder für eine Gruppe der Formel IV

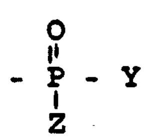 (IV)

steht, wobei

Y Hydroxy oder $C_1$-$C_4$-Alkyloxy und

Z $C_1$-$C_4$-Alkyl bedeuten; und

A für eine C,C-Einfach- oder eine C,C-Doppelbindung steht;

mit der Maßgabe, daß $R^1$ nicht 2-Pyridyl bedeutet, falls

$R^2$ für eine Gruppe der Formel IV steht und A ≠ einer C,C-Doppelbindung ist, falls X = OH und n = 0 sind.

Unter den Verbindungen der Formel Ib seien insbesondere solche genannt worin

$R^1$ für einen Rest der Formel II

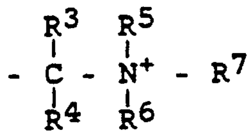

(II)

steht, wobei

$R^3$ und $R^4$ Wasserstoff;

$R^5$ ein freies Elektronenpaar oder Wasserstoff;

$R^6$ und $R^7$ unabhängig voneinander Wasserstoff; $C_1$-$C_4$-Alkyl; Phenyl-$C_1$-$C_2$-Alkyl;

$R^6$ und $R^7$ zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff- oder Stickstoffatom ersetzt sein kann;

$R^6$ Wasserstoff und $R^7$ $C_1$-$C_4$-Acyl; $C_1$-$C_6$-Alkylcarbonyl, Benzoyl oder den Rest einer natürlich vorkommenden $\alpha$-Aminosäure oder $\gamma$-Aminobuttersäure, bedeuten;

$R^5$, $R^6$, $R^7$ unabhängig voneinander $C_1$-$C_4$-Alkyl oder $C_3$-$C_6$-Cycloalkyl bedeuten;

$R^2$ für einen Rest der Formel III

$-(CH_2)_n-X$ (III)

steht, wobei

n 0, 1 oder 2 bedeutet;

X für Carboxy; Halogenformyl; $C_1$-$C_4$-Alkyloxycarbonyl; Benzyloxycarbonyl oder $c_3$-$C_6$-Cycloalkylcarbonyl, die ein- oder mehrfach durch $C_1$-$C_6$-Alkyl substituiert sein können; oder für Carbonyl steht, das mit einer natürlich vorkommenden $\alpha$-Aminosäure oder $\gamma$-Aminobuttersäure peptidisch verknüpft ist; Aminocarbonyl, bedeutet, wobei Amino durch $C_1$-$C_4$-Alkyl mono- oder disubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann; und

A für eine C,C-Einfach- oder eine C,C-Doppelbindung steht, sowie Verbindungen der Formel Ib, worin

$R^1$ für einen Rest der Formel II

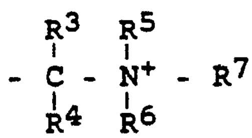

(II)

steht, wobei

$R^3$ und $R^4$ Wasserstoff;

$R^5$ ein freies Elektronenpaar;

$R^6$ und $R^7$ unabhängig voneinander Wasserstoff oder $C_1$-$C_4$-Alkyl;

$R^6$ und $R^7$ zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff- oder Stickstoffatom ersetzt sein kann;

$R^6$ Wasserstoff und $R^7$ $C_1$-$C_6$-Alkylcarbonyl bedeuten;

$R^5$, $R^6$, $R^7$ unabhängig voneinander $C_1$-$C_4$-Alkyl bedeuten;

$R^2$ für einen Rest der Formel III

$-(CH_2)_n-X$ (III)

steht, wobei

n 2 bedeutet und

X für Carboxy, $C_1$-$C_4$-Alkyloxycarbonyl; Benzyloxycarbonyl; Cyclohexylcarbonyl, das ein-

oder mehrfach durch $C_1$-$C_6$-Alkyl substituiert sein kann; Carbonyl, das mit einer natürlich vorkommenden $\alpha$-Aminosäure peptidisch verknüpft ist; Aminocarbonyl, wobei beide Amino-Reste zusammen mit dem sie verknüpfenden Stickstoffatom einen fünf- bis sechsgliedrigen Heterozyklus bilden, wobei ein Kohlenstoffatom durch ein Sauerstoff- oder Stickstoffatom ersetzt sein kann; und

A für eine C,C-Einfach- oder eine C,C-Doppelbindung steht.

Allgemein bevorzugt sind Verbindungen der Formel Ia, worin A für eine C,C-Doppelbindung steht.

Ferner seien insbesondere genannt:
5-Aminomethyl-isoxazol-3-propionsäure-benzylester-Hydrochlorid,
5-Aminomethyl-isoxazol-3-propionsäure-ethylester-Hydrochlorid,
5-Aminomethyl-isoxazol-3-propionsäure-(+)-menthylester-Toluol-4-sulfonat,
5-Aminomethyl-isoxazol-3-propionsäure-(-)-menthylester-Toluol-4-sulfonat,
5-Aminomethyl-isoxazol-3-propionsäure-cis-(3,3,5)-trimethylcyclohexylester-Toluol-4-sulfonat,
2-(5-Aminomethyl-isoxazol-3-yl)-ethyl-2-(P-methyl)-phosphinsäure-ethylester-Hydrochlorid,
5-Aminomethyl-isoxazol-3-propionsäure-methylester-Hydrochlorid,
5-Aminomethyl-isoxazol-3-propionsäure,
3-Carboxy-2-isoxazolin-5-yl-carbonsäure-(-)-menthylester-Dicyclohexylammoniumsalz.
3,5-Dicarboxy-2-isoxazolin,
5-Hydroxymethyl-isoxazol-3-propionsäure-Natriumsalz,
5-Trimethylammonio-methyl-isoxazol-3-propionsäure-cis-(3,3,5)-trimethylcyclohexylester-iodid 3-Hydroxyiminomethyl-isoxazol-5-propionsäure-methylester,
5-(1-Amino-1-methyl-ethyl)-isoxazol-3-propionsäure,
5-Benzylaminomethyl-isoxazol-3-propionsäure,
5-Dimethylaminomethyl-isoxazol-3-propionsäure,
2-(5-Aminomethyl-isoxazol-3-yl)-ethyl-2-(P-methyl)-phosphinsäure-Hydrochlorid,
5-Acetamidomethyl-isoxazol-3-propionsäure,
5-Trimethylammoniomethyl-isoxazol-3-propionsäure-(-)-menthylester-Jodid,
5-Trimethylammoniomethyl-isoxazol-3-propionsäure-(+)-menthylester-Jodid,
5-(L-Phenylalanyl-amino)-methyl-isoxazol-3-propionsäure-(+)-menthylester-Hydrochlorid,
5-(L-Phenylalanyl-amino)-methyl-isoxazol-3-propionsäure-(-)-menthylester-Hydrochlorid,
5-(L-Phenylalanyl-amino)-methyl-isoxazol-3-propionsäuremethylester-Hydrochlorid,
5-Guanidinomethyl-isoxazol-3-propionsäure,
N-(5-Aminomethyl-isoxazol-3-yl)-propionyl-glycin,
Bis-(3-[2-carboxy]-ethyl-isoxazol-5-yl-methyl)-amin-Diammoniumsalz,
5-Aminomethyl-2-isoxazolin-3-propionsäure-(+)-menthylester-Toluol-4-sulfonat,
5-Aminomethyl-2-isoxazolin-3-propionsäure-(-)-menthylester-Toluol-4-sulfonat,
3-(2-Carboxyethyl)-5-(2-pyridyl)-2-isoxazolin,
5-Trimethylammoniomethyl-isoxazol-3-propionsäureester,
5-Piperidinomethyl-isoxazol-3-propionsäure-Hydrochlorid,
5-Aminomethyl-isoxazol-3-propionsäureamid-Hydrochlorid,
5-(L-Phenylalanyl-amino)-methyl-isoxazol-3-yl-propionylglycin-Trifluoracetat.

Gegenstand der Erfindung sind weiterhin Verfahren zur Herstellung von Verbindungen der Formel Ia, Ib oder Ic, ihrer gegebenenfalls stereoisomeren Form und gegebenenfalls ihrer stereoisomeren Salze, die dadurch gekennzeichnet sind, daß man ein Nitriloxid der Formel V

$$\bar{O}-\overset{+}{N}\equiv C-R^2 \qquad (V)$$

a) für den Fall, daß A in Formel Ia eine C,C-Einfachbindung ist, mit einem Olefin der Formel VI, oder

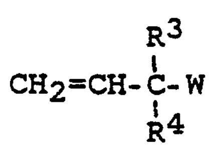

(VI)

b) für den Fall, daß A in Formel Ia eine C,C-Doppelbindung ist, mit einem Propargylderivat der Formel

VII im Sinne einer 1.3-dipolaren Cycloaddition umsetzt,

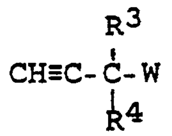

(VII)

wobei $R^2$, $R^3$ und $R^4$ die vorgenannten Bedeutungen haben und W eine Gruppe der Formel $NR^5R^6R^7$ mit den vorgenannten Bedeutungen für $R^5$, $R^6$ und $R^7$, oder ein durch ein gegebenenfalls substituiertes Amin der Formel $NR^5R^6R^7$ austauschbaren Substituenten, wie zum Beispiel Alkylsulfonyl oder Arylsulfonyl oder Halogen, hier vorzugsweise Chlor oder Brom darstellt, oder

c) eine nach a) oder b) hergestellte Verbindung der allgemeinen Formel VIII

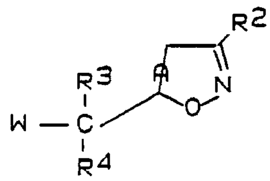

(VIII)

mit den oben genannten Bedeutungen für $R^2$, $R^3$, $R^4$ und A durch Substitution der Gruppe W mit einem gegebenenfalls substituierten Amin der Formel $NR^5R^6R^7$ in eine Verbindung der allgemeinen Formel Ia überführt, oder

d) zur Darstellung einer Substanz der allgemeinen Formel Ib oder Ic ein Alken- oder Alkinsäurederivat der allgemeinen Formel IX, X, XII oder XIII

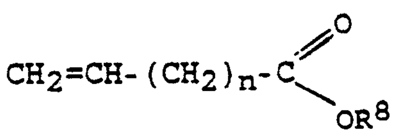

(IX)

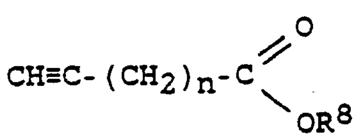

(X)

$CH_2=CH-(CH_2)_n-O-R^{10}$ (XII)

$CH\equiv C-(CH_2)_n-O-R^{10}$ (XIII)

wobei $R^{10}$ Wasserstoff, eine Alkylgruppe, eine beliebige Schutzgruppe für eine Alkoholfunktion oder eine veresterte Carboxygruppe darstellt, n die vorgenannte Bedeutung hat, und $R^8$ einen beliebigen Alkyl- oder Aralkylrest mit einem Nitriloxid der allgemeinen Formel XI darstellt,

$O \leftarrow N\equiv C-R^2$ (XI)

wobei $R^2$ die vorgenannte Bedeutung hat, mit der Maßgabe, daß $R^2$ nicht $\alpha$-Aminosäure oder ein Dipeptid sein kann im Sinne einer 1.3-dipolaren Cycloaddition umsetzt, gegebenenfalls die vorliegende Schutzgruppe $R^{10}$ nach literaturbekannten Verfahren entfernt, die Alkoholfunktion in ein zum Austausch mit Aminen aktiviertes Derivat wie z.B. Halogenid oder Tosylat überführt, und danach mit einem Amin der allgemeinen Formel $NR^5R^6R^7$ mit der vorgenannten Bedeutung für $R^5$-$R^7$ umsetzt, oder

e) einen nach a)-d) hergestellten Carbonsäureester der Formel Ia oder Ib zur Carbonsäure verseift beziehungsweise einen gegebebenfalls vorliegenden Benzylester hydrogenolytisch spaltet, oder

f) einen nach Verfahren a)-d) hergestellten Carbonsäurealkylester mit einem entsprechend substituierten primären oder sekundären Amin in das Amid überführt, oder

g) einen nach a) oder b) hergestellten Phosphinsäuremonoalkyl- oder Phosphonsäuredialkylester der allgemeinen Formel Ia nach an sich literaturbekannten Verfahren zum Phosphonsäurehalbester, zur Phosphonsäure oder zur Phosphinsäure verseift, oder

h) eine nach e) erhaltene Carbonsäure zunächst in ein aktiviertes Säurederivat überführt, dieses anschließend mit Alkoholen verestert, mit primären und sekundären Aminen in die Amide, oder mit gegebenenfalls carboxygeschützten Aminosäuren oder niederen Peptiden in am Stickstoff acylierte Peptide überführt, und anschließend gegebenenfalls die Carboxyschutzgruppe am Peptidteil entfernt oder zum Beispiel durch Umesterung in eine andere Gruppe umwandelt, oder

i) in einer nach Verfahren a)-h) erhaltenen Verbindung der allgemeinen Formel Ia einen gegebenenfalls als Schutzgruppe verwendeten Rest $R^7$ nach literaturbekannten Verfahren vom Stickstoffatom entfernt, oder

j) eine nach Verfahren i) hergestellte Verbindung, deren Carboxygruppe in veresterter Form oder als Amid vorliegt, durch Umsetzung mit einem aktivierten Carbonsäurederivat oder gegebenenfalls einer am Stickstoff geschützten Aminosäure oder einem niederen Peptid unter Verwendung von in der Peptidchemie üblichen Reagenzien in ein am Stickstoff acyliertes Derivat überführt, oder

k) eine nach Verfahren j) hergestellte Verbindung durch Spaltung des gegebenenfalls vorhandenen Carbonsäuresters in eine freie Carbonsäure überführt, oder

l) eine nach Verfahren j) oder k) hergestellte Verbindung durch Spaltung einer gegebenenfalls mit dem Aminosäureteil eingeführte N-Schutzgruppe in eine freie Aminoverbindung oder ein Betain überführt, oder

m) eine nach Verfahren c)-i) -hergestellte Verbindung der allgemeinen Formel Ia oder Ib durch Umsetzung mit einem Phosphinsäuremonoalkyl- oder Phosphonsäuredialkylester der Alkylierungsmittel, vorzugsweise einem Alkylhalogenid, in eine quartäre Ammoniumverbindung überführt, oder

n) eine nach Verfahren a)-m) hergestellte Verbindung der allgemeinen Formel Ia und Ib, die aufgrund ihrer chemischen Struktur in diastereoisomeren oder enantiomeren Formen auftritt, in an sich bekannter Weise in die reinen Stereoisomeren auftrennt, wobei man

o) die nach Verfahren a)-n) hergestellten Verbindungen der allgemeinen Formel Ia und Ib entweder in freier Form isoliert oder im Falle des Vorliegens von sauren oder basischen Gruppen gegebenenfalls in physiologisch verträgliche kristalline Salze umwandelt.

Die Herstellung physiologisch verträglicher Salze aus zur Salzbildung befähigten Verbindungen der Formeln Ia und Ib, einschließlich deren stereoisomeren Formen, erfolgt in an sich bekannter Weise. So bilden die Carbonsäuren, Phosphon- und Phosphinsäuren sowie die Phosphonsäurehalbester mit basischen Reagenzien, wie Hydroxiden, Carbonaten, Hydrogencarbonaten, Alkoholaten sowie Ammoniak oder organischen Basen, beispielsweise Trimethyl-oder Triethylamin, Ethanolamin oder auch basischen Aminosäuren, etwa Lysin, Ornithin oder Arginin stabile Alkali-, Erdalkali-, bzw. gegebenenfalls substituierte Ammoniumsalze, wobei im Falle der Phosphonsäuren durch Umwandlung nur einer der beiden sauren OH-Gruppen auch stabile Hydrogenphosphonate erhältlich sind. Sofern die Verbindungen der Formeln Ia und Ib basische Gruppen im Rest $R^1$ aufweisen, lassen sich mit starken Säuren auch stabile, nichttoxische Säureadditionssalze herstellen. Hierfür kommen sowohl anorganische als auch organische Säuren, wie Chlorwasserstoff-, Bromwasserstoff-, Schwefel-, Phosphor-, Methansulfon-, Benzolsulfon-, p-Toluolsulfon-, 4-Brombenzolsulfon-, Cyclohexylamidosulfon-, Trifluormethylsulfon-, Essig-, Oxal-, Wein-, oder Trifluoressigsäure in Frage.

Im Falle der durch Peralkylierung von Aminoverbindungen nach Verfahren m) entstandenen quartären Ammoniumsalze kommen als Gegenion bevorzugt die aus dem Alkylierungsmittel austretenden Anionen, wie zum Beispiel Alkyl- oder Arylsulfonat sowie Bromid und Iodid in Frage, wobei sich mittels geeigneter Ionenaustauscher diese auch durch andere physiologisch verträgliche Anionen ersetzen lassen.

Die Herstellung und Umsetzung der als Ausgangsstoffe für 1.3-dipolare Cycloadditionen verwendeten Nitriloxide ist in einer Monographie beschrieben (K.P.G. Torsell: Nitrile Oxides, Nitrones and Nitronates in Organic Synthesis, VCH Verlagsgesellschaft, Weinheim, 1988). Die als Vorstufen dienenden Hydroxamoylhalogenide sind nach literaturbekannten Verfahren durch Halogenierung entsprechender Aldoxime oder im Falle von Chloroxyiminoessigsäureestern über eine Diazotierungsreaktion ausgehend von Glycinalkylestern (G. S. Skinner, J. Am Chem. Soc. 46 (1924), 731 ff) erhältlich.

Im Falle des Formoxyiminonitriloxides wurde das nach Literatur (A. P. Kozikowski, J. Org. Chem. 48 (1983), 366) erhaltene Chlorglyoxim mit Triethylamin dehydrohalogeniert. Die ebenfalls verwendeten Nitroverbindungen sind teilweise literaturbekannt oder lassen sich nach im Prinzip literaturbekannten Methoden herstellen, so zum Beispiel die 4-Nitrobuttersäureester durch Fluorid- oder Basenkatalysierte Addition von Nitromethan an Acrylsäurederivate (S. Kanbe et al., Scient. Pap. Inst. phys. chem. Res. (Jap.) 58 (1964),

118 - 121; D. W. Chasar, Synthesis 1982, 841 - 42; N. Ono, Synthesis 1984, 226 - 227).

Durch Verwendung von 1,8-Diazabicylo[5.4.0]undec-7-en (DBU) als Base und eine spezielle Reaktionsführung, nämlich die Zugabe der Acrylsäureester zu einem katalytische Mengen DBU enthaltenden 5 bis 50-fachen Überschuß an Nitromethan in einem Temperaturbereich von etwa 60 bis 100 °C, bevorzugt etwa 70 bis 90 °C, lassen sich die literaturbekannten Ausbeuten wesentlich verbessern, sowie das Entstehen der normalerweise als schwer abtrennbaren Nebenprodukte auftretenden Bis-Addukte weitestgehend verhindern. Auch die mit Ausbeuten von ca. 30 % beschriebene Addition von Nitromethan an Vinylphosphonsäureester (T.A. Mastryukova, Izv. Akad. Nauk. SSSR, Ser. khim., 6 (1971), 1353 - 1354) läßt sich mit dieser Variante wesentlich verbessern und sogar auf die entsprechenden Alkyl-vinylphosphinsäureester übertragen, was einer bisher nicht literaturbekannten Methode entspricht.

Durch vorherige Umsetzung von Acrylchlorid mit entsprechend substituierten Alkoholen unter Verwendung eines Säurefängers, wie zum Beispiel Triethylamin, sind die hier ebenfalls verwendeten, teilweise nicht literaturbekannten Ester von höheren, zyklischen, sowie auch chiralen Alkoholen racemisierungsfrei zugänglich, die vorteilhaft bei den nachfolgenden Syntheseschritten mitgeführt werden können. Die weiterhin als Reaktionspartner dienenden, gegebenenfalls substituierten Allyl- und Propargylaminderivate der allgemeinen Formeln VI und VII sind als Grundkörper zumeist literaturbekannt oder sogar käuflich. Im Falle von primären oder sekundären Aminen werden vor der Cycloaddition literaturbekannte Schutzgruppen, bevorzugt Acyl- oder Urethanschutzgruppen, wie zum Beispiel Acetyl, Benzoyl, tert.-Butoxycarbonyl, tert.-Butyl oder Benzyloxycarbonyl eingeführt, die nach der Cycloaddition gegebenenfalls nach literaturbekannten Verfahren wieder entfernbar sind. Diese Schutzgruppen werden bevorzugt nach aus der Peptidchemie bekannten Methoden eingeführt, so zum Beispiel die N-tert.-Butoxycarbonylgruppe durch Umsetzung des entsprechenden Amins mit Di-tert.-butyldicarbonat in aliphatischen Ethern unter Verwendung eines Amines wie zum Beispiel Triethylamin.

Die Erzeugung der leicht zur Oligomerisierung neigenden Nitriloxide geschieht vorteilhaft "in situ" in Gegenwart der Verbindungen VI oder VII als Reaktionspartner ohne zwischenzeitliche Isolierung. Im Falle der Erzeugung aus Nitroverbindungen nach Mukaiyama werden zur Dehydratisierung bevorzugt aromatische Isocyanante, wie zum Beispiel Phenylisocyanat oder bevorzugt 1.4-Phenylendiisocyanat oder Toluol-2.4-diisocyanat eingesetzt. Dabei empfiehlt es sich, in einem gegenüber den Reaktionspartnern inerten aprotischen Lösungs- oder Verteilungsmittel zu arbeiten, wie zum Beispiel Ethylacetat, Dimethylformamid, Dimethylacetamid, Dialkylether, Tetrahydrofuran, halogenierten Kohlenwasserstoffen, beispielsweise Dichlormethan, Chloroform oder Dichlorethan, Kohlenwasserstoffen, wie Hexan, Cyclohexan, Benzol, Toluol oder anderen substituierten Aromaten, wobei auch Mischungen der vorgenannten Lösungsmittel in Frage kommen. Zur Erzeugung der Nitriloxide aus Hydroxamoylhalogeniden werden organische oder anorganische Basen, wie zum Beispiel tertiäre Amine, Alkalicarbonate oder -Hydroxide verwendet. Dabei arbeitet man unter Verwendung organischer Basen bevorzugt in den vorgenannten, gegebenenfalls chlorierten aliphatischen oder aromatischen Kohlenwasserstoffen oder aliphatischen, auch cyclischen Ethern, während bei Verwendung anorganischer Basen zusätzlich auch in zweiphasigen Lösungsmittelgemischen, wie zum Beispiel Ethylacetat/Wasser oder Dichlormethan/Wasser gearbeitet werden kann. Die Herstellung der Nitriloxide und die Cycloaddition erfolgen in der Regel bei Temperaturen zwischen etwa -20 °C und + 80 °C, vorzugsweise jedoch zwischen 0 °C und + 40 °C.

Die Reaktionspartner der Cycloaddition werden bevorzugt äquimolar eingesetzt, jedoch kann im Falle eines zur Oligomerisierung neigenden Nitriloxides das zum Abfangen benötigte Olefin auch in einem bis zu zehnfachen Überschuß eingesetzt werden. Auch die zur Freisetzung der Nitriloxide aus Hydroxamoylhalogeniden verwendete Base kann äquimolar oder in einem mehrfachen Überschuß eingesetzt werden. Um die Nitriloxide in niedriger Stationärkonzentration zu erzeugen, empfiehlt sich die Freisetzung durch Base oder Isocyanat kontinuierlich über einen längeren Zeitraum, der vorzugsweise 2 bis 24 Stunden beträgt. Dieses läßt sich vorteilhaft durch langsames Zutropfen eines der benötigten Reaktionspartner in eine die restlichen Reagenzien enthaltende Lösung durchführen.

Die Spaltung der im Substituenten $R^1$ der allgemeinen Formel Ia mitgeführten Acyl- oder Urethanschutzgruppen erfolgt meist nach aus der Peptidchemie bekannten Methoden, wobei im Falle der vorzugsweise verwendeten tert.-Butoxycarbonylgruppe eine saure Abspaltung, zum Beispiel mit alkoholischer Salzsäure oder Trifluoressigsäure vorzuziehen ist, während die Benzyloxycarbonylschutzgruppe sowohl hydrogenolytisch als auch durch alkalische Verseifung entfernbar ist. Die zur protonolytischen Schutzgruppenabspaltung verwendete Säure wird dabei vorteilhaft in einem größeren Überschuß eingesetzt, im Falle der Verwendung von Trifluoressigsäure empfiehlt sich zur Beschleunigung der Reaktion sogar die Verwendung der reinen Säure als Solvens oder ein Gemisch mit einem halogenierten Kohlenwasserstoff, wie zum Beispiel Dichlormethan. Bei Verwendung von Halogenwasserstoffsäuren wird bevorzugt eine alkoholische, vorzugsweise eine methanolische oder ethanolische Lösung derselben verwendet. Die saure Schutzgruppenspaltung läßt sich in einem Temperaturbereich von etwa 0 bis +80 °C, bevorzugt jedoch etwa 20 bis 40 °C durchführen. Für die hydrogenolytische Spaltung der Benzyloxycarbonyl-Schutzgruppe kommen die literaturbekannten Lösungsmittel, wie zum Beispiel niedere Alkohole oder Eisessig, vorzugsweise Methanol in Betracht. Als Katalysatoren werden bevorzugt Heterogenkatalysatoren, beispielsweise Palladium auf Kohle verwendet. Die Hydrierung kann bei Normaldruck oder unter Wasserstoff-Überdruck in einem Hydrierautoklaven bei Temperaturen von etwa 0 bis +80 °C, vorzugsweise bei Raumtemperatur durchgeführt werden. Die im Rest $R^2$ der Formeln Ia und Ib mitgeführten Estergruppen können sowohl sauer als auch alkalisch, sowie im Falle von Benzylestern auch hydrogenolytisch gespalten werden. Im Falle substituierter cyclischer Ester, wie zum Beispiel (+) und (-)-Menthylestern hat sich eine Mischung aus Trifluoressigsäure und Trifluormethansulfonsäure zusammen mit Thioanisol besonders bewährt (s. H. Yajima et al., Chem. Pharm. Bull. 34 (1986), 4356 - 4361). Im Falle der verwendeten tert.-Butylester lassen sich die oben zur Abspaltung der N-tert.-Butoxycarbonyl-Schutzgruppe beschriebenen Bedingungen ebenfalls vorteilhaft anwenden, so daß bei Verwendung entsprechender Bausteine die Schutzgruppen an beiden Substituenten des Heterozyklus simultan abgespalten werden können. Die ebenfalls verwendeten Alkylester werden bevorzugt der alkalischen Hydrolyse mit einem äquimolaren bis fünffachen Überschuß an Alkalihydroxid in wäßriger oder wäßrig-alkoholischer Lösung unterzogen. Im Falle der 3-Alkoxycarbonyl-isoxazoline und -isoxazole genügen aufgrund der aktivierten Estergruppe äquimolare Mengen an Alkalihydroxid und Temperaturen von etwa 0 bis +20 °C, während die über eine oder mehrere Methylengruppen an den Heterozyklus gebundenen Estergruppen drastischere Bedingungen in Form höherer Temperaturen und/oder größerer Überschüsse an Alkalihydroxid benötigen. Für die Spaltung der als Alkohol-Schutzgruppe bevorzugt verwendeten tert.-Butyl- oder Tetrahydropyranyl-Reste werden bevorzugt Säuren, wie zum Beispiel Trifluoressigsäure oder alkoholische, bevorzugt methanolische Salzsäure verwendet. Ein Überschuß an Säure oder im Falle von Trifluoressigsäure die Verwendung der reinen Säure als Lösungsmittel hat ebenfalls einen positiven Einfluß auf die Geschwindigkeit der Reaktion. Als Reaktionstemperatur kommt ein Bereich von etwa 0 bis +80 °C, vorzugsweise jedoch etwa 20 bis 40 °C in Frage. Die Umwandlung der Phosphonsäure- und Phosphinsäureester in die entsprechenden freien Säuren gelingt vorteilhaft unter sauren Bedingungen, bevorzugt in wasserfreiem Medium, so zum Beispiel durch Verwendung eines 2 bis 100-fachen Überschusses an Bromwasserstoffsäure in organischen Säuren, wie zum Beispiel Essigsäure in einer Konzentration von 0,5 bis 4-normal, bevorzugt 2- bis 4-normal, wobei als Reaktionstemperatur zur schonenden Spaltung bevorzugt ein Bereich von etwa +20 bis +50 °C gewählt wird. Zur Herstellung der Phosphonsäurehalbester aus Phosphonsäurediestern, sowie auch von Phosphinsäuren aus Phosphinsäureestern werden die entsprechenden Ester in der Regel einer alkalischen Hydrolyse, vorzugsweise in einem wäßrigen Milieu unterworfen. Dabei wird zum Auflösen des Diesters vorteilhaft ein mit Wasser mischbarer niederer Alkohol verwendet und danach eine 1- bis 5-normale wäßrige Base, beispielsweise Natriumhydroxid zugegeben. Die Base kann in stöchiometrischen Mengen oder in bis zu 10-fachem, vorzugsweise in etwa 2- bis 4-fachem Überschuß in einem Temperaturbereich von etwa 0 bis +50 °C, insbesondere von etwa 20 bis 40 °C verwendet werden.

Durch vorteilhafte Kombination der Ester für den Nitriloxidteil und Urethan- oder Acylschutzgruppen für den Aminteil kann man Heterozyklen der Formeln Ia und Ib herstellen, deren funktionelle Säure- beziehungsweise Basen-Gruppe durch Verwendung alkalischer, saurer, oder hydrogenolytischer Methoden, die teilweise oben beschrieben sind, gemeinsam oder getrennt freigesetzt werden können. Zur Substitution eines austauschbaren Substituenten W der allgemeinen Formel VIII, der hier vorzugsweise ein Halogenatom darstellt, wird mit einem sekundären, bevorzugt im etwa 2 bis 50-fachen Überschuß verwendeten Amin umgesetzt. Als Lösungsmittel kommen die oben für die Durchführung der Cycloaddition angegebenen, sowie im Falle von in flüssiger Form vorliegenden Aminen auch das Amin selbst in Betracht. Die Reaktionstemperatur kann von 0 °C bis zur Siedetemperatur des verwendeten Lösungsmittel betragen, vorzugsweise etwa 0 °C - +50 °C. Bei Verwendung flüchtiger oder gasförmiger Amine wird die Reaktion vorteilhaft in einem Autoklaven unter Überdruck durchgeführt. Beim Einsatz von Verbindungen der allgemeinen Formel VIII, die im Rest $R^2$ einen primären Ester, wie zum Beispiel Methyl- oder Ethylester enthalten, kann mit einem entsprechenden Überschuß an Amin und gegebenenfalls Erhöhung der Reaktionstemperatur auch die gleichzeitige Überführung der Ester- in die entsprechende Amidfunktion erreicht werden.

Verbindungen der Formel Ia mit einer freien Carbonsäurefunktion in $R^2$ können nach Aktivierung der Carboxygruppe mit primären und sekundären Aminen sowie auch mit carboxygeschützten Aminosäuren oder niederen Peptiden als Reaktionspartnern in die entsprechenden Amide überführt werden. Zur Aktivierung benutzt man vorteilhaft aus der Peptidchemie bekannte Methoden, wie zum Beispiel die Hydroxybenzotriazol/Dicyclohexylcarbodiimid-Methode (W. König, R. Geiger, Chem. Ber. 103 (1970), 788-798 und 2034-2040; Z. Naturforsch. 216 (1966) 426), die Aktivierung mittels Propanphosphonsäureanhydrid (PPA) (Angew. Chem. Int. Ed. 19 (1980) 133) oder mittels Methylethylphosphinsäureanhydrid (MEPA) (US-Patent 4,426,325), wobei als Lösungsmittel neben aliphatischen und zyklischen Ethern auch chlorierte Kohlenwasserstoffe, sowie Formamid und Dimethylformamid Verwendung finden können, als Hilfsbasen bevorzugt tertiäre Amine, wie zum Beispiel Triethylamin, N-Ethylmorpholin oder Pyridin verwendet werden, und im Temperaturbereich von etwa -10 °C bis +50 °C, vorzugsweise bei etwa 0 °C bis +20 °C gearbeitet wird. Verbindungen der Formel Ia mit freien Aminogruppen im Rest $R^1$ und vorzugsweise geschützter Carboxygruppe in $R^2$ können mit am Stickstoff geschützten Aminosäuren, niederen Peptiden und sonstigen zur Acylierung befähigten Carbonsäurederivaten auf prinzipiell gleiche Weise in die Amide beziehungsweise Peptide überführt werden. Dabei versteht sich von selbst, daß bei Anwendung einer entsprechend abgestuften, aus der Peptidchemie im Prinzip bekannten Schutzgruppentechnik sowohl in der $R^1$-, als auch in der $R^2$-Seitenkette der Verbindungen Ia beliebige weitere Aminosäuren ankondensiert werden können.

Sofern die Peptid- oder sonstigen Acylderivate in der Form freier Amino- oder Carbonsäurefunktionen gewünscht werden, lassen sich die entsprechenden Schutzgruppen einzeln oder auch gemeinsam auf den oben bereits beschriebenen Wegen entfernen. Dabei kann man zum Beispiel durch Verwendung von starken Säuren, wie zum Beispiel Chlor- oder Bromwasserstoffsäure in aliphatischen primären Alkoholen sowohl die gegebenenfalls vorhandenen N-Urethanschutzgruppen abspalten, als auch gleichzeitig in $R^2$ vorliegende Estergruppe, wie zum Beispiel einen Alkyl-, tert.-Butyl- oder Benzylester zu den entsprechenden Estern des verwendeten Alkohols umestern.

Zur Synthese von Verbindungen der allgemeinen Formel Ib kann, ausgehend von Penten- oder Pentinsäurederivaten sowie am Sauerstoff entsprechend geschützter Nitroethanolderivate im Prinzip die für Ia beschriebene 1.3-dipolare Cycloaddition nach Mukaiyama herangezogen werden. Nach Abspaltung der Sauerstoff-Schutzgruppe, wie oben beschrieben, wird die Hydroxyfunktion in ein aktiviertes Derivat überführt, was beispielsweise durch Umsetzung mittels Thionylchlorid oder Phosphoroxychlorid zum 3-Chlormethyl-isoxazol bzw. -isoxazolin gelingt. Das verwendete Reagenz wird dabei äquimolar oder bevorzugt in einem bis zu fünffachen Überschuß eingesetzt. Als Lösungsmittel kommen halogenierte Kohlenwasserstoffe oder bevorzugt aliphatische Äther, wie zum Beispiel Diethylether oder Tetrahydrofuran in Frage, die Reaktionstemperatur kann etwa 0 bis +60 °C, vorzugsweise jedoch etwa 20 bis 40 °C betragen.
Anschließend wird mit Ammoniak oder einem entsprechend substituierten Amin zum ggf. substituierten 3-Aminomethylderivat umgesetzt, was bevorzugt durch Verwendung eines 2 bis 100-fachen Überschusses an Amin in einem niederen Alkohol, zum Beispiel Methanol oder Ethanol in einem Temperaturbereich von 0 °C bis zum Siedepunkt des verwendeten Lösungsmittels geschieht. Um bei Verwendung von alkylestersubstituierten Heterozyklen gleichzeitige Amidbildung zu vermeiden haben sich Temperaturen von etwa 0 bis +20 °C und der Zusatz katalytischer Mengen Tetrabutylammoniumiodid bewährt. Sofern eine freie Carbonsäurefunktion im 5-Substituenten gewünscht wird, kann die mitgeführte Estergruppe nach den oben beschriebenen oder literaturbekannten Verfahren verseift werden. Sofern danach weitere Reaktionen am Carboxysubstituenten durchgeführt werden sollen, empfiehlt sich der vorherige Schutz einer primären oder sekundären Aminogruppe mit einer der voranstehend beschriebenen N-Schutzgruppen. Durch entsprechende Wahl der Schutzgruppen können so insgesamt die für Ia beschriebenen Variationen der Reste $R^1$ und $R^2$ ebenfalls für Verbindungen der allgemeinen Formel Ib angewendet werden.

Zur Überführung in quartäre Ammoniumderivate werden bevorzugt Verbindungen der allgemeinen Formel Ia oder Ib, die in $R^1$ eine primäre, sekundäre, oder tertiäre Aminogruppe tragen, mit einem Überschuß eines Alkylierungsmittels, beispielsweise eines Alkylhalogenides, eines Mesylates oder eines Tosylates, hier vorzugsweise eines Alkyliodides sowie einer Base, wie zum Beispiel Alkalicarbonat oder -hydroxid in dipolaren aprotischen Lösungsmitteln, wie zum Beispiel Aceton, Dimethylsulfoxid oder Dimethylformamid oder Gemischen niederer Alkohole, wie zum Beispiel Methanol oder Ethanol mit Wasser bei Temperaturen von etwa 0 °C bis +50 °C umgesetzt. Im Falle der bevorzugten Verwendung von Alkyliodiden kann das entstehende Tetraalkylammoniumiodid direkt seiner pharmakologischen Verwendung zugeführt werden.

Sofern die Verbindungen der allgemeinen Formeln Ia und Ib in diastereoisomeren oder enantiomeren Formen auftreten und bei der gewählten Synthese als deren Gemische anfallen, gelingt die Trennung in die reinen Stereoisomeren entweder durch Chromatographie an einem gegebenenfalls chiralen Trägermaterial, oder, sofern die racemischen Verbindungen der Formeln Ia und Ib zur Salzbildung befähigt sind, durch fraktionierte Kristallisation der mit einer optisch aktiven Base oder Säure als Hilfsstoff gebildeten diastereomeren Salze. Auch kann im Falle von Peptiden oder Estern chiraler Alkohole der Formel Ia die Chiralität des in enantiomerenreiner Form eingebrachten Aminosäure- oder Alkoholrestes zur Trennung der Diastereomeren genutzt werden. Als chirale Stationärphasen für die dünnschicht- oder säulenchromatographische Trennung von Enantiomeren der in der Regel racemisch anfallenden 2-Isoxazoline mit asymmetrischem C-Atom in 5-Position eignen sich zum Beispiel modifizierte Kieslgelträger (sogenannte Pirkle-Phasen) sowie

hochmolekulare Kohlenhydrate, wie zum Beispiel Triacetylcellulose. Für analytische Zweck sind nach entsprechender, dem Fachmann bekannter Derivatisierung, auch gaschromatographische Methoden an chiralen Stationärphasen anwendbar. Zur Enantiomerentrennung der racemischen Carbonsäuren, Phosphonsäuren, und Phosphinsäuren werden mit einer optisch aktiven, in der Regel kommerziell erhältlichen Base, wie zum Beispiel (-)-Nicotin, (+)- und (-)-Phenylethylamin, Chininbasen, L-Lysin oder L- und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren nach gängigen Verfahren gewonnen. Auf prinzipiell gleiche Weise kann man die racemischen Aminoverbindungen der Formeln Ia und Ib mit optisch aktiven Säuren, wie zum Beispiel (+)-Campher-10-sulfonsäure, gegebenenfalls hydroxysubstituierten D- und L-Weinsäuren, D- und L-Milchsäure sowie (+) und (-)-Mandelsäure in die reinen Enantiomeren überführen. Die Verwendung chiraler Ester der hierfür eingesetzten Aminoverbindungen begünstigt oft die Trennung der diastereomeren Salze durch fraktionierte Kristallisation.

Die erfindungsgemäßen Arzneimittel, die als Wirkstoffe die Verbindungen der allgemeinen Formeln Ia, Ib oder Ic

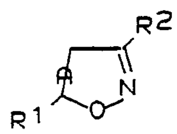 (Ia) 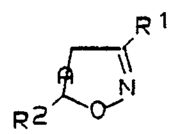 (Ib) 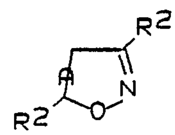 (Ic)

enthalten, worin

$R^1$ für 2-, 3-, 4-Pyridyl oder einen Rest der Formel II

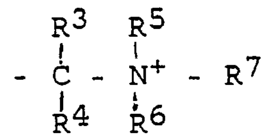 (II)

steht, wobei

$R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_6$-Alkyl; $C_3$-$C_6$-Cycloalkyl; $C_6$-$C_{12}$-Aryl-$C_1$-$C_4$-Alkyl; Carbamimidoyl; $C_1$-$C_6$-Alkylcarbonyl, $C_1$-$C_4$-Alkenylcarbonyl, $C_1$-$C_6$-Alkyloxycarbonyl, $C_6$-$C_{12}$-Aryl-$C_1$-$C_4$-Alkylcarbonyl, $C_6$-$C_{10}$-Aryl-$C_1$-$C_4$-Alkyloxycarbonyl, den Rest einer natürlich vorkommenden $\alpha$-Aminosäure oder $\gamma$-Aminobuttersäure, die durch $C_1$-$C_6$-Alkyl, Hydroxy, Halogen, Amino oder Nitro substituiert sein können; oder

$R^6$ und $R^7$ zusammen mit dem sie verknüpfenden Stickstoffatom einen fünf- bis siebengliedrigen Heterozyklus bilden, wobei ein Kohlenstoffatom durch ein Schwefel-, Sauerstoff- oder Stickstoffatom ersetzt sein kann, bedeuten; oder

$R^5$, $R^6$, $R^7$ unabhängig voneinander $C_1$-$C_4$-Alkyl oder $C_3$-$C_6$-Cycloalkyl bedeuten oder $R^6$ und $R^7$ zusammen mit dem sie verknüpfenden Stickstoffatom einen fünf- bis siebengliedrigen Heterozyklus bilden;

$R^2$ für einen Rest der Formel III

$-(CH_2)_n-X$ (III)

steht, wobei

n 0 oder eine ganze Zahl von 1 bis 4 bedeutet;

X für Hydroxy; $C_1$-$C_4$-Alkyloxy; Carboxy; Halogenformyl; Formyl; Oxyimino; $C_1$-$C_{12}$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden $\alpha$-Aminosäure, $\gamma$-Aminobuttersäure oder einem Dipeptid peptidisch verknüpft ist, oder Aminocarbonyl bedeutet, wobei Amino durch $C_1$-$C_6$-Alkyl mono- oder disubstituiert oder durch Phenyl-$C_1$-$C_6$-Alkyl monosubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann;
oder X für eine Gruppe der Formel IV

$$-\overset{\overset{\displaystyle O}{\|}}{\underset{\underset{\displaystyle Z}{|}}{P}}-Y \qquad (IV)$$

steht, wobei

Y und Z unabhängig voneinander Hydroxy, $C_1$-$C_4$-Alkyl oder $C_1$-$C_4$-Alkyloxy bedeuten; und
A für eine C,C-Einfach- oder eine C,C-Doppelbindung steht,

gegebenfalls in stereoisomerenreiner Form und/oder als physiologisch verträgliche Salze, entweder für sich allein, beispielweise in Mikrokapseln, in Mischungen untereinander oder vorzugsweise in Kombination mit geeigneten pharmazeutischen Trägerstoffen, Verdünnungsmitteln und/oder anderen Hilfstoffen, können parenteral, rektal oder oral verabreicht werden. Geeignete feste oder flüssige galenische Zubereitungsformen sind beispielsweise Granulate, Pulver, Dragees, Tabletten, (Mikro)kapseln, Suppositorien, Sirupe, Säfte, Suspensionen, Emulsionen, Tropfen oder injizierbare Lösungen sowie Präparate mit protrahierter Wirkstoffe-Freigabe, bei deren Herstellung üblicherweise Hilfsmittel, wie Trägerstoffe, Spreng-, Binde-, Überzugs-, Quellungs-, Gleit-, oder Schmiermittel, Geschmackstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, wie Lebertran, Sonnenblumen-, Erdnuß-, oder Sesamöl, Polyethylenglykole und Lösungsmittel, wie etwa steriles Wasser, physiologische Kochsalzlösung und ein- oder mehrwertige Alkohole, z.B. Glycerin gennant. Bei der Herstellung von wäßrigen Lösungen der stark sauer reagierenden Carbon-, Phosphon- und Phosphinsäuren gemäß Formeln Ia und Ib wird der Wirkstoff zweckmäßig so formuliert, daß er in Salzform mit physiologisch verträglichem pH-Wert vorliegt.
Vorzugsweise werden die pharmazeutischen Präparate in Dosierungseinheiten hergestellt und verabreicht, wobei jede Einheit als aktiven Bestandteil eine bestimmte Dosis mindestens einer Verbindung gemäß Formeln Ia und Ib, gegebenfalls in stereoisomerenreiner und/oder Salzform, enthält. Bei festen Dosierungseinheiten, wie Tabletten, Kapseln, Dragees, oder Suppositorien, kann diese Dosis bis zu etwa 1000 mg, bevorzugt jedoch etwa 50 bis 300 mg, und bei Injektionslösungen in Ampullenform bis zu etwa 300 mg, vorzugsweise aber etwa 10 bis 100 mg, betragen.
Für die Behandlung eines erwachsenen, etwa 70 kg schweren Patienten sind - je nach Wirksamkeit der Verbindungen gemäß Formeln Ia und Ib, bei Mensch und Tier Tagesdosen von etwa 50 bis 3000 mg Wirkstoff, vorzugsweise etwa 150 bis 1000 mg, bei oraler Verabreichung und von etwa 50 bis 1000 mg, bevorzugt etwa 100 bis 300 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen. Schließlich können die Verbindungen der Formeln Ia und Ib, ihre gegebenfalls stereoisomeren Formen und/oder gegebenfalls ihre physiologisch verträglichen Salze bei der Herstellung der vorgenannten galenischen Zubereitungsformen auch zusammen mit anderen geeigneten Wirkstoffen, beispielsweise durchblutungsfördernden Substanzen, Plättchenaggregationshemmer, Thrombozytenaggregationshemmern und Kalziumantagonisten kombiniert werden. **Beispiele**

Die folgenden Beispiele erläutern die Erfindung, ohne sie jedoch in ihrem Umfang einzuschränken.
Die Strukturen der nachfolgend beschriebenen Verbindungen wurden durch Elementaranalysen, IR-, $^1$H-NMR- und $^{13}$C-NMR-Spektren gesichert. Die $\delta$-Werte der nachfolgend aufgeführten NMR-Spektren sind in ppm, die Kopplungskonstanten J in Hz angegeben. Die Strukturformeln der nachfolgend beschriebenen Herstellungsbeispiele sind in Tabelle 1 zusammengestellt.

**Tabelle 1: Strukturformeln der Beispiele**

| Beisp.-Nr. | Formel | Beisp.Nr. | Formel |
|---|---|---|---|
| 1 | 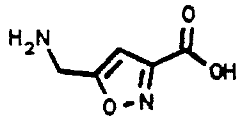 | 10 | 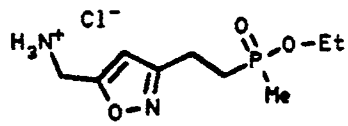 |
| 2 | 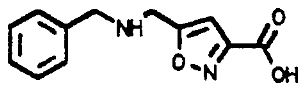 | 11 | 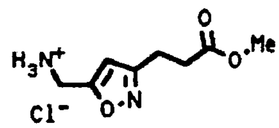 |
| 3 | 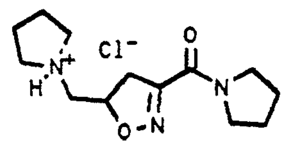 | 12 | 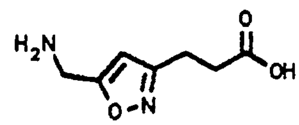 |
| 4 | 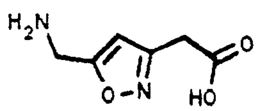 | 13 | 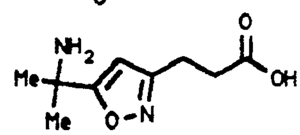 |
| 5 | 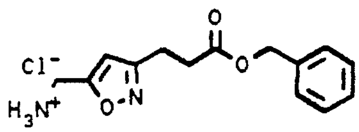 | 14 | 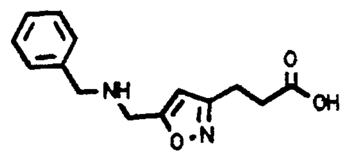 |
| 6 | 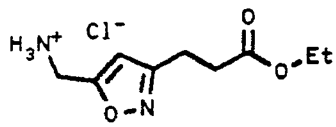 | 15 | 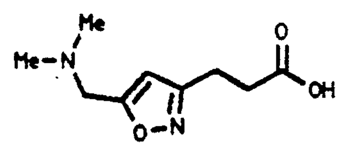 |
| 7 | 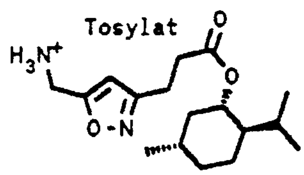 | 16 | 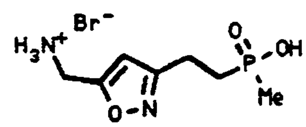 |
| 8 | 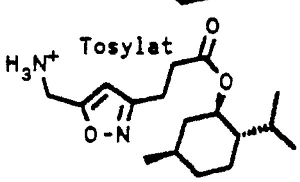 | 17 | 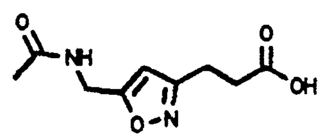 |
| 9 | 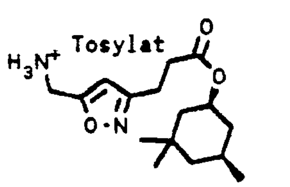 | 18 | 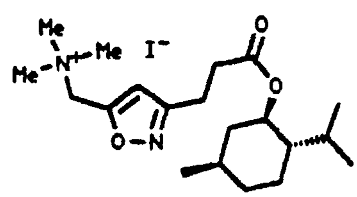 |

Tabelle 1 (Fortsetzung)

| Beisp.-Nr. | Formel | Beisp.Nr. | Formel |
|---|---|---|---|
| 19 | 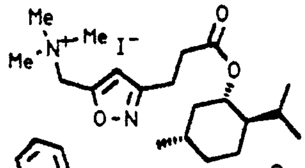 | 28 | Tosylat 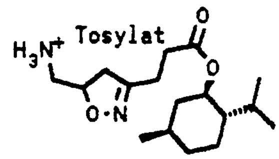 |
| 20 | 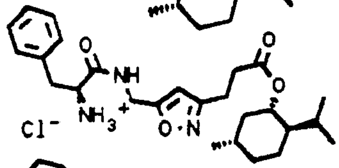 | 29 | 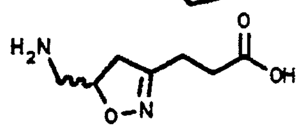 Racemat |
| 21 | 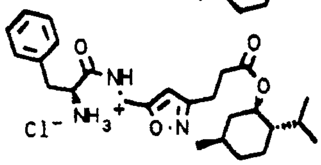 | 30 | 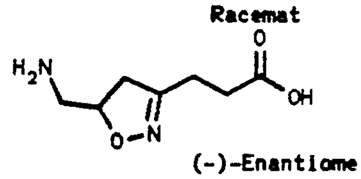 (-)-Enantiomer |
| 22 | 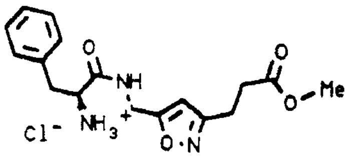 | 31 | (+)-Enantiomer |
| 23 | 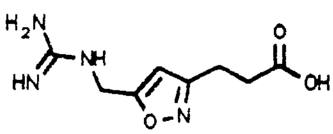 | 32 | 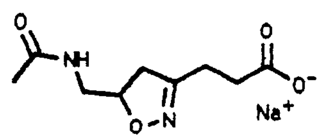 |
| 24 | 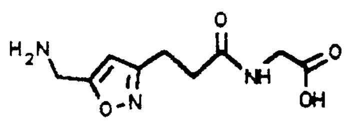 | 33 | 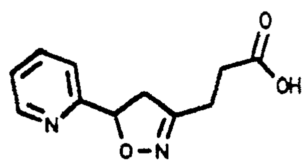 |
| 25 | 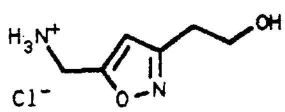 | 34 | Tosylat 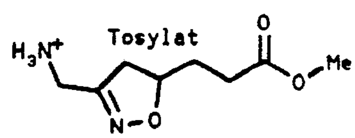 |
| 26 | 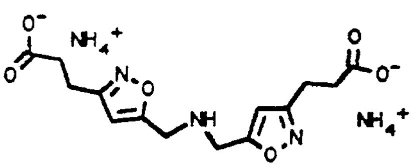 | 35 | 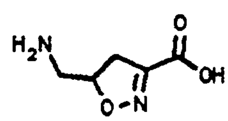 |
| 27 | Tosylat 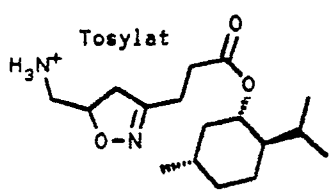 | 36 | 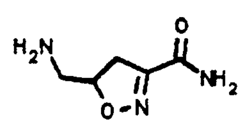 Trifluoracetat |

Tabelle 1 (Fortsetzung)

| Beisp.-Nr. | Formel | Beisp.Nr. | Formel |
|---|---|---|---|
| 37 | 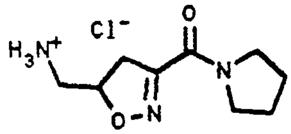 | 46 | 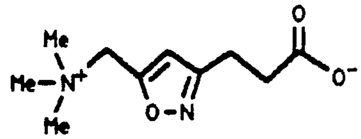 |
| 38 | 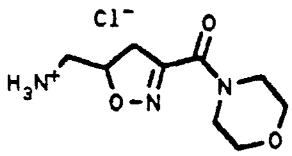 | 47 | 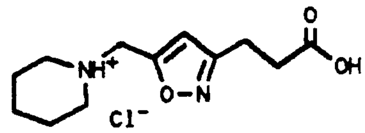 |
| 39 | 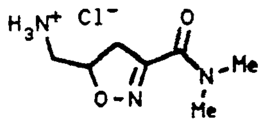 | 48 | 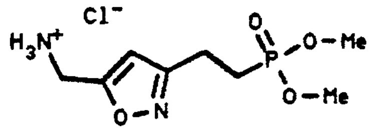 |
| 40 | 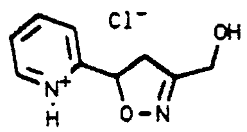 | 49 | 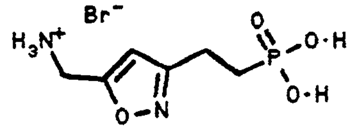 |
| 41 a<br>41 b | 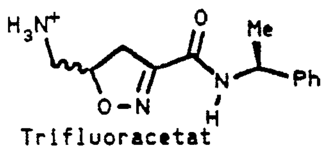<br>Trifluoracetat | 50 | 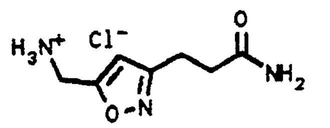 |
| 42 | 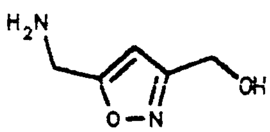 | 51 | 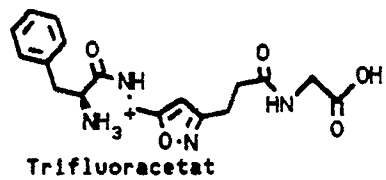<br>Trifluoracetat |
| 43 | 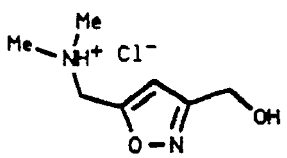 | 52 | 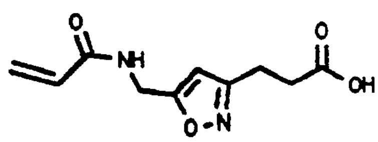 |
| 44 | 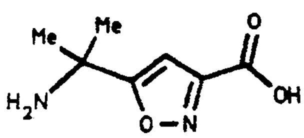 | 53 | 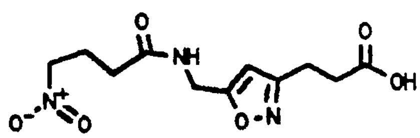 |
| 45 | 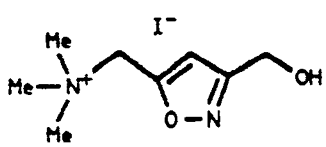 | 54 | 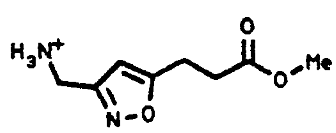<br>Oxalat |

**Tabelle 1 (Fortsetzung)**

| Beisp.-Nr. | Formel | Beisp.Nr. | Formel |
|---|---|---|---|
| 55 | 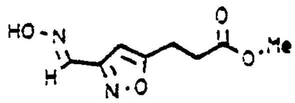 | | |
| 56 | 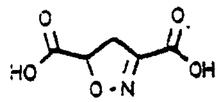 | | |
| 57 | 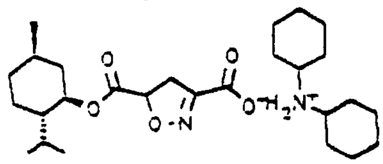 | | |
| 58 | 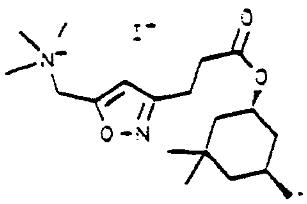 | | |
| 59 | 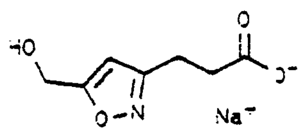 | | |

**Beispiel 1: 3-Carboxy-5-aminomethyl-isoxazol**

a) N-tert.-Butoxycarbonyl-propargylamin

24 g (0.44 mol) Propargylamin und 44.5 g (0.44 mol) Triethylamin werden in 350 ml Diethylether vorgelegt, unter Eiskühlung eine Lösung von 96 g (0.44 mol) Di-tert.-butyldicarbonat in 150 ml Diethylether zugetropft und dann ca 2 Stunden bei Raumtemperatur nachgerührt. Man wäscht mit gesätt. Ammoniumchloridlösung bis pH 6 und danach mit Wasser. Nach Trocknen und Einengen kann das Produkt aus Petrolether im Tiefkühlfach kristallisiert werden. Ausbeute: 62 g, Schmelzpunkt: 41 °C.

b) Cycloaddition mit Ethoxycarbonylnitriloxid

Chloroxyiminoessigsäureethylester wird ausgehend von Glycinethylester-hydrochlorid nach einer Literaturvorschrift (G.S. Skinner, J. Am. Chem. Soc. 46 (1924), 731) hergestellt. 14.55 g (0.096 mol) dieses Produktes werden, gelöst in 100 ml Tetrahydrofuran, bei Raumtemperatur während ca 5 Stunden zu einer Lösung von 17.07 g (0.11 mol) des geschützten Propargylamines aus a) und 11.14 g (0.11 mol) Triethylamin in 400 ml Diethylether getropft. Man rührt über Nacht nach, wäscht mit verd. Ammoniumchloridlösung und Wasser, trocknet und engt i. Vak. ein. Ausbeute: 25 g. Das Produkt 5-tert.-Butoxycarbonylaminomethyl-isoxazol-3-carbonsäureethylester kann durch Kristallisation aus Ethylacetat/Petrolether weiter gereinigt werden, wobei ein Schmelzpunkt von 72 °C resultiert.

c) Verseifung des Carbonsäureesters

9.75 g (0.036 mol) des Produktes aus b) werden in 80 ml Ethanol gelöst, 75 ml 1N NaOH zugesetzt und bei Raumtemperatur unter DC-Kontrolle bis zur Beendigung der Verseifung gerührt. Nach Entfernen des Ethanols i.Vak. wird mit verd. Salzsäure bis pH 2-3 angesäuert, mehrfach mit Dichlormethan oder Ethylacetat extrahiert, mit wenig Wasser gewaschen, getrocknet und eingeengt. Kristallisation aus Diethylether/Petrolether liefert 7.18 g analysenreines Produkt vom Schmelzpunkt 120 - 121 °C.

d) Spaltung der tert.-Butoxycarbonylschutzgruppe

6.9 g des Produktes aus c) werden in 125 ml Dichlormethan gelöst, unter Eiskühlung ca 25 ml Trifluoressigsäure zugesetzt, und danach bei Raumtemperatur unter DC-Kontrolle bis zur Beendigung der Reaktion gerührt. Nach Einengen i. Vak. wird der Überschuß an Trifluoressigsäure mehrmals mit Dichlormethan abgeschleppt und der verbliebene feste Rückstand mit Diethylether ausgerührt. Man erhält 7.16 g des analysenreinen Trifluoracetates von 5-Aminomethyl-3-carboxy-isoxazol. Zur Überführung in das Betain löst man das Salz in Wasser, stellt mit verd. Ammoniaklösung auf pH 7 ein und fällt das Betain durch langsame Zugabe von Aceton aus. Schmelzpunkt: 153 °C.

$^1$H-NMR (Trifluoracetat in DMSO-$d_6$): $\delta$ = 4.5 (s, 2H, $CH_2$-N), 7.1 (s, 1H, 4-H), 9.4 (sb, 5 acide H).

**Beispiel 2: 5-Benzylaminomethyl-3-carboxy-isoxazol**

N-Benzyl-propargylamin wird, wie in Beispiel 1a) beschrieben, mittels Di-tert.-butyldicarbonat geschützt. Cycloaddition mit Ethoxycarbonylnitriloxid, Verseifung der Estergruppe, Abspaltung der N-Schutzgruppe und Fällung des Betains aus dem zunächst anfallenden Trifluoracetat erfolgen wie in Beisp. 1b) - 1d) beschrieben. Das analysenrein erhaltene Produkt zeigt einen Schmelzpunkt von 128 °C.

$^1$H-NMR (Betain in $D_2O$/NaOD): $\delta$ = 3.6 u. 3.7 (2s, jew. 2H, 2 $CH_2$), 6.3 (s, 1H, 4-H), 7.0 - 7.3 (m, 5 Aryl-H).

**Beispiel 3: 5-Pyrrolidinomethyl-3-pyrrolidinocarbonyl-2-isoxazolin - Hydrochlorid**

a) Cycloaddition mit Allylbromid

48.39 g (0.4 mol) Allylbromid und 45.47 g (0.3 mol) Chloroxyimino-essigsäureethylester werden in 700 ml Diethylether vorgelegt und während 6 Stunden eine Lösung von 35.44 g (0.35 mol) Triethylamin in 200 ml Ether Zugetropft. Man rührt über Nacht, filtriert vom ausgefallenen Salz ab, wäscht mit verd. Ammoniumchloridlösung und mit Wasser bis zum Neutralpunkt. Nach Trocknen und Einengen verbleiben 65 g an 5-Brommethyl-3-ethoxycarbonyl-2-isoxazolin, die aus Diethylether/Petrolether umkristallisiert werden können. Schmelzpunkt: 54 °C.

b) Substitutionsreaktion mit Pyrrolidin

11.8 g (0.05 mol) des Produktes aus a) werden in 200 ml Ethanol und 82 ml Pyrrolidin gelöst und für 2.5 Stunden zum Rückfluß erhitzt. Nach Einengen i. Vak. wird mit 2 N Salzsäure aufgenommen, mit Diethylether gewaschen, die wäßrige Phase neutral gestellt, mehrfach mit Dichlormethan extrahiert, die organische Phase getrocknet und eingeengt, und zuletzt das gewünschte Produkt aus Diethylether durch Zugabe von ethanolischer Salzsäure gefällt. Es verbleiben 10.7 g analysenreines Hydrochlorid vom Schmelzpunkt 207 °C.

$^1$H-NMR (Hydrochlorid in $CDCl_3$): $\delta$ = 1.8 - 2.4 (m, 8H, 4 $CH_2$), 2.8 - 4.0 (m, 12H, 4-H u. 4 $CH_2$-N), 5.3 - 5.9 (mc, 1H, 5-H).

**Beispiel 4: 5-Aminomethyl-isoxazol-3-essigsäure**

a) 3-Hydroxyiminopropionsäureethylester

Die Synthese erfolgt in Analogie zu einer Literaturvorschrift (US-Pat. 4 499 278), ausgehend von Ameisensäureethylester und Ethylacetat.

b) Cycloaddition mit N-tert.-Butoxycarbonyl-propargylamin

6.6 g (0.05 mol) des Produktes aus a) werden, gelöst in 100 ml Dichlormethan, bei 0 °C vorgelegt und während 30 min eine Lösung von 6 g (0.055 mol) tert.-Butylhypochlorit in 20 ml Dichlormethan zugetropft. Man rührt nach Entfernen des Kühlbades 1 Stunde nach, fügt eine Lösung von 7.8 g (0.05 mol) N-tert.-Butoxycarbonyl-propargylamin in 50 ml Dichlormethan hinzu, tropft dann während 6 Stunden eine Lösung von 8.3 ml (0.06 mol) Triethylamin in 100 ml Dichlormethan zu und rührt über Nacht. Die Reaktionslösung wird mit Wasser, verd. Citronensäure und erneut Wasser gewaschen, getrocknet und eingeengt. Man erhält 14.2 g eines Öles, das durch Chromatographie an Kieselgel unter Verwendung von tert.-Butylmethylether und Petrolether (1:1) als Eluens weiter gereinigt werden kann.

c) Verseifung des Ethylesters und Spaltung der N-Schutzgruppe

Diese beiden Schritte erfolgen analog zu Beispiele 1c) und 1d). Das als Öl verbleibende Trifluoracetat des Produktes wird in Aceton gelöst, mit alkoholischer Ammoniaklösung ein pH-Wert von 5 bis 6 eingestellt, das ausgefallende Betain abgesaugt und i. Vak. getrocknet. Ausgehend von 8 g aus Stufe b) werden 3.5 g an analysenreinem Betain vom Schmelzpunkt 216 °C erhalten.

$^1$H-NMR (Betain in $D_2O$): $\delta$ = 3.6 (s, 2H, $CH_2$), 4.3 (s, 2H, $CH_2$-N), 6.5 (s, 1H, 4-H).

Für den Cycloadditionsschritt der nachfolgenden Beispiele 5 bis 11 werden Nitrobuttersäurebausteine in einer Variante der Methode von Mukaiyama (J. Am. Chem. Soc. 82 (1960), 5339 - 5342) mittels Isocyanaten dehydratisiert. Der Syntheseweg, ausgehend von Acrylsäurederivaten und Nitromethan ist stellvertretend für die nicht kommerziell erhältlichen Derivate im Folgenden für den Nitrobuttersäurebenzylester beispielhaft beschrieben. Durch Verwendung entsprechend substituierter Vinylphosphonsäure- und Vinylphosphinsäureester ist diese Methode auch auf 4- Nitropropyl-phosphin- und -phosphonsäurederivate übertragbar.

286 g (2.65 mol) Benzylalkohol und 366 ml (2.65 mol) Triethylamin werden unter Eiskühlung in 3 l tert.-Butylmethylether vorgelegt. Man tropft 240 g (2.65 mol) Acrylsäurechlorid hinzu, rührt 2 Stunden bei Raumtemperatur nach, wäscht die organische Phase mehrmals mit Wasser, trocknet und engt i. Vak. ein. Der in quantitativer Ausbeute anfallende Acrylsäurebenzylester wird bei einer Badtemperatur von 70 °C zu einer vorgelegten Lösung aus 2.5 l Nitromethan und 10 ml 1,8-Diazabicyclo-[5.4.0]undec-7-en (DBU) getropft, wobei man den durch Spuren an Acrylsäure möglicherweise abfallenden pH-Wert durch Zugabe entsprechender Mengen an DBU konstant hält. Nach Abklingen der exothermen Reaktion (Temperaturanstieg bis 90 °C) läßt man 60 min abkühlen, wäscht mehrmals mit verd. wäßr. Salzsäure und Wasser, trocknet, und engt i. Vak. ein, wobei 464 g eines rötlichbraunen Öles verbleiben, das ohne weitere Reinigung zur Cycloaddition eingesetzt werden kann.

Auf gleichem Weg können beispielsweise die nachfolgend verwendeten Nitroverbindungen hergestellt werden:

4-Nitrobuttersäure-methyl- und -ethylester
4-Nitrobuttersäure-tert.-butylester
4-Nitrobuttersäure-(+)- und -(-)-menthylester
4-Nitrobuttersäure-cis-(3.3.5)-trimethyl-cyclohexylester
3-Nitropropyl-phosphonsäuredimethyl- und -diethylester (ausgehend von Vinylphosphonsäureestern)
3-Nitropropyl-P-Methyl-phosphinsäureethylester (ausgehend von Vinyl-P-methyl-phosphinsäureethylester).

**Beispiel 5: 5-Aminomethyl-isoxazol-3-propionsäurebenzylester-Hydrochlorid**

a) Cycloaddition

78 g (0.5 mol) N-tert.-Butoxycarbonyl-propargylamin werden zusammen mit 2.5 ml Triethylamin und 96 g (0.6 mol) Phenylendiisocyanat in 2 l Toluol bei 70 °C vorgelegt. 112 g (0.5 mol) des voranstehend beschriebenen 4-Nitrobuttersäurebenzylesters, dem 2.5 ml Triethylamin zugesetzt werden, werden innerhalb 5 Stunden zugetropft. Man rühr über Nacht bei Raumtemperatur, saugt vom ausgefallenen Harnstoff ab und wäscht mit Dichlormethan nach. Nach Einengen verbleiben 200 g eines öligen Rohproduktes, das durch Chromatographie an Kieselgel (Laufmittel: Petrolether/tert.-Butylmethylether-Gemische) weiter gereinigt werden kann.

b) Abspaltung der N-Schutzgruppe

25 g (0.064 mol) des unter a) erhaltenen Produktes werden bei Raumtemperatur mit einer Lösung von 75 ml Trifluoressigsäure in 375 ml Dichlormethan bis zur Beendigung der Gasbildung behandelt (ca 1 Stunde). Das nach Einengen verbleibende Trifluoracetat wird mit Methanol aufgenommen und durch Zugabe von etherischer Salzsäure das Hydrochlorid gefällt, das durch Umkristallisation aus Methanol/tert.-Butylmethylether in eine analysenreine Form überführt werden kann. Ausbeute: 15 g;
Schmelzpunkt: 172 °C.

$^1$H-NMR (Hydrochlorid in DMSO-$d_6$): $\delta$ = 2.8 - 3.0 (m, 4H, 2 $CH_2$), 4.2 (s, 2H, $CH_2$-N), 5.1 (s, 2H, $CH_2$-O), 6.6 (s, 1H, 4-H), 7.25 - 7.45 (m, 5 Aryl-H).

Die nachfolgend in Tabelle 2a aufgeführten Beispiele 6 bis 11 der allgemeinen Formel Ia, worin A für eine C,C-Doppelbindung steht, werden analog zu Beispiel 5 durch Cycloaddition entsprechend substituierter Nitroverbindungen an N-tert.-Butoxycarbonyl-propargylamin und Abspaltung der N-Schutzgruppe synthetisiert.

Tabelle 2a

| Beispiel | $R^1$ | $R^2$ | Schmp [°C] | $^1$H-NMR, δ (ppm) | Bem. |
|---|---|---|---|---|---|
| 6 | $-CH_2-NH_2$ x HCl | $-(CH_2)_2-CO_2Et$ | 165 | (in MeOH-$d_4$): 1.27 (t, J=6.5, 3H, OEt), 2.73 u. 3.0 (jew. mc, 2H, $-CH_2-CH_2-$), 4.13 (q, J=6.5, 2H, OEt), 4.35 (s, 2H, $CH_2$-N), 4.9 (sb, 4H, $NH_3^+$), 6.6 (s, 1H, 4-H) | |
| 7 | $-CH_2-NH_2$ x Tos-OH | $-(CH_2)_2-COO$ 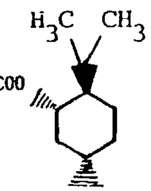 | 157 | (in DMSO-$d_6$): 0.7 (d, 3H, J=6.8, $CH_3$), 0.8-2.0 (m, 15H, 2$CH_3$, 3$CH_2$-, 3CH), 2.3 (s, 3H, Tos-OH), 2.7 und 2.9 (jew. mc, 2H, $-CH_2-CH_2-$), 4.3 (s, 2H, $-CH_2$-N), 4.5-4.7 (dt, 1H, CHO), 6.5 (s, 1H, 4-H), 7.1 u. 7.5 (AA'BB', Tos-OH), 8.4 (acide H) | $\alpha_D^{20}$ = + 29.5° (c = 1, Et-OH) |
| 8 | $-CH_2-NH_2$ x Tos-OH | $-(CH_2)_2-COO$ 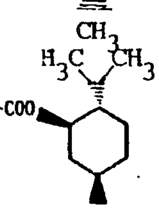 | 157-158 | NMR wie Beisp. 7 | $\alpha_D^{20}$ = - 28.9° (c = 1, Et-OH) |
| 9 | $-CH_2-NH_2$ x Tos-OH | $-(CH_2)_2-COO$ 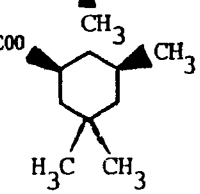 | 175 | (in DMSO-$d_6$): 0.7-2.0 (m, 16H, 3$CH_3$, 3$CH_2$, 1CH), 2.3 (s, Tos-OH), 2.65 u. 2.9 (jew. mc, 2H, $-CH_2-CH_2-$), 4.3 (s, 2H, $CH_2$-N), 4.7 - 4.9 (mc, 1H, CHO), 6.5 (s, 1H, 4-H), 7.1 u. 7.5 (AA'BB', Tos-OH), 8.5 (3 acide H) | Racemat |
| 10 | $-CH_2-NH_2$ x HCl | $-(CH_2)_2-\overset{O}{\underset{OEt}{P}}$ Me | 134 | (in MeOH-$d_4$): 1.3 (t, 3H, J=7, OEt), 1.5 (d, 3H, J=13.8, P-Me), 2.7 und 3.0 (jew. mc, 2H, $-CH_2-CH_2-$), 4.1 (mc, 2H, OEt), 4.35 (s, 2H, $CH_2$-N), 6.6 (s, 1H, 4-H) | |
| 11 | $-CH_2-NH_2$ x HCl | $-(CH_2)_2-CO_2Me$ | 183 | (in DMSO-$d_6$): 2.7 und 2.9 (jew. mc, 2H, $-CH_2-CH_2-$), 3.6 (s, 3H, $OCH_3$), 4.2 (s, 2H, $CH_2$-N), 6.6 (s, 1H, 4-H), 8.9 (3 acide H) | |

**Beispiel 12: 5-Aminomethyl-isoxazol-3-propionsäure**

Die Cycloaddition wird, wie in Beispiel 5 beschrieben, ausgehend von 4-Nitrobuttersäure-tert.-butylester und N-tert.-Butoxycarbonyl-propargylamin durchgeführt. Das erhaltene Rohprodukt wird analog zu Beispiel 5b) mit einem Überschuß an Trifluoressigsäure in Dichlormethan behandelt, wobei gleichzeitig sowohl Spaltung der Esterals auch der Urethanschutzgruppe erfolgt. Der nach Einengen verbleibende Rückstand wird in Aceton aufgenommen, mit Aktivkohle behandelt, filtriert, und der pH-Wert mit konz. Ammoniaklösung auf 6 eingestellt. Das als Betain auskristallisierende Produkt wird nach ca 24 Stunden abgesaugt, mit Aceton gewaschen und getrocknet. Es kann bei Bedarf aus Wasser/Aceton umkristallisiert werden.

Schmelzpunkt: 218 °C (unter Zersetzung)

$^1$H-NMR (Betain in 1N NaOD): δ = 2.5 u. 2.9 (jew. t, 2H, J=7.5, - $CH_2$-$CH_2$-), 3.9 (s, 2H, $CH_2$-N), 6.3 (s, 1H, 4-H).

**Beispiel 13: 5-(1-Amino-1-methyl-ethyl)-isoxazol-3-propionsäure**

3,3-Dimethylpropargylamin wird, wie in Beispiel 1a) beschrieben, mit der tert.-Butoxycarbonyl-Schutzgruppe versehen. Die Cycloaddition mit 4-Nitrobuttersäure-tert.-butylester erfolgt, wie in Beispiel 5a) beschrieben. Abspaltung der Schutzgruppen und Überführung des Trifluoracetates in das Betain analog zu Beispiel 12 liefert ein analysenreines Produkt vom Schmelzpunkt 219 °C.

$^1$H-NMR ( Betain in $D_2O$): δ = 1.7 (s, 6H, 2$CH_3$), 2.5 u. 2.9 (jew. t, 2H, J=7.5, -$CH_2$-$CH_2$-), 6.4 (s, 1H, 4-H).

**Beispiel 14: 5-Benzylaminomethyl-isoxazol-3-propionsäure**

Das analog zu Beispiel 2 mit der BOC-Schutzgruppe versehene N-Benzyl-N-tert.-butoxycarbonyl-propargylamin wird, wie in **Beispiel 5a) beschrieben, mit 4-Nitrobuttersäure-tert.-butylester umgesetzt, dann die Schutzgruppen analog zu** Beispiel 12 entfernt, und das Produkt als Betain ausgefällt. Schmelzpunkt: 153 °C.

$^1$H-NMR (Betain in MeOH-$d_4$): δ = 2.7 u. 3.0 (jew. t, 2H, J=7.5, - $CH_2$-$CH_2$-), 3.85 u. 3.93 (jew. s, 2H, 2$CH_2$-N), 6.2 (s, 1H, 4-H), 7.2 - 7-4 (m, 5 Aryl-H).

**Beispiel 15: 5-Dimethylaminomethyl-isoxazol-3-propionsäure**

Die Cycloaddition erfolgt, wie in Beispiel 5a) beschrieben, ausgehend von N,N-Dimethylpropargylamin und 4-Nitrobuttersäure-tert.-butylester. Die Abspaltung der tert.Butylestergruppe wird analog zu Beispiel 12 durchgeführt. Das erhaltene Rohprodukt wird an Kieselgel chromatographisch gereinigt (Laufmittel: tert.-Butylmethylether/Methanol-Gemische unter Zugabe von 1% wäßr. Ammoniak), und danach aus Aceton durch Einstellen des pH-Wertes mittels Trifluoressigsäure auf 6.5 das Betain ausgefällt. Schmelzpunkt: 85 °C.

$^1$H-NMR (Betain in DMSO-$d_6$)): δ = 2.2 (s, 6H, 2 N-Me), 2.6 u. 2.76 (jew. mc, 2H, -$CH_2$-$CH_2$-), 3.6 (s, 2H, $CH_2$-N), 6.3 (s, 1H, 4-H).

**Beispiel 16: 2-(5-Aminomethyl-isoxazol-3-yl)-ethyl-2-(P-methyl)-**phosphinsäure-Hydrochlorid

5 g (0.019 mol) des Produktes aus Beispiel 10 werden ca 70 Stunden bei Raumtemperatur mit einer 33 %-igen Lösung von HBr in Eisessig behandelt. Nach Einengen i. Vak. wird mit Aceton ausgerührt und anschließend aus Aceton/Wasser umkristalisiert. Ausbeute: 3 g Hydrobromid; Schmelzpunkt: 198 °C.

$^1$H-NMR (Hydrobromid in DMSO-$d_6$): δ = 1.3 (d, J=14, 3H, P-Me), 1.95 u. 2.85 (jew. mc, 2H, -$CH_2$-$CH_2$-), 4.3 (s, 2H, $CH_2$-N), 6.6 (s, 1H, 4-H), 8.55 (4 acide H).

**Beispiel 17: 5-Acetamidomethyl-isoxazol-3-propionsäure**

7 g (0.0413 mol) des Produktes aus Beispiel 12 werden unter intensivem Rühren in einer Lösung von 70 ml Acetanhydrid in 140 ml Pyridin suspendiert, wobei das Edukt langsam in Lösung geht. Nach Beendigung der Reaktion (DC-Kontrolle) wird i. Vak. eingeengt, und das Produkt aus Aceton kristallisiert. Ausbeute: 5.5 g; Schmelzpunkt: 134 °C.

$^1$H-NMR (in DMSO-$d_6$): δ = 1.9 (s, 3H, Ac), 2.8 - 3.05 (m, 4H, -$CH_2$-$CH_2$-), 4.3 (d, J=6, 2H, $CH_2$-N), 6.3 (s, 1H, 4-H), 8.5 (t, J=6, 1H, NH).

**Beispiel 18: 5-Trimethylammoniomethyl-isoxazol-3-propionsäure-(-)-menthylester - Iodid**

9.6 g (0.02 mol) des Produktes aus Beispiel 8 werden zusammen mit 16.6 g (0.12 mol) Kaliumcarbonat in 250 ml Aceton vorgelegt. Nach Zutropfen von 6.25 ml (0.1 mol) Methyljodid wird 3 Tage bei Raumtemp. nachgerührt. Man filtriert vom ausgefallenen Salz, engt ein und kristallisiert aus Wasser. Ausbeute: 6.5 g; Schmelzpunkt: 131 °C (Zers.).

$[\alpha]_D^{20}$ = -40.0° (c = 1 in Ethanol)

$^1$H-NMR (Iodid in MeOH-$d_4$): δ = 0.7 (d, J=7, 3H, Me), 0.85 - 2.05 (m, 15H, 2Me, 3$CH_2$, 3CH), 2.77 u.

3.05 (jew. mc, 2H, $-CH_2-CH_2-$), 3.2 (s, 9H, $NMe_3^+$), 4.7 (mc, 1H, CHO), 4.9 (s, 2H, $CH_2$-N), 6.85 (s, 1H, 4-H).

**Beispiel 19: 5-Trimethylammoniomethyl-isoxazol-3-propionsäure-(+)-menthylester - Iodid**

Das Produkt aus Beispiel 7 wird, wie in Beispiel 18 beschrieben, mit Methyljodid umgesetzt und aus Wasser kristallisiert. Schmelzpunkt: 130 °C.
$[\alpha]_D^{20}$ = +39.3° (c = 1 in Ethanol)
$^1$H-NMR: wie Beispiel 18

**Beispiel 20: 5-(L-Phenylalanyl-amino)-methyl-isoxazol-3-propionsäure-(+)-menthylester - Hydrochlorid**

a) Peptidknüpfung

12.8 g (0.042 mol) der Basenform aus Beispiel 7 werden in 200 ml Tetrahydrofuran vorgelegt. Nach Zugabe von 11.05 g (0.042 mol) N-tert.-Butoxycarbonyl-L-Phenylalanin und 26.7 ml (0.21 mol) N-Ethylmorpholin werden bei 0 °C 26.9 ml (= 0.042 mol) einer 50 %-igen Lösung von Propanphosphonsäureanhydrid in Dichlormethan zugetropft, und danach 4 Stunden unter Erwärmen auf Raumtemp. nachgerührt. Man verdünnt mit Ethylacetat und wäscht die organische Phase mit Citronensäure und Wasser, trocknet, und engt ein. Das Produkt kann aus tert.-Butyl-methylether/Petrolether kristallisiert werden. Ausbeute: 17.5 g; Schmelzpunkt 122 °C.

b) Spaltung der Schutzgruppe

Abspaltung der N-Schutzgruppe erfolgt wie in Beispiel 5b) beschrieben. Der verbleibende Rückstand wird in Ethylacetat aufgenommen und durch Schütteln mit 1N Natronlauge in die Base überführt, die aus Petrolether kristallisiert werden kann.

Analysenreines Hydrochlorid läßt sich aus Methanol mittels etherischer Salzsäure fällen. Der Schmelzpunkt beträgt 148 °C.
$[\alpha]_D^{20}$ = +15.9° (c = 1 in Wasser)
$^1$H-NMR (Hydrochlorid in MeOH-$d_4$): $\delta$ = 0.7 (d, 3H, J=7, Me), 0.75 - 2.0 (m, 15H, 2Me, $3CH_2$, 3CH), 2.7 u. 2.95 (jew. mc, 2H, - $CH_2$-$CH_2$-), 3.0 - 3.3 (mc, 2H, Ph-$CH_2$), 4.1 (mc, 1H, CHN), 4.35 - 4.8 (mc, 3H, CHO u. $CH_2$-N), 6.1 (s, 1H, 4-H), 7.2 - 7.45 (m, 5 Aryl-H).

**Beispiel 21: 5-(L-Phenylalanyl-amino)-methyl-isoxazol-3-propionsäure-(-)-menthylester - Hydrochlorid**

Ausgehend vom Produkt aus Beispiel 8 wird analog zu Beispiel 20 die Peptidknüpfung und die Schutzgruppenspaltung durchgeführt. Das als Hydrochlorid gefällte Produkt hat einen Schmelzpunkt von 135 °C.
$[\alpha]_D^{20}$ = -43.5° (c = 1 in Wasser).
$^1$H-NMR : wie Beisp. 20

**Beispiel 22: 5-(L-Phenylalanyl-amino)-methyl-isoxazol-3-propionsäure-methylester - Hydrochlorid**

Ausgehend vom Produkt aus Beispiel 11 wird analog zu Beispiel 20 die Peptidknüpfung und die Schutsgruppenspaltung durchgeführt. Das als Hydrochlorid gefällte Produkt hat einen Schmelzpunkt von 133 °C.
$[\alpha]_D^{20}$ = +3.6° (c = 1 in Wasser).
$^1$H-NMR (Hydrochlorid in MeOH-$d_4$): $\delta$ = 2.65 u. 2.9 (jew. mc, 2H, $-CH_2-CH_2-$), 3.1 (mc, 2H, Ph-$CH_2$), 3.63 (s, 3H, OMe), 4.03 (t, J=7.5, 1H, CHN), 4.3 - 4.55 (AB, J=16, 2H, $CH_2$-N), 6.0 (s, 1H, 4-H), 7.1 - 7.4 (m, 5 Aryl-H).

**Beispiel 23: 5-Guanidinomethyl-isoxazol-3-propionsäure**

6.8 g (0.04 mol) des Produktes aus Beispiel 12 werden in 40 ml 1N Natronlauge gelöst und 5.56 g S-Methyl-isothioharnstoff-Sulfat (= 0.04 mol Harnstoff) in fester Form zugegeben. Man rührt 24 Stunden bei

Raumtemperatur, wobei das Produkt langsam auskristallisiert, saugt ab und wäscht gründlich mit Wasser. Ausbeute: 2.8 g; Schmelzpunkt >297 °C.

$^1$H-NMR (Betain in TFA-$d_1$): δ = 2.95 u. 3.22 (jew. mc, 2H, $-CH_2-CH_2-$), 4.7 (s, 2H, $CH_2$-N), 6.5 (s, 1H, 4-H).

**Beispiel 24: N-(5-Aminomethyl-isoxazol-3-yl)-propionyl-glycin**

a) Selektive Esterspaltung

100 g des Cycloadduktes aus Beispiel 5a) (Rohprodukt) werden zur Verseifung des Benzylesters in 500 ml Isopropanol und 500 ml 1N Natronlauge gelöst, nach Beendigung der Reaktion (DC-Kontrolle) die wäßr. Lösung zweimal mit tert.-Butylmethylether extrahiert, unter Eiskühlung mit wäßr. Salzsäure angesäuert und das Produkt mehrfach mit Ethylacetat aus der Wasserphase extrahiert. Nach Trocknen und Einengen wird aus tert.-Butylmethylether kristallisiert. Ausbeute: 32 g; Schmelzpunkt: 80 °C.

b) Peptidknüpfung

11.6 g (0.043 mol) des Produktes aus a) werden zusammen mit 7.1 g (0.043 mol) Glycinbenzylester und 27.23 ml (0.215 mol) N-Ethylmorpholin in 210 ml Tetrahydrofuran und 210 ml N,N-Dimethylformamid gelöst, und unter Eiskühlung 27.23 ml (= 0.043 mol) einer 50 %-igen Lösung von Propanphosphonsäureanhydrid in Dichlormethan zugetropft. Man rührt unter DC-Kontrolle ca 5 Stunden bei Raumtemperatur nach, verdünnt mit Ethylacetat und wäscht mit Citronensäure und Wasser. Nach Trocknen und Einengen verbleiben 17 g eines öligen Produktes.

c) Verseifung der Estergruppe

12 g des Produktes aus b) werden in 390 ml Isopropanol gelöst und 150 ml 1N Natronlauge zugesetzt. Nach Beendigung der Reaktion wird unter Eiskühlung mit Salzsäure angesäuert, und das Produkt mehrmals mit Ethylacetat extrahiert, die organische Phase getrocknet und eingeengt, wobei 5.9 g eines Öles verbleiben.

d) Spaltung der N-tert.-Butoxycarbonyl-Schutzgruppe

5 g des Produktes aus c) werden in einer Mischung aus 15 ml Trifluoressigsäure und 75 ml Dichlormethan aufgenommen, nach ca 1 Stunde i. Vak. eingeengt, mit Aceton aufgenommen und mittels konz. wässr. Ammoniak ein pH-Wert von 6.5 eingestellt. Das Betain wird abgesaugt und getrocknet. Ausbeute: 2.5 g; Schmelzpunkt 204 °C.

$^1$H-NMR (in $D_2O$): δ = 2.65 u. 3.0 (jew. t, 2H, J=7.6,$-CH_2-CH_2-$), 3.6 (s, 2H, Gly-$CH_2$), 4.3 (s, 2H, $CH_2$-N), 6.5 (s, 1H, 4-H).

**Beispiel 25: 5-Aminomethyl-3-(2-hydroxy)-ethyl-isoxazol -Hydrochlorid**

a) Cycloaddition

Die Cycloaddition wird ausgehend von 38.8 g (0.25 mol) N-tert.-Butoxycarbonyl-propargylamin und 39.56 g (0.25 mol) 3-Nitropropanol-tert.-butylether (vergl. R. Öhrlein et al., Synthesis 1986, 535 - 538) analog zu Beispiel 5a) durchgeführt. Das Produkt 5-tert.-Butoxycarbonylamino-methyl-3-(2-hydroxy)-ethyl-isoxazol wird in öliger Form in 61.6 g Ausbeute isoliert.

b) Spaltung der Schutzgruppen

Die N- und O-Schutzgruppen werden, wie in Beispiel 5b) beschrieben, mittels Trifluoressigsäure gespalten und die Lösung eingeengt. Zur Reinigung wird das Rohprodukt in Methanol auf eine Säule mit stark saurem Kationenaustauscher ($H^+$-Form) gegeben, mit Methanol gewaschen, dann das Produkt mit 4N methanolischer Ammoniaklösung eluiert, und die Produktfraktionen eingeengt. Aus Methanol kann anschließend mit etherischer Salzsäure analysenreines Hydrochlorid mit einem Schmelzpunkt von 163 °C gefällt werden.

$^1$H-NMR (Hydrochlorid in $D_2O$): δ = 2.85 u. 3.8 (jew. t, 2H, J=6, $-CH_2-CH_2-$), 4.3 (s, 2H, $CH_2$-N), 6.5 (s,

1H, 4-H).

**Beispiel 26: Bis-(3-[2-Carboxy]-ethyl-isoxazol-5-yl-methyl)-amin - Diammoniumsalz**

a) Cycloaddition

Dipropargylamin wird, wie in Beispiel 1a) beschrieben, mit der N-tert.-Butoxycarbonylschutzgruppe versehen. 30 g (0.16 mol) dieser Verbindung werden mit 60 g (0.32 mol) 4-Nitro-buttersäure-tert.-butylester unter Verwendung von 60 g (0.36 mol) Phenylendiisocyanat analog zu Beispiel 5a) der 1.3-dipolaren Cycloaddition unterzogen. Die erhaltenen 100 g Rohprodukt können durch Chromatographie an Kieselgel unter Verwendung von tert.-Butylmethylether/Petrolether-Gemischen gereinigt werden.

b) Spaltung der Schutzgruppe

Die Spaltung der BOC-Schutzgruppe und des tert.-Butylethers erfolgt analog zu Beispiel 5b) mit Trifluoressigsäure. Der nach Einengen verbleibende Rückstand wird in Aceton aufgenommen und durch Zugabe von konz. wäßr. Ammoniak das Ammoniumsalz gefällt. Ausbeute: 15 g; Schmelzpunkt: 146 °C aus 30 g Vorstufe.

$^1$H-NMR (Ammoniumsalz in DMSO-$d_6$): $\delta$ = 2.36 u. 2.74 (jew. t, 2H, J=6.5, -$CH_2$-$CH_2$-), 3.8 (s, 2H, $CH_2$-N), 5.4 (sb, $NH4^+$), 6.2 (s,1H, 4-H).

**Beispiel 27: 5-Aminomethyl-2-isoxazolin-3-propionsäure-(+)-menthylester - Toluol-4-sulfonat**

a) Cycloaddition

Allylamin wird, wie in Beispiel 1a) beschrieben, mit der N-tert.-Butoxycarbonylschutzgruppe versehen. Das Produkt wird nach dem Einengen in fester Form mit einem Schmelzpunkt von 38 °C isoliert. 41.1 g (0.26 mol) davon werden mit 72 g (0.26 mol) 4-Nitrobuttersäure-(+)-menthylester und 50.1 g (0.3 mol) Phenylendiisocyanat in Analogie zu Beispiel 5a) der Cycloaddition unterzogen. Man erhält 95 g eines öligen Rohproduktes, das analog zu Beispiel 26a) weiter gereinigt werden kann.

b) Spaltung der BOC-Schutzgruppe

Die Spaltung der Schutzgruppe erfolgt in Analogie zu Beispiel 5b). Das Produkt wird durch Extraktion mittels tert.- Butylmethylether aus verd. Natronlauge als Base isoliert und als Tosylat aus tert.-Butylmethylether durch Zugabe eines Äquivalentes an Toluol-4-sulfonsäure gefällt. Laut GG-Analyse liegt ein Gemisch der beiden C-5 - Epimeren im Verhältnis von ca 1:1 vor. Schmelzpunkt: 144 - 145 °C.

$^1$H-NMR (Tosylat in DMSO-$d_6$): $\delta$ = 0.7 (d, J=6.8, 3H, Me), 0.8 - 1.95 (m, 15H, 2Me, $3CH_2$, 3CH), 2.3 (s, Tos-OH), 2.55 - 3.3 (m, 8H, -$CH_2$-$CH_2$-, CH2-N, 4-H), 4.5 - 4.8 (m, 2H, 5-H u. CHO), 7.1 u. 7.66 (AA'BB', Tos-OH), 8.0 (acide H).

**Beispiel 28: 5-Aminomethyl-2-isoxazolin-3-propionsäure-(-)-menthylester - Toluol-4-sulfonat**

Die Herstellung erfolgt, ausgehend von 4-Nitrobuttersäure-(-)-menthylester in Analogie zu Beispiel 27. Das als Tosylat gefällte Produkt hat einen Schmelzpunkt von 142 - 144 °C und liegt ebenfalls als Epimerengemisch vor.
$^1$H-NMR: wie Beispiel 27

**Beispiel 29: 5-Aminomethyl-2-isoxazolin-3-propionsäure, Racemat**

Das racemische Produkt wird ausgehend von 4-Nitrobuttersäure-tert.-butylester und N-tert.-Butoxycarbonyl-allylamin in Analogie zu Beispiel 12 hergestellt. Nach Spaltung der Schutzgruppen wird in Aceton aufgenommen und das Betain durch Einstellen des pH-Wertes auf 6 mittels konz. wäßr. Ammoniak. gefällt. Der Schmelzpunkt beträgt 201 °C (unter Zers.).

$^1$H-NMR (Betain in $D_2O$): $\delta$ = 2.48 u. 2.65 (jew. mc, 2H, -$CH_2$-$CH_2$-), 2.85 - 3.45 (m, 4H, 4-H u. $CH_2$-N), 4.8 (sb, acide H), 4.9 (mc, 1H, 5-H).

**Beispiel 30: 5-Aminomethyl-2-isoxazolin-3-propionsäure, (-)-Enantiomer**

a) Enantiomerentrennung

80 g (0.26 mol) der freigesetzten Base des Produktes aus Beispiel 27 werden in 2 l Methylethylketon gelöst und 93 g (0.26 mol) (-)-O,O'-Dibenzoyl-L-weinsäure zugesetzt, worauf sich langsam Kristalle abscheiden. Nach Absaugen wird aus gleichem Lösungsmittel umkristallisiert, wobei 30 g des nach GC-Analyse >98% enantiomerenreinen Salzes vom Schmelzpunkt 162 °C verbleiben.

b) Spaltung des Menthylesters

20 g (0.03 mol) aus a) werden durch Ausschütteln mit tert.-Butylmethylether aus 1N Natronlauge in die Base überführt. Der nach Einengen verbleibende Rückstand wird bei 0 °C in 270 ml Trifluoressigsäure aufgenommen, mit 3.5 ml Thioanisol und 26.5 ml Trifluormethansulfonsäure versetzt, 1 Stunde im Eisbad gerührt, und langsam mit ca 300 ml Diethylether versetzt. Nach vollständiger Kristallisation wird abgesaugt, der Rückstand mit Ethanol aufgenommen, und mit konz. wässr. Ammoniak der pH-Wert auf 6.0 eingestellt. Das so isolierte Betain wird aus Wasser/Ethanol umkristallisiert, wobei 2.5 g des nach GC >99% enantiomerenreinen Betains vom Schmelzpunkt 176 °C (Zers.) resultieren.
$[\alpha]_D^{20}$ = -123.5° (c = 1 in Wasser).
$^1$H-NMR (Betain in $D_2O$): wie Racemat

**Beispiel 31: 5-Aminomethyl-2-isoxazolin-3-propionsäure, (+)-Enantiomer**

a) Enantiomerentrennung

Die aus der fraktionierten Kristallisation von Beispiel 30a) verbleibende Mutterlauge wird wie oben beschrieben in die freie Base überführt. 66 g (0.21 mol) davon werden in Ethanol gelöst, und nach Zugabe von 31.5 g (0.21 mol) D(-)-Weinsäure das Tartrat gefällt und aus Ethanol umkristallisiert. Man erhält 30 g des nach GC-Analyse >98% enantiomerenreinen Salzes vom Schmelzpunkt 163 °C.

b) Spaltung des Menthylesters

Die Spaltung des Menthylesters erfolgt nach Freisetzen der Base, wie in Beisp. 30b) beschrieben. Aus 20 g Tartrat werden nach Umkristallisation 2.9 g des nach GC >99% enantiomeren-reinen Betains vom Schmelzpunkt 183 °C (Zers.) erhalten.
$[\alpha]_D^{20}$ = +121° (c = 1 in Wasser)

**Beispiel 32: 5-Acetamidomethyl-2-isoxazolin-3-carbonsäure-Natriumsalz**

2.5 g (0.015 mol) des Racemates aus Beispiel 29 werden analog zu Beispiel 17 acetyliert, wobei 2.8 g eines amorphen Produktes resultieren. Nach Aufnehmen in Aceton kann durch Zugabe der äquivalenten Menge einer 10%-igen acetonischen Lösung von Natrium-2-ethylhexanoat das analysenreine Natriumsalz gefällt werden. Schmelzpunkt : 238 °C.

$^1$H-NMR (Na-Salz in $D_2O$): $\delta$ = 1.94 (s, 3H, Ac), 2.37 u. 2.54 (jew. mc, 2H, $-CH_2-CH_2-$), 2.75 u. 3.1 (AB v. ABX, 2H, 4-H), 3.3 (d, J=5, 2H, CH2-N), 4.7 (mc, 1H, 5-H).

**Beispiel 33: 3-(2-Carboxyethyl)-5-(2-pyridyl)-2-isoxazolin**

59 g (0.38 mol) 4-Nitropropionsäure-tert.-butylester werden mit 80 g (0.76 mol) 2-Vinylpyridin und 69.5 g (0.42 mol) Phenylendiisocyanat analog zu Beispiel 5a) cycloaddiert. Der nach Aufarbeitung verbleibende Rückstand wird in Dichlormethan aufgenommen und über eine kurze, mit Celite gefüllte Säule filtriert. Man erhält 77 g eines Öles, das analog zu Beispiel 12 der Esterspaltung unterzogen wird. Die weitere Reinigung erfolgt durch Chromatographie an Kieselgel unter Verwendung von Methanol/tert.-Butylmethylethergemischen unter Zusatz von 1% wäßr. Ammoniak. Nach Einengen der Produktfraktionen wird kristallines Produkt vom Schmelzpunkt 93 °C als Betain durch Ansäuern einer acetonischen Lösung mittels Trifluoressigsäure bis pH 4 erhalten.

$^1$H-NMR (Betain in DMSO-$d_6$): $\delta$ = 2.5 (mc, 4H, $-CH_2-CH_2-$, 3.2 u. 3.4 (AB v. ABX, 2H, 4-H), 5.5 (mc, 1H, 5-H), 7.2 - 7.5, 7.8 u. 8.55 (jew. m, 5 Pyridyl-H).

**Beispiel 34: 3-Aminomethyl-2-isoxazolin-5-propionsäuremethylester-Toluol-4- sulfonat**

a) Cycloaddition

Zur Herstellung der Olefinkomponente wird 4-Pentencarbonsäure nach literaturbekannten Verfahren mittels Thionylchlorid und Methanol verestert. Die Cycloaddition wird analog zu Beispiel 5a) mittels 2-Nitroethanol-2-tetrahydropyranylether (s. V. Jäger et al, Angew. Chem 93 (1981), 576 - 578) durchgeführt. Das Rohprodukt kann ohne weitere Reinigung weiterverarbeitet werden.

b) Spaltung der Tetrahydropyranyl-Schutzgruppe

200 g des Produktes aus a) werden mit 500 ml einer 1 N methanolischen Salzsäure über Nacht bei Raumtemperatur behandelt. Nach Einengen wird an Kieselgel mittels Petrolether/tert.-Butylmethylether chromatographiert.

c) 3-Chlormethyl-2-isoxazolin-5-propionsäuremethylester

7.4 ml (0.081 mol) Phosphoroxychlorid und 33.8 ml (0.243 mol) Triethylamin werden in 200 ml Tetrahydrofuran vorgelegt, und 5 g (0.027 mol) des Produktes aus b), gelöst in 50 ml Tetrahydrofuran, bei Raumtemperatur zugetropft. Nach 20 h wird mit Methanol und danach mit Wasser hydrolysiert, das Produkt mit Dichlormethan extrahiert, getrocknet, eigeengt und wie unter b) chromatographisch gereinigt.

d) Substitution mittels $NH_3$

2 g aus c) werden zusammen mit 100 mg Tetrabutylammoniumiodid in 50 ml 2N methanolischer Ammoniaklösung gelöst und unter DC-Kontrolle 3 - 5 d bei Raumtemperatur belassen. Nach Beendigung der Reaktion wird eingeengt und an Kieselgel chromatographiert (Eluens: Methanol/tert.-Butylmethylether unter Zusatz von 1 % wäßr. $NH_3$). Anschließend kann mittels 4-Toluolsulfonsäure aus Methanol/tert.-Butylmethylether das Tosylat kristallin mit einem Schmelzpunkt von 168 °C erhalten werden.

$^1$H-NMR (Tosylat in $CDCl_3$/MeOH-$d_4$ 1:1): $\delta$ = 1.9 u. 2.4 (jew. m, 2H, $CH_2$-$CH_2$), 2.33 (s, Tos-OH), 2.7 u. 3.13 (AB v. ABX, 2H, 4-H), 3.62 (s, 3H, COOMe), 3.85 (sb, 2H, $CH_2$-N), 4.7 (mc, 1H, 4-H)7.2 u. 7.7 (AA'BB', Tos-OH).

**Beispiele 35 - 54:**

Die in Tabelle 2b aufgeführten Beispiele 35-58 wurden analog zu den voranstehend beschriebenen, in der Tabelle jeweils zitierten Beispielen synthetisiert und anhand der spektroskopischen Daten charaktierisiert.

Tabelle 2 b

| Beispiel-Nr. | Formel | Edukt(e), Bem. | analog zu Beispiel | Schmelzpunkt [°C] | $^1$H-NMR d [ppm] |
|---|---|---|---|---|---|
| 35 | 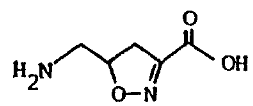 | 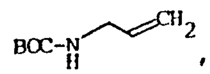 , 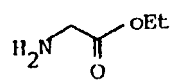 | 1 b-d | 137-138 | (in $D_2O$): 2.9-3.5 (m, 4H, $2CH_2$), 4.9-5.1 (mc, 1H, 5-H) |
| 36 | 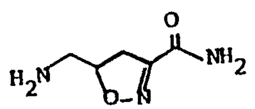 x CF3COOH | 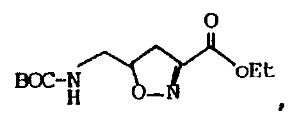 , $NH_3$ (Raumtemperatur) | 3b, Spaltung:1d | 117 | (in DMSO-$d_6$) 2.8-3.7 (m, 4H, 2 $CH_2$), 4.8-5.3 (mc, 1H, 5-H) 7.7-8.8 ($NH_2$ u. $NH_3^+$) |
| 37 | 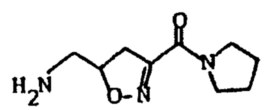 x HCl | 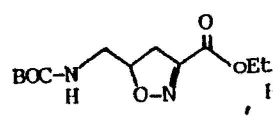 , 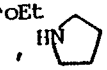 (Rückfluß) | 3b, Spaltung:1d | 147-148 | (in DMSO-$d_6$): 1.6-2.0(m, 4H, $2CH_2$),2.9-3.8(m, 8H, $4CH_2$),4.8-5.35 (mc, 1H, 5-H) 8.5-9.0 (acide H) |
| 38 | 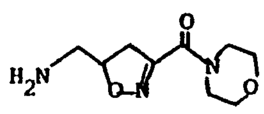 x HCl | 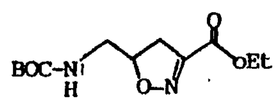 , 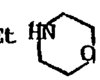 | 3b, Spaltung:1d | 171-173 | (in DMSO-$d_6$): 2.8-3.75 (m, 12H, $6CH_2$), 4.7-5.2 (mc, 1H, 5-H), 8.2-8.5 (acide H) |
| 39 | 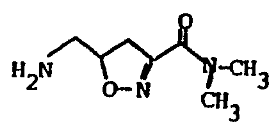 x HCl | 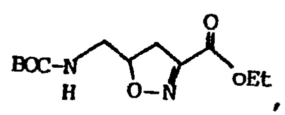 , 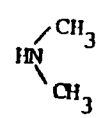 | 3b, Spaltung:1d | 142 | (in DMSO-$d_6$): 2.9 und 3.05 (jew. sb, 3H, $2CH_3$), ca. 2.8-3.5 (m, 4H, $2CH_2$), 4.65-5.15 (mc, 1H, 5-H) 8.25-8.7 (acide H) |

Tabelle 2 b (Fortsetzung)

| Beispiel-Nr. | Formel | Edukt(e), Bem. | analog zu Beispiel | Schmelzpunkt [°C] | $^1$H-NMR d [ppm] |
|---|---|---|---|---|---|
| 40 | 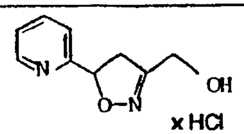 x HCl | 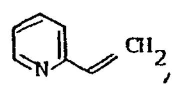, 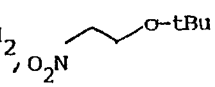 | 25 a,b | 133,6 | (In $D_2O$): 3.0-4.05 (AB von ABX, 2H, 4-H), 5.7-6.05 (X v. ABX, 1H, 5-H), 4.35 (sb, 2H, $CH_2$-O), 7.5-8.6 (m, 4 Aryl-H) |
| 41a | 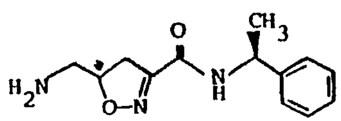 x $CF_3COOH$ | 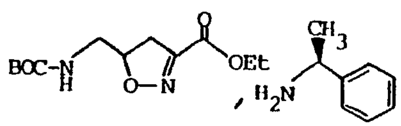 Die Diastereomeren wurden durch fraktionierte Kristallisation aus Ethylacetat/Petroläther getrennt erhalten | 3b, Spaltung:1d | 122 ($[a]_D^{20}$ = -104.2°, c=1, $H_2O$) | (In DMSO-$d_6$): Identisch für beide Epimere 1.5 (d,J=7.2, 3H, $CH_3$), 2.95-3.5 (m, $2CH_2$), 4.8-5.15 (m, 2H, CHN und CHO), 7.1-7.5 (m, 5 Aryl-H), 8.1 ($NH_3^+$), 9.0 (d, J=8.7, NH) |
| 41b | 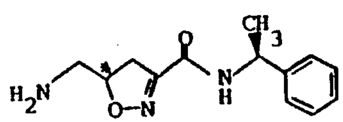 x $CF_3COOH$ | | | 134 ($[a]_D^{20}$ = +48.4°, c = 1, $H_2O$ | |

Tabelle 2 b. (Fortsetzung)

| Beispiel-Nr. | Formel | Edukt(e), Bem. | analog zu Beispiel | Schmelzpunkt [$^{o}C$] | $^{1}H$-NMR d [ppm] |
|---|---|---|---|---|---|
| 42 | 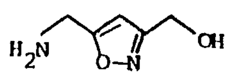 | 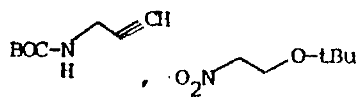 | 25 a,b | 67 | (In DMSO-$d_6$): 2.8-3.3 (acide H) 3.7 und 4.3 (jew. s, 2H, $2CH_2$) 6.1 (s, 1H, 4-H) |
| 43 | 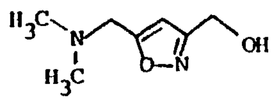 x HCl | 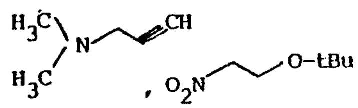 | 25 a,b | 127 | (In DMSO-$d_6$): 2.7 (s, 6H, $NMe_2$), 4.4-4.45 (jew.s, 2H, $2CH_2$), 6.7 (s, 1H, 4-H) |
| 44 | 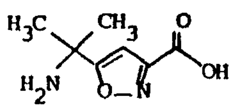 | 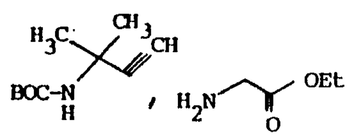 | 1 a - d | 174 | (In DMSO-$d_6$): 1.65 (sb,6H,2Me), 6.8 (s,1H,4-H), 8.5-8.85 (acide H). |
| 45 | 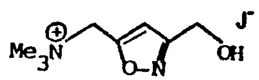 | 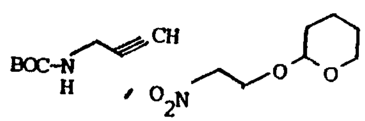 | CA, Spaltung: 25a-d Methylierung: 18 | 86 | (In DMSO-$d_6$): 3.1 (s, 9H, $NMe_3$), 4.4 (d,J=6, 2H, $CH_2OH$, 4.8 (s, 2H, $CH_2N$), 5.3(t, J=6, OH), 6.7 (s, 1H, 4-H) |
| 46 | 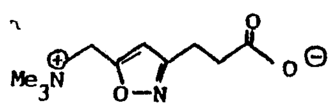 | Methylierung in Aceton/Methanol/ Wasser 2:2:1 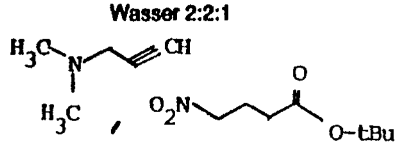 | 1b-d Methylierung: 18 | 202 | (In $D_2O$): 2.52 u. 2.95 (jew.t, J=7, 2H,-$CH_2$-$CH_2$-), 3.17 (s, 9H, $NMe_3^+$), 4.7 (s, 2H, -$CH_2$-$N^+$), 6.77 (s, 1H, 4-H) |

Tabelle 2 b (Fortsetzung)

| Beispiel-Nr. | Formel | Edukt(e), Bem. | analog zu Beispiel | Schmelzpunkt [°C] | $^1$H-NMR d [ppm] |
|---|---|---|---|---|---|
| 47 | 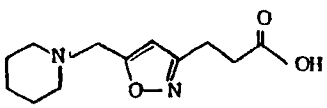<br>x HCl | 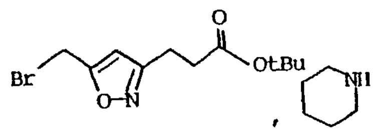 | Cycloadd.und Substitution:<br>3a,b<br>Spaltung:1d | 132-134 | (in DMSO-$d_6$):<br>1.5-1.9 (m, 6H, 3$CH_2$)<br>2.6 u. 2.9 (jew. t, J=7, 2H,-$CH_2$-$CH_2$-),<br>3.2-3.6 (m, 4H, 2 $CH_2$-N)<br>4.5 (sb, 2H, $CH_2$-N), 6.75 (s, 1H, 4-H) |
| 48 | 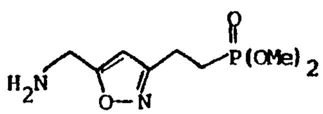<br>x HCl | 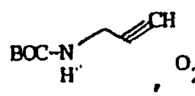 , 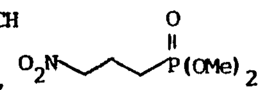 | 5a, 1d | 127 /Zers. | (in DMSO-$d_6$):<br>2.0-2.3 und 2.7-2.9 (jew. m, 2H, -$CH_2$-$CH_2$-),<br>3.65 (d, J= 10.8, 6H, 2 OMe). 4.2 (s, 2H, $CH_2$-N), 6.7 (s, 1H, 4-H),<br>8.95 (acide H) |
| 49 | 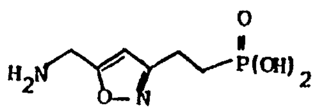<br>x HBr | Beispiel 48 | 16 | ab 200 Zers. | (in $D_2O$/NaOD):<br>1.55-1.8 und 2.75 - 2.9 (jew. mc, 2H,-$CH_2$-$CH_2$-),<br>4.2 (s, 2H, $CH_2$-N), 6.43 (s, 1H, 4-H) |
| 50 | 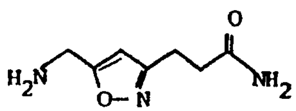<br>x HCl | 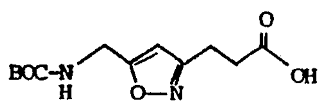 , $NH_3$ | 24 b<br>Spaltung:1d | 238-239 | (in DMSO-$d_6$):<br>2.44 und 2.84 (jew. t,2H,-$CH_2$-$CH_2$-),<br>4.2 (s,2H,$CH_2$-N), 6.55 (s,1H,4-H),<br>6.9 und 7.5 (jew. sb,1H,-$CONH_2$),<br>8.9 ($NH_3^+$) |

Tabelle 2 b. (Fortsetzung)

| Beispiel-Nr. | Formel | Edukt(e), Bem. | analog zu Beispiel | Schmelzpunkt [°C] | $^1$H-NMR d [ppm] |
|---|---|---|---|---|---|
| 51 | 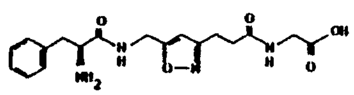<br>x $CF_3COOH$ | 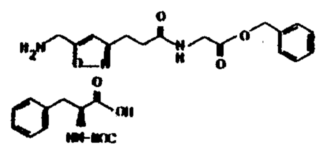 | Peptid: 24b<br>Debenzyl.:24c<br>Boc-Spaltung:24d | 117-122 | (in $CDCl_3$/MeOH-$d_4$ 1:1):<br>2.6 und 2.93(gew. mc, 2H,-$CH_2$-$CH_2$-),<br>3.1(mc, 2H, Ph-$CH_2$), 3.9(sb, 2H,<br>-$CH_2$-COOH) 4.1 (t, J=7.5, 1H, CHN),<br>4.4(sb, 2H, $CH_2$-N), 6.0 (s, 1H, 4-H),<br>7,1-7.4 (m, 5-Aryl-H). |
| 52 | 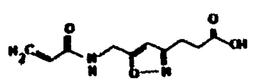 | 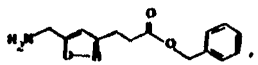, 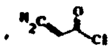<br>$NEt_3$ in Tetrahydro-furan | Acyl. ähnlich 17<br>Debenzyl: 24c | 115 | (in MeOH-$d_4$):<br>2.65 und 2.92 (gew.t,2H,J=7,-$CH_2$-$CH_2$-),<br>4.5 (sb, 2H, $CH_2$-N, 5.6-5.75<br>(m, 1H, =CH-) 6.1 (s, 1H, 4-H)<br>6.15-6.3 (m, 2H, =$CH_2$) |
| 53 | 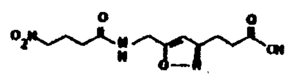 | 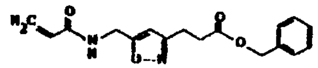<br>$O_2N$-$CH_3$ | siehe Vorbemerkung zu Beisp. 5 | 98-100 | (in $CDCl_3$/MeOH-$d_4$ 1 : 1):<br>2.2-2.4 (m, 4H, $CH_2$-$CH_2$-<br>2.65 und 2.92 (gew.t,J=7, 2H,-$CH_2$-$CH_2$),<br>4.4 (s, 2H, $CH_2$-N),4.5(t, 2H, $O_2NCH_2$)<br>6.1 (s, 1H, 4-H) |
| 54 | 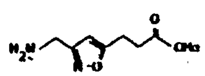<br>Oxalat | 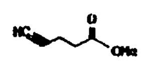<br>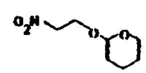 | 34a-c | 175 | (in MeOH-$d_4$):<br>2.77 und 3.1 (gew. t, J=7, 2H,-$CH_2$-$CH_2$-)<br>3.68 (s, 3H, $CH_3$-) 4.2(s, 2H, $CH_2$-N)<br>6.3 (s,1H,4-H) |

Tabelle 2 b. (Fortsetzung)

| Beispiel-Nr. | Formel | Edukt(e), Bem. | analog zu Beispiel | Schmelzpunkt [°C] | $^1$H NMR d [ppm] |
|---|---|---|---|---|---|
| 55 | 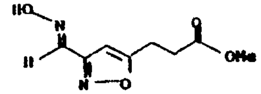 | 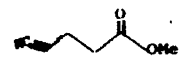 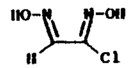 | 1b | 99 | (in $CDCl_3$): 2.75 und 3.1 (jew.t, J=7,2H, $-CH_2-CH_2$); 3.7 (s,3H,OMe); 6.4 (s, 1H,4-H); 8.2 (s, 1H, Oxim-CH); 9.3 (sb, 1H, Oxim-OH) |
| 56 | 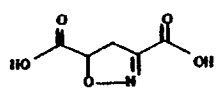 | 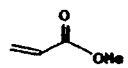 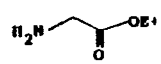 | 1b,c (Überschuß NaOH) | 135 | (in DMSO-$d_6$): 2.9-3.6 (AB von ABX,2H,4-H) 4.9-5.25 (X von ABX,1H,5-H) |
| 57 | 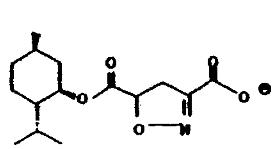 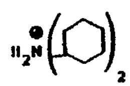 | 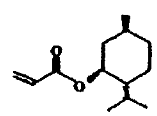 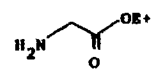 | 1b,c (äquimolar NaOH) | 103 | (in $CDCl_3$): 0.7 (d,3H,J=7,$CH_3$) 0.8-2.15 (m,35H,2$CH_3$,13$CH_2$,3CH) 3.1 (mc,2H,2CHN) 3.3-3.65 (AB von ABX,2H,4-H) 4.75 (mc,1H,Menthyl-CHO) 5.05 (X von ABX,1H,5-H) |
| 58 | 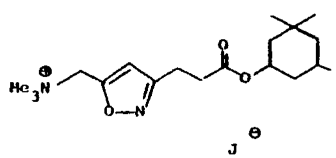 | Beispiel 9 | 18 | 158 | (in MeOH-$d_4$): 0.7-2.0 (m,16H,3$CH_3$,3$CH_2$,1CH) 2.72 und 3.05 (jew.mc,2H,$-CH_2-CH_2$) 3.22 (s,9H, $NMe_3$) 4.8-5.0 (m,CHO,$CH_2$-N und HDO) 6.82 (s,1H,4-H) |

Beispiel 59: 5-Hydroxymethyl-isoxazol-3-propionsäure Natriumsalz

a) Propargylalkohol-tert.-butylether

101 ml (1.9 mol) Propargylalkohol wird in einer Druckflasche bei 0 °C in ca 1 l Dichlormethan gelöst, 2o

ml konz. Schwefelsäure zugesetzt und 450 g (8 mol) Isobuten einkondensiert. Nach 5 Stunden bei 0 °C wird der Überschuß Isobuten entfernt, mit Natruimcarbonatlösung gewaschen, getrocknet, eingeengt und das Produkt destilliert.

b) Cycloaddition mit Nitrobuttersäurebenzylester

33.7 g (0.3 mol) des Produktes aus a) werden mit 66.9 g (0.3 mol) Nitrobuttersäurebenzylester, 0.35 mol Phenylendiisocyanat und Triethylamin analog zu Beispiel 5 cycloaddiert. Das Rohprodukt kann durch Chromatograhie an Kieselgel (Ethylacetat/Petrolether) gereinigt werden.

c) Spaltung der tert.-Butylether-Schutzgruppe

100 g des Rohproduktes aus b) werden in 1.5 l Dichlormethan und 300 ml Trifluoressigsäure gelöst, und der Reaktionsverlauf per DC kontrolliert. Nach Einengen wird an Kieselgel chromatographiert (mobile Phase: Ethylacetat/Petrolether 1:1). Es verbleibt ein öliges Produkt.

d) Verseifung des Benzylesters

2 g (0.0017 mol) des Produktes aus c) werden in 10 ml Ethanol gelöst, 10 ml 1 N Natronlauge zugesetzt und bei Raumtemperatur bis zur Beendigung der Verseifung gerührt (DC-Kontrolle). Das Produkt wird durch Zugabe von Aceton ausgefällt und mehrmals aus Methanol/tert.-Butyl-methylether umkristallisiert. Schmelzpunkt: 168 °C

$^1$H-NMR ($D_2O$): $\delta$ = 2.45 und 2.85 (jew. t, 2H, $-CH_2-CH_2-$), 4.6 (s, 2H, $CH_2-O$), 6.26 (s, 1H, 4-H).

Die Verbindungen der Formel I wurden zur Charakterisierung ihrer wertvollen neuroprotektiven Eigenschaften und guten Verträglichkeit in folgenden experimentellen Testanordnungen untersucht, die sich anerkanntermaßen besonders gut für die Beurteilung der Wirkqualität derartiger im ZNS aktiven Verbindungen eignen.

**1. "High affinity"-Aufnahme von 3H-Aspartat in einer Vesikelpräparation aus Rattengehirn**

**Testprinzip:** Frische synaptische Vesikel (Synaptosomen) aus Rattencortex werden mit Pufferlösung und $^3$H-D-Aspartat inkubiert und $^3$H-D-Aspartat in den Vesikeln gemessen. Präparate, die im Vergleich zu Kontroll-Synaptosomen die hoch affinen $^3$H-D-Aspartat-Aufnahme erhöhen, sowie diejenigen, die den Verlust von $^3$H-D-Aspartat durch Wiederfreisetzung verhindern, gelten als besonders interessant.

**Versuchsprotokoll:** Die Versuche zur $^3$H-D-Aspartat-Aufnahme wurden nach der Methode von Anand et al. (Biochem. Pharmacol., 35 (1986), 1055-1057) durchgeführt. Die synaptischen Vesikel wurden aus Gehirncortex von männlichen Sprague-Dawley Ratten präpariert. Die Präparate wurden in vier Konzentrationen als Doppelproben parallel zu Kontrollansätzen mit Puffer getestet. Das Inkubationsmedium (pH 7.4) enthält: 1.2 mM $KH_2PO_4$, 5 mM KCl, 5 mM Pyruvat, 1.2 mM Glucose, 1.2 mM $MgCl_2$, 114 mM NaCl, 25 mM $NaHCO_3$. Für die unspezifische $^3$H-D-Aspartat-Aufnahme wurde NaCl durch 114 mM Cholinchlorid und $NaHCO_3$ durch 25 mM $KHCO_3$ ersetzt. Ein Aliquot der Vesikelsuspension (ca. 25 µg/ml Test) wurde dem auf 37 °C temperierten, mit Carbogen (5% $CO_2$, 95% $O_2$) begasten Testansatz zugesetzt und 2 Minuten vorinkubiert. Durch Zugabe von 50 µl $^3$H-D-Aspartat (spezifische Aktivität 25 Ci/mMol, 10 pMol/ml Test) wurde die dreiminütige Inkubation der Aminosäureaufnahme gestartet. Die Trennung der Vesikel vom Inkubationsmedium erfolgte durch Filtration und zusätzliches Waschen der anhaftenden markierten Aminosäuren mit dem Cell-Harvester Titertek (Fa. Flow Laboratories, Meckenheim). Die $^3$H-Aspartat-Aufnahme wurde mittels eines Flüssigkeits-szintillationszählers (Canberra-Packard, Modell 1500, Frankfurt) gemessen.

Die Präparatwirkung ist in Prozent der spezifischen $^3$H-Aspartat-Aufnahme, bezogen auf Kontrollwerte (100 %), in Tabelle 3 angegeben. Zum Vergleich dient Adenosintriphosphat (ATP) als Standardsubstanz (siehe Tab. 3), die bei 1 mM eine 35 %-ige Erhöhung der $^3$H-Aspartat-Aufnahme bewirkt. ATP ist unwirksam bei geringerer Konzentration als 500 µm, zeigt aber eine konzentrationsabhängige Wirkung bei höherer Konzentration.

**Tabelle 3** **Stimulierung der $^{3}H$-Aspartat-Aufnahme**

| Beispiel Nr. | Präparat-konzentration µM | Stimulierung (%) |
|---|---|---|
| 1. | 500 | 15 |
| 2. | 500 | 12 |
| 3. | 500 | 20 |
| 5. | 10 | 26 |
| 6. | 10 | 17 |
| 8. | 10 | 13 |
| 9. | 100 | 19 |
| 10. | 500 | 23 |
| 12. | 500 | 17 |
| 13. | 500 | 33 |
| 14. | 500 | 19 |
| 16. | 1000 | 19 |
| 18. | 100 | 46 |
| 19. | 100 | 25 |
| 22. | 100 | 23 |
| 24. | 500 | 28 |
| 25. | 500 | 20 |
| 31. | 500 | 21 |
| 32. | 100 | 19 |
| 39. | 1000 | 14 |
| 50. | 500 | 15 |
| 56. | 500 | 17 |
| 58. | 10 | 35 |
| ATP | 500 | 31 |
| ATP | 1000 | 35 |
| ATP | 2000 | 57 |
| ATP | 5000 | 121 |

**2. Hemmung der $^{3}H$-Acetylcholin-Freisetzung aus Striatum-Schnitten des Rattenhirnes**

**Testprinzip:** Für den Test wurden frische Striatumschnitte aus Rattengehirn mit Pufferlösung in einer speziellen Kammer überspült und die Freisetzung des Tritium-markierten Acetylcholin nach Stimulierung mit NMDA gemessen. Präparate mit NMDA- antagonistischer Wirkung hemmen die Acetylcholinfreisetzung.

**Versuchsprotokoll:** Die Superfusionsversuche wurden nach der Methode von Wichman et al. (Naunyn-Schmied. Arch. Pharmacol., 338 (1988), 623-631) durchgeführt. Rattengehirnschnitte, 0.5 mm dick, wurden 30 min lang in 2 ml Inkubationsmedium, welches 0.1 µmol/l $^{3}H$-Cholin mit einer spezifischen Aktivität von 80 Ci/mmol enthielt, vorinkubiert. In jede von 6 Superfusionskammern wurde ein Schnitt gebracht und mit einer Geschwindigkeit von 1.5 ml/min 90 min lang superfundiert. Von dem Superfusat wurden ständig 2 min-Fraktionen gesammelt. Jeder Schnitt wurde zweimal je zwei Minuten lang mit 25 µmol/l NMDA superfundiert, um die Freisetzung von Acetylcholin zu stimulieren. Der erste Stimulus ($S_1$) erfolgt zwischen

der 54. und der 56. Minute und der 2. Stimulus ($S_2$) zwischen der 76. und 78. Minute. Das Inkubationsmedium enthielt in mmol/l: NaCl 118, KCl 4.8, $CaCl_2$ 1.2, $MgSO_4$ 1.2, $NaHCO_3$ 25, $KH_2PO_4$ 1.2, Glucose 10. Das Medium wurde mit Carbogen bei 37 °C gesättigt. Der pH-Wert wurde auf 7.4 eingestellt. NMDA und die zu prüfenden neuen Präparate wurden in Superfusionsmedium gelöst. Die Superfusion wurde mit $Mg^{2+}$-freiem Medium ausgeführt. Tritium aus den aufgelösten Schnitten und aus dem Superfusat wurde durch Flüssigkeitsszintillationszählung unter Verwendung eines Packard® 1900 CA (Frankfurt) bei einer 50 %igen Effizienz bestimmt.

**Berechnung der Ergebnisse:**

Basisfreisetzung und NMDA-stimulierte Freisetzung für $S_1$ und $S_2$ wurden in %/min jeder Fraktion ausgedrückt (Limberger et al., Naunyn Schmied. Arch. Pharmacol. 339 (1988), 53-61). Der Wert für die stimulierte Freisetzung $S_2/S_1$ wurde für die Kontrolle mit 0.69 ± 0.09 errechnet. Der Wert nimmt ab, wenn eine Substanz, wie z.B. der literaturbekannte NMDA-Rezeptor-Antagonist CPP (3-(+)-2-carboxypiperazin-4-yl-propyl-1-phosphonsäure) die NMDA-stimulierte Freisetzung hemmt. Die Hemmung der stimulierten Freisetzung durch Präparate wurde in Prozent, bezogen auf Kontrollwert berechnet, und ist in Tabelle 4 angegeben.

**Tabelle 4 Hemmung der $^3$H-Acetylcholinfreisetzung aus Striatumschnitten**

| Beispiel Nr. | Präparat-konzentration µM | Hemmung (%) |
|---|---|---|
| 1. | 10 | 13 |
| 7. | 10 | 28 |
| 8. | 10 | 30 |
| 18. | 10 | 22 |
| 19. | 10 | 16 |
| 20. | 10 | 18 |
| 21. | 10 | 20 |
| 22. | 10 | 15 |
| 33. | 10 | 26 |
| 34. | 10 | 9 |
| 56. | 10 | 11 |
| 59. | 10 | 26 |
| CPP | 10 | 30 |

**3. Hemmung der $^3$H-MK-801-Bindung an Membranen aus Rattengehirn**

**Testprinzip:** MK-801 ist ein nicht kompetetiver Antagonist der an NMDA-Rezeptoren gekoppelte Ionenkanäle (Yoneda et al., Brain Res. 499 (1989), 305) bindet. Im Test werden Membranen aus Gesamtgehirn mit Präparat und $^3$H-MK-801 inkubiert. Die Bindung von $^3$H-MK-801 bzw. dessen Verdrängung durch Präparate (Antagonisten) wird auf radiochemischem Wege ermittelt.

**Versuchsprotokoll:** Die Bindungsversuche werden nach der Methode von Reynolds et al. (Proc. Natl. Acad. Sci. USA, 84 (1987), 7744) durchgeführt. Zunächst erfolgt die Membranpräparation nach Reynolds. Dann werden Testansätze pipettiert, wobei Puffer sowie Prüfpräparat vorgelegt werden, dann mit Protein 2

Minuten vorinkubiert, und mit $^3H$-MK801 gestartet wird. Die Membranen werden bei 37 °C aufgetaut, 10 Minuten bei 37 °C inkubiert und 15 Sekunden bei halbmaximaler Geschwindigkeit mittels Polytron homogenisiert, dann wieder gekühlt verwendet. Das Inkubationsmedium enthält: Hepes-Puffer 20 mM pH 7.4, Präparate in drei Konzentrationen 5 µM, 10 µM und 50 µM, Membranen 500 µg/ml Test, $^3H$-MK801 1 nM. Die Inkubation erfolgt 60 Minuten bei Zimmertemperatur (23 °C), dann wird durch Filtration gestoppt. Dazu wird Glasfaserpapier Nr. 32 der Firma Schleicher und Schüll, das am Tag vor dem Versuch 30 Minuten in Polyäthylenimin 0.03 % eingelegt und danach getrocknet wird, verwendet. Die ausgestanzten Filter werden in 4 ml Scintillationsflüssigkeit "Packard 199TM" (Frankfurt) gezählt. Tritium wird durch Flüssigkeitsszintillations-Zählung unter Verwendung eines Packard 1500 bestimmt. Berechnung der Ergebnisse: Die Berechnung der Bindung in femtomol/mg Protein in 60 min. erfolgt mit einem Kleinrechner (HP-97). Antagonistiert eine Substanz die Bindung, nimmt der Wert ab. Substanzwirkungen werden prozentual, d. h. in % Bindung gegenüber der Kontrolle in der Tabelle 5 angegeben. Zur Überprüfung des Testes wird immer die Wirkung des Vergleichspräparates CPP (3-(+)-2-carboxypiperazin-4-yl-propyl-1-phosphonsäure) bei 5 µM, 10 µM, 50 µM ermittelt.

**Tabelle 5 Hemmung der $^3H$-MK-801-Bindung am NMDA-Rezeptor-Kanal Komplex**

| Beispiel Nr. | Präparat-konzentration µM | Hemmung (%) |
|---|---|---|
| 2. | 50 | 10 |
| 5. | 50 | 78 |
| 19. | 50 | 16 |
| 21. | 50 | 45 |
| 57. | 50 | 43 |
| CPP | 50 | 81 |

**4. Inhibierung der NMDA-induzierten Krampfe an der Maus**

Die nachfolgend aufgeführten Beispiele zeigen neuroprotektive Wirkung, indem sie die durch NMDA (N-Methyl-(D)-aspartat) induzierten Krämpfe antagonisieren. Dazu werden intravenöse Injektionen von 20-30 mg/kg NMDA an männlichen NMRI (Naval Medical Research Institute)-Mäusen von 22-24 g Körpergewicht vorgenommen, was zu Stereotypien, klonischen Krämpfen und in der Mehrzahl der Fälle zur Letalität führt. Um eine NMDA-antagonistische Wirkung der Testsubstanzen zu untersuchen, verabreicht man diese oral eine Stunde vor NMDA. Anschließend werden die Tiere auf die oben genannten Symptome hin 60 min beobachtet. Als Bewertungskriterium gilt die Anzahl der Tiere mit klonischen Krämpfen und die Inzidenz der Letalität, ausgedrückt in Prozent Änderung gegenüber der Kontrollgruppe. Als Vergleichspräparate werden der literaturbekannte GABA-Agonist Muscimol sowie die als NMDA-Antagonist beschriebene Substanz MK 801 verwendet, die ebenfalls als aktiv gegen neurodegenerative Veränderungen beschrieben sind.

Da die untersuchten Beispiele gegenüber den Standardsubstanzen beträchtliche Unterschiede in der Toxizität aufweisen, sind in Tabelle 6 ebenfalls die $LD_{50}$ Werte aufgeführt.

**Tabelle 6: Inhibierung der NMDA-induzierten Konvulsionen sowie Toxizität an der Maus**

| Beisp. Nr. | Dosis [mg/kg] | Hemmung d. Convulsionen | Hemmung d. Mortalität | $LD_{50}$ [mg/kg] |
|---|---|---|---|---|
| Muscimol | 0.3 po. | 40 % | 60 % | 3-10 iv. |
| MK 801 | 0.3 po. | 60 % | 60 % | 30-60 iv. |
| 1 | 63 po. | 50 % | 50 % | >200 iv. |
| 6 | 50 po. | 20 % | 40 % | >100 iv. |
| 8 | 50 po. | 70 % | 100 % | 50-100 iv. |
| 10 | 50 po. | 10 % | 70 % | >100 iv. |
| 12 | 50 po. | 45 % | 60 % | >100 iv. |
| 14 | 50 po. | 40 % | 40 % | >300 ip. |
| 15 | 50 po. | 40 % | 100 % | >100 iv. |
| 16 | 50 po. | 30 % | 30 % | - |
| 17 | 30 po. | 30 % | 100 % | >300 ip. |
| 20 | 50 po. | 10 % | 100 % | 25-50 iv. |
| 21 | 50 po. | 30 % | 40 % | 25-50 iv. |
| 23 | 50 po. | 40 % | 40 % | >300 ip. |
| 26 | 50 po. | 40 % | +/-0 % | >100 iv. |
| 27 | 50 po. | 20 % | 20 % | 50-100 iv. |
| 28 | 50 po. | 20 % | 20 % | - |
| 29 | 50 po. | 20 % | 20 % | >200 iv. |
| 32 | 50 po. | 30 % | 50 % | >100 iv. |
| 53 | 50 po. | 33 % | 33 % | - |
| 55 | 50 po. | 33 % | 100 % | - |

**5. Neuroprotektion nach Vorderhirn-Ischämie am Gerbil**

Anhand der nachfolgend beschriebenen, an anderer Stelle publizierten (K. Rudolphi et. al, J. Cereb. Blood Flow Metab., 7:74, 1987) Versuchsdurchführung ergibt sich, daß die unten aufgeführten Beispiele in der Lage sind, zerebrale Nervenzellen gegen schwere ischämische Schädigungen zu schützen.

Bei 20 männlichen Mongolischen Gerbils wird unter Halothan-Narkose durch beidseitigen Verschluß der A. Carotis mittels Aneurysmaclips eine 5-minütige Ischämie des Vorderhirns mit anschließender Rezirkulation erzeugt. 7 Tage später werden die Tiere unter Halothan-Narkose dekapitiert, die Gehirne in Carnoy-Lösung immersionsfixiert und das Ausmaß des Neuronenschadens in der $CA_1$-Region des Hippocampus mit Hilfe eines 5-stufigen histopathologischen Scores "blind" bestimmt. Die Prüfsubstanz wird je einer Gruppe von 10 Gerbils intraperitoneal oder per os 15 Minuten vor der Ischämie verabreicht. Pro Testsubstanz wird eine ischämische Kontrollgruppe von ebenfalls 10 Tieren mit dem jeweiligen Lösungsmittel behandelt. Die Ergebnisse sind in Tabelle 7 als % Änderung des mittleren neurologischen Verhaltens Scores bzw. histopathologischen Scores (Summe der individuellen Scores/Anzahl der Tiere) der behandelten vs. Kontrollgruppe zusammengefaßt.

**Tabelle 7: Nekrosen der $CA_1$-Pyramidalzellen nach dreiminütiger Vorderhirn-Ischämie am Mongolischen Gerbil**

| Beisp. Nr. | Dosis (mg/kg) | Zellnekrosen (% Änderung) | Verhaltensscore (% Änderung) |
|---|---|---|---|
| Muscimol | 5 ip. | -74 | +41 |
| MK 801 | 5 ip. | -23 | +24 |
| 8 | 50 po. | -46 | 0 |
| 12 | 10 ip. | -32 | 0 |

| Beisp. Nr. | Dosis (mg/kg) | Zellnekrosen (% Änderung) | Verhaltensscore (% Änderung) |
|---|---|---|---|
| 12 | 50 po. | -14 | 0 |
| 16 | 50 po. | -27 | 0 |
| 18 | 50 po. | -23 | +15 |
| 19 | 50 po. | -25 | 0 |

Aus der Zusammenstellung ist ersichtlich, daß die Standardsubstanzen Muscimol und MK 801 zwar ebenfalls zu einer Reduktion der absterbenden Zellen fuhren, im Verhaltensscore jedoch deutlich stärkere negative postischämische Symptome zeigen, während die Testsubstanzen die neurologischen Symptome nicht negativ beeinflussen.

**6. Der photochemisch induzierte Infarkt des Vorderhirnes an der Ratte läßt sich wie folgt testen:**

Die theoretische Basis und praktische Anwendung dieser Methode wurde schon früher beschrieben (Grome et al, J. Cereb. Blood Flow Metabol. 8 (1988) 89-95). Männliche Sprague-Dawley Ratten (300-350 g) werden mit 1% Halothan in Sauerstoff betäubt. Ein kleiner Schnitt in der Kopfhaut wird durchgeführt und 1 ml des photochemisch aktiven Farbstoffes Bengal Rose (5 mg/ml in Kochsalzlösung) i.v. appliziert. Anschließend wird ein Teil des Schädels (Durchmesser von 3 mm) mit grünen Licht (570 nm) einer Xenonlampe (75 W) 15 Minuten lang beleuchtet. Zu diesem Zeitpunkt wird die Narkose beendet und die Ratten zufallsmäßig in Medikament- und Placebo-behandelte Gruppen (n = 6) eingeteilt. In den Experimenten in denen nur eine Dosis der Substanz verabreicht wird, findet die Applikation 30 Minuten nach Induktion der Ischämie statt. 24 Stunden später werden diese Tiere durch Dekapitation getötet. Die Gehirne werden schnell entnommen und eingefroren. Serienkryostatschnitte durch die ganze Ausdehnung der Läsion werden mit Kresylviolett gefärbt. Zur Volumenbestimmung der durch Ischämie bedingte Zerstörung werden 90 dieser Schnitte mittels eines Bildanalysesystems vermessen. Das Volumen wird in Mikroliter berechnet und als prozentuale Änderung zur Placebo-behandelten Kontrolle ausgedrückt. In den zwei auf das ischämische Ereignis folgenden Stunden werden die Tiere intensiv beobachtet und ein Verhaltensscore mit einer Skala von 0 (keine Symptome) bis 4 (schwere motorische Symptome inklusive Schwierigkeiten bei der Futteraufnahme) erstellt.

**Patentansprüche**

1. Verfahren zur Herstellung einer Verbindung der Formel Ia, Ib oder Ic

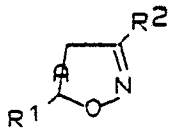 (Ia) 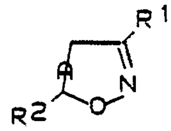 (Ib) 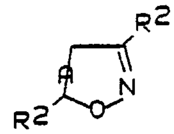 (Ic)

in welcher

$R^1$ für 2-, 3-, 4-Pyridyl oder einen Rest der Formel II

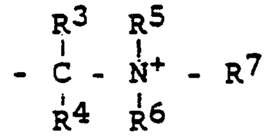 (II)

steht, wobei

$R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_6$-Alkyl; $C_3$-$C_6$-Cycloalkyl; $C_6$-$C_{12}$-Aryl-$C_1$-$C_4$-Alkyl; Carbamimidoyl; $C_1$-$C_6$-Alkylcarbonyl, $C_1$-$C_4$-Alkenylcarbonyl, $C_1$-$C_6$-Alkyloxycarbonyl, $C_6$-$C_{12}$-Aryl-$C_1$-$C_4$-Alkylcarbonyl, $C_6$-$C_{10}$-Aryl-$C_1$-$C_4$-Alkyloxycarbonyl, $C_6$-$C_{10}$-Arylcarbonyl, den Rest einer natürlich vorkommenden $\alpha$-Aminosäure oder $\gamma$-Aminobuttersäure, die durch $C_1$-$C_6$-Alkyl, Hydroxy, Halogen, Amino oder Nitro substituiert sein können; oder

$R^6$ und $R^7$ zusammen mit dem sie verknüpfenden Stickstoffatom einen fünf- bis siebengliedrigen Heterozyklus bilden, wobei ein Kohlenstoffatom durch ein Schwefel-, Sauerstoff- oder Stickstoffatom ersetzt sein kann, bedeuten; oder

$R^5$, $R^6$, $R^7$ unabhängig voneinander $C_1$-$C_4$-Alkyl oder $C_3$-$C_6$-Cycloalkyl bedeuten, oder $R^5$ $C_1$-$C_4$-Alkyl bedeutet und $R^6$ und $R^7$ zusammen mit dem sie verknüpfenden Stickstoffatom einen fünf- bis siebengliedrigen Heterozyklus bilden, oder $R^5$, $R^6$ und $R^7$ zusammen mit dem sie verknüpfenden Stickstoffatom einen sechs- bis zwölfgliedrigen Heterozyklus bilden;

R2 für einen Rest der Formel III

$-(CH_2)_n-X$ (III)

steht, wobei

n 0 oder eine ganze Zahl von 1 bis 4 bedeutet;

X für Hydroxy; $C_1$-$C_4$-Alkyloxy; Carboxy; Halogenformyl; Formyl; Oxyimino; $C_1$-$C_{12}$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden $\alpha$-Aminosäure, $\gamma$-Aminobuttersäure oder einem Dipeptid peptidisch verknüpft ist, oder Aminocarbonyl bedeutet, wobei Amino durch $C_1$-$C_6$-Alkyl mono- oder disubstituiert oder durch Phenyl-$C_1$-$C_6$-Alkyl monosubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann;

oder X für eine Gruppe der Formel IV

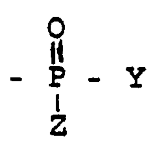 (IV)

steht, wobei

Y und Z unabhängig voneinander Hydroxy, $C_1$-$C_4$-Alkyl oder $C_1$-$C_4$-Alkyloxy bedeuten; und
A für eine C,C-Einfach- oder eine C,C-Doppelbindung steht;

mit der Maßgabe, daß $R^1$ nicht 2-, 3- oder 4-Pyridyl bedeutet, falls $R^2$ für eine Gruppe der Formel IV steht und $n \neq 0$ ist, falls X für Hydroxy oder Methyloxy steht, sowie ihre gegebenenfalls stereoisomere Form und ihr physiologisch verträgliches Salz, dadurch gekennzeichnet, daß man ein Nitriloxid der Formel V

$$\overset{-}{O}-\overset{+}{N}\equiv C-R^2 \qquad (V)$$

a) für den Fall, daß A in Formel Ia eine C,C-Einfachbindung ist, mit einem Olefin der Formel VI, oder

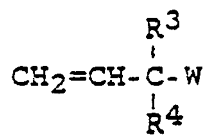

(VI)

b) für den Fall, daß A in Formel Ia eine C,C-Doppelbindung ist, mit einem Propargylderivat der Formel VII im Sinne einer 1.3-dipolaren Cycloaddition umsetzt,

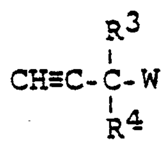

(VII)

wobei $R^2$, $R^3$ und $R^4$ die vorgenannten Bedeutungen haben und W eine Gruppe der Formel $NR^5R^6R^7$ mit den vorgenannten Bedeutungen für $R^5$, $R^6$ und $R^7$, oder ein durch ein gegebenenfalls substituiertes Amin der Formel $NR^5R^6R^7$ austauschbaren Substituenten, wie zum Beispiel Alkylsulfonyl oder Arylsulfonyl oder Halogen, hier vorzugsweise Chlor oder Brom darstellt, oder

c) eine nach a) oder b) hergestellte Verbindung der allgemeinen Formel VIII

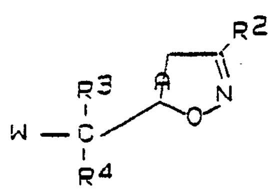

(VIII)

mit den oben genannten Bedeutungen für $R^2$, $R^3$, $R^4$ und A durch Substitution der Gruppe W mit einem gegebenenfalls substituierten Amin der Formel $NR^5R^6R^7$ in eine Verbindung der allgemeinen Formel Ia überführt, oder

d) zur Darstellung einer Substanz der allgemeinen Formel Ib oder Ic ein Alken- oder Alkinsäurederivat der allgemeinen Formel IX,X,XII oder XIII

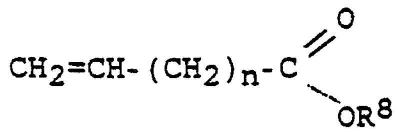 (IX)

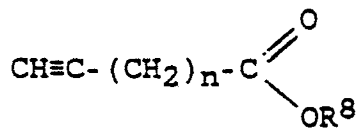 (X)

$CH_2 = CH-(CH_2)_n-O-R^{10}$ (XII)

$CH \equiv C-(CH_2)_n-O-R^{10}$ (XIII)

wobei $R^{10}$ Wasserstoff, eine Alkylgruppe, eine beliebige Schutzgruppe für eine Alkoholfunktion oder eine veresterte Carboxygruppe darstellt, n die vorgenannte Bedeutung hat, und $R^8$ einen beliebigen Alkyl- oder Aralkylrest mit einem Nitriloxid der allgemeinen Formel XI darstellt,

$O \leftarrow N \equiv C-R^2$ (XI)

wobei $R^2$ die vorgenannte Bedeutung hat, mit der Maßgabe, daß $R^2$ nicht α-Aminosäure oder ein Dipeptid sein kann im Sinne einer 1.3-dipolaren Cycloaddition umsetzt, gegebenenfalls die vorliegende Schutzgruppe $R^{10}$ nach literaturbekannten Verfahren entfernt, die Alkoholfunktion in ein zum Austausch mit Aminen aktiviertes Derivat wie z.B. Halogenid oder Tosylat überführt, und danach mit einem Amin der allgemeinen Formel $NR^5R^6R^7$ mit der vorgenannten Bedeutung für $R^5$-$R^7$ umsetzt, oder

e) einen nach a)-d) hergestellten Carbonsäureester der Formel Ia oder Ib zur Carbonsäure verseift beziehungsweise einen gegebebenfalls vorliegenden Benzylester hydrogenolytisch spaltet, oder

f) einen nach Verfahren a)-d) hergestellten Carbonsäurealkylester mit einem entsprechend substituierten primären oder sekundären Amin in das Amid überführt, oder

g) einen nach a) oder b) hergestellten Phosphinsäuremonoalkyl- oder Phosphonsäuredialkylester der allgemeinen Formel Ia nach an sich literaturbekannten Verfahren zum Phosphonsäurehalbester, zur Phosphonsäure oder zur Phosphinsäure verseift, oder

h) eine nach e) erhaltene Carbonsäure zunächst in ein aktiviertes Säurederivat überführt, dieses anschließend mit Alkoholen verestert, mit primären und sekundären Aminen in die Amide, oder mit gegebenenfalls carboxygeschützten Aminosäuren oder niederen Peptiden in am Stickstoff acylierte Peptide überführt, und anschließend gegebenenfalls die Carboxyschutzgruppe am Peptidteil entfernt oder zum Beispiel durch Umesterung in eine andere Gruppe umwandelt, oder

i) in einer nach Verfahren a)-h) erhaltenen Verbindung der allgemeinen Formel Ia einen gegebenenfalls als Schutzgruppe verwendeten Rest $R^7$ nach literaturbekannten Verfahren vom Stickstoffatom entfernt, oder

j) eine nach Verfahren i) hergestellte Verbindung, deren Carboxygruppe in veresterter Form oder als Amid vorliegt, durch Umsetzung mit einem aktivierten Carbonsäurederivat oder gegebenenfalls einer am Stickstoff geschützten Aminosäure oder einem niederen Peptid unter Verwendung von in der Peptidchemie üblichen Reagenzien in ein am Stickstoff acyliertes Derivat überführt, oder

k) eine nach Verfahren j) hergestellte Verbindung durch Spaltung des gegebenenfalls vorhandenen Carbonsäuresters in eine freie Carbonsäure überführt, oder

l) eine nach Verfahren j) oder k) hergestellte Verbindung durch Spaltung einer gegebenenfalls mit dem Aminosäureteil eingeführte N-Schutzgruppe in eine freie Aminoverbindung oder ein Betain überführt, oder

m) eine nach Verfahren c)-i) hergestellte Verbindung der allgemeinen Formel Ia oder Ib durch Umsetzung mit einem Alkylierungsmittel, vorzugsweise einem Alkylhalogenid, in eine quartäre Ammoniumverbindung überführt, oder

n) eine nach Verfahren a)-m) hergestellte Verbindung der allgemeinen Formel Ia und Ib, die aufgrund ihrer chemischen Struktur in diastereoisomeren oder enantiomeren Formen auftritt, in an sich bekannter Weise in die reinen Stereoisomeren auftrennt, wobei man

o) die nach Verfahren a)-n) hergestellten Verbindungen der allgemeinen Formel Ia und Ib entweder

in freier Form isoliert oder im Falle des Vorliegens von sauren oder basischen Gruppen gegebenenfalls in physiologisch verträgliche kristalline Salze umwandelt.

2. Verfahren nach Anspruch 1, dadurch gekennzeichnet, daß man eine Verbindung der Formel Ia oder Ib, in welcher $R^1$ für 2-, 3-, 4-Pyridyl oder für einen Rest der Formel II

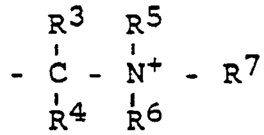

(II)

steht, wobei $R^3$ und $R^4$ unabhängig voneinander Wasserstoff oder $C_1$-$C_4$-Alkyl;

| | |
|---|---|
| $R^5$ | ein freies Elektronenpaar oder Wasserstoff; |
| $R^6$ und $R^7$ | unabhängig voneinander Wasserstoff; $C_1$-$C_4$-Alkyl; Phenyl-$C_1$-$C_2$-Alkyl; |
| $R^6$ und $R^7$ | zusammen mit dem sie verknüpfenden Stickstoffatom einen fünf- bis siebengliedrigen Heterozyklus bilden, wobei ein Kohlenstoffatom durch ein Schwefel-, Sauerstoff- oder Stickstoffatom ersetzt sein kann, bedeuten; |
| $R^6$ | Wasserstoff und $R^7$ Carbamimidoyl; $C_1$-$C_6$-Alkylcarbonyl, $C_1$-$C_4$-Alkenylcarbonyl, $C_1$-$C_6$-Alkyloxycarbonyl, Phenyl-$C_1$-$C_4$-Alkylcarbonyl, Benzyloxycarbonyl, Benzoyl, den Rest einer natürlich vorkommenden $\alpha$-Aminosäure oder $\gamma$-Aminobuttersäure, die durch $C_1$-$C_4$-Alkyl, Hydroxy, Halogen, Amino oder Nitro substituiert sein können; |
| $R^5$, $R^6$, $R^7$ | unabhängig voneinander $C_1$-$C_4$-Alkyl oder $C_3$-$C_6$-Cycloalkyl bedeuten; |
| $R^2$ | für einen Rest der Formel III |

$-(CH_2)_n-X$ (III)

steht wobei

| | |
|---|---|
| n | 0 oder eine ganze Zahl von 1 bis 3 bedeutet; |
| X | für Hydroxy; $C_1$-$C_4$-Alkyloxy; Carboxy; $C_1$-$C_4$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden $\alpha$-Aminosäure, $\gamma$-Aminobuttersäure oder einem Dipeptid peptidisch verknüpft ist; Aminocarbonyl bedeutet, wobei Amino durch $C_1$-$C_4$-Alkyl mono- oder disubstituiert oder durch Phenyl-$C_1$-$C_4$-Alkyl monosubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff-oder Stickstoffatom ersetzt sein kann; |
| | oder X für eine Gruppe der Formel IV |

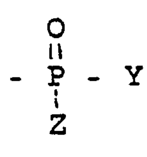

(IV)

steht, wobei

| | |
|---|---|
| Y und Z | unabhängig voneinander Hydroxy, $C_1$-$C_4$-Alkyl oder $C_1$-$C_4$-Alkyloxy bedeuten; und |
| A | für eine C,C-Einfach- oder eine C,C-Doppelbindung steht; |

mit der Maßgabe, daß $R^1$ nicht 2-, 3-, oder 4-Pyridyl bedeutet, falls $R^2$ für eine Gruppe der Formel IV steht, herstellt.

3. Verfahren nach Anspruch 1 oder 2, dadurch gekennzeichnet, daß man eine Verbindung der Formel Ia, in welcher $R^1$ für 2-Pyridyl oder für einen Rest der Formel II

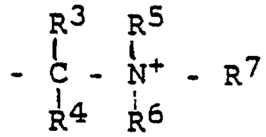

(II)

steht, wobei $R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_4$-Alkyl; Phenyl-$C_1$-$C_2$-Alkyl;

$R^6$ und $R^7$ zusammen mit dem sie verknüpfenden Stickstoffatom einen fünf- bis siebengliedrigen gesättigten Heterozyklus bilden, wobei ein Kohlenstoffatom durch ein Schwefel-, Sauerstoff- oder Stickstoffatom ersetzt sein kann, bedeuten;

$R^6$ Wasserstoff und $R^7$ Carbamimidoyl, $C_1$-$C_6$-Alkylcarbonyl oder den Rest einer natürlich vorkommenden $\alpha$-Aminosäure oder $\gamma$-Aminobuttersäure bedeuten;

$R^5$, $R^6$, $R^7$ unabhängig voneinander $C_1$-$C_4$-Alkyl bedeuten;

$R^2$ für einen Rest der Formel III

$-(CH_2)_n-X$ (III)

steht wobei

n 0, 1 oder 2 bedeutet;

X für Hydroxy; $C_1$-$C_4$-Alkyloxy; Carboxy; Halogenformyl; $C_1$-$C_4$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden $\alpha$-Aminosäure oder $\gamma$-Aminobuttersäure peptidisch verknüpft ist; Aminocarbonyl bedeutet, wobei Amino durch $C_1$-$C_4$-Alkyl mono- oder disubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann;

oder X für eine Gruppe der Formel IV

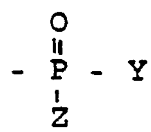

(IV)

steht, wobei

Y und Z unabhängig voneinander Hydroxy, $C_1$-$C_4$-Alkyl oder $C_1$-$C_4$-Alkyloxy bedeuten; und

A für eine C,C-Einfach- oder eine C,C-Doppelbindung steht;

mit der Maßgabe, daß $R^1$ nicht 2-Pyridyl bedeutet, falls $R^2$ für eine Gruppe der Formel IV steht und A $\neq$ einer C,C-Doppelbindung ist, falls X = OH und n = O sind, herstellt.

**4.** Verfahren nach Anspruch 1, dadurch gekennzeichnet, daß man eine Verbindung der Formel Ia, in welcher $R^1$ für 2-Pyridyl oder für einen Rest der Formel II

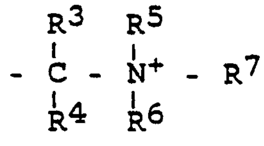

(II)

steht, wobei

$R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_4$-Alkyl; Benzyl;

$R^6$ und $R^7$ zusammen mit dem sie verknüpfenden Stickstoffatom einen fünf- bis sechsgliedrigen

gesättigten Heterozyklus bilden, wobei ein Kohlenstoffatom durch ein Schwefel-, Sauerstoff-oder Stickstoffatom ersetzt sein kann;

$R^6$ Wasserstoff und $R^7$ Carbamimidoyl; $C_1$-$C_6$-Alkylcarbonyl, den Rest einer natürlich vorkommenden $\alpha$-Aminosäure;

$R^5$, $R^6$, $R^7$ unabhängig voneinander $C_1$-$C_4$-Alkyl bedeuten;

$R^2$ für einen Rest der Formel III

$-(CH_2)_n$-X (III)

steht, wobei

n 0, 1 oder 2 bedeutet;

X für Hydroxy; Carboxy; $C_1$-$C_4$-Alkyloxycarbonyl; Benzyloxycarbonyl; Cyclohexyloxycarbonyl, das ein-oder mehrfach durch $C_1$-$C_6$-Alkyl substituiert sein kann; Carbonyl, das mit einer natürlich vorkommenden $\alpha$-Aminosäure peptidisch verknüpft ist; Aminocarbonyl, wobei beide Amino-Reste zusammen mit dem sie verknüpfenden Stickstoffatom einen fünf- bis sechsgliedrigen Heterozyklus bilden, wobei ein Kohlenstoffatom durch ein Sauerstoff- oder Stickstoffatom ersetzt sein kann;
oder für eine Gruppe der Formel IV

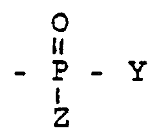

(IV)

steht, wobei

Y Hydroxy oder $C_1$-$C_4$-Alkyloxy und

Z $C_1$-$C_4$-Alkyl bedeuten; und

A für eine C,C-Einfach- oder eine C,C-Doppelbindung steht;

mit der Maßgabe, daß $R^1$ nicht 2-Pyridyl bedeutet, falls $R^2$ für eine Gruppe der Formel IV steht und A ≠ einer C,C-Doppelbindung ist, falls X = OH und n = O sind, herstellt.

5. Verfahren nach Anspruch 1, dadurch gekennzeichnet, daß man eine Verbindung der Formel Ib, in welcher $R^1$ für einen Rest der Formel II

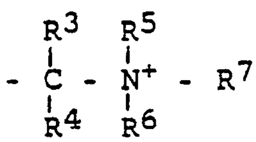

(II)

steht, wobei

$R^3$ und $R^4$ Wasserstoff;

$R^5$ ein freies Elektronenpaar oder Wasserstoff;

$R^6$ und $R^7$ unabhängig voneinander Wasserstoff; $C_1$-$C_4$-Alkyl; Phenyl-$C_1$-$C_2$-Alkyl;

$R^6$ und $R^7$ zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff- oder Stickstoffatom ersetzt sein kann;

$R^6$ Wasserstoff und $R^7$ $C_1$-$C_4$-Acyl; $C_1$-$C_6$-Alkylcarbonyl, Benzoyl oder den Rest einer natürlich vorkommenden $\alpha$-Aminosäure oder $\gamma$-Aminobuttersäure, bedeuten;

$R^5$, $R^6$, $R^7$ unabhängig voneinander $C_1$-$C_4$-Alkyl oder $C_3$-$C_6$-Cycloalkyl bedeuten;

$R^2$ für einen Rest der Formel III

$-(CH_2)_n$-X (III)

steht, wobei

n 0, 1 oder 2 bedeutet;

X für Carboxy; Halogenformyl; $C_1$-$C_4$-Alkyloxycarbonyl; Benzyloxycarbonyl oder $C_3$-$C_6$-

Cycloalkylcarbonyl, die ein- oder mehrfach durch $C_1$-$C_6$-Alkyl substituiert sein können; oder für Carbonyl steht, das mit einer natürlich vorkommenden $\alpha$-Aminosäure oder $\gamma$-Aminobuttersäure peptidisch verknüpft ist; Aminocarbonyl, bedeutet, wobei Amino durch $C_1$-$C_4$-Alkyl mono- oder disubstituiert sein kann, oder beide Amino-Reste zusammen mit dem sie verknüpfenden Stickstoffatom einen fünf- bis sechsgliedrigen Heterozyklus bilden, wobei ein Kohlenstoffatom durch ein Sauerstoff- oder Stickstoffatom ersetzt sein kann; und

A für eine C,C-Einfach- oder eine C,C-Doppelbindung steht, herstellt.

6. Verfahren nach Anspruch 1, dadurch gekennzeichnet, daß man eine Verbindung der Formel Ib, in welcher $R^1$ für einen Rest der Formel II

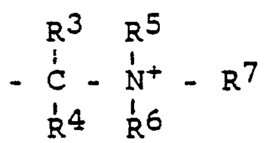

(II)

steht, wobei

| | |
|---|---|
| $R^3$ und $R^4$ | Wasserstoff; |
| $R^5$ | ein freies Elektronenpaar; |
| $R^6$ und $R^7$ | unabhängig voneinander Wasserstoff oder $C_1$-$C_4$-Alkyl; |
| $R^6$ und $R^7$ | zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff-oder Stickstoffatom ersetzt sein kann; |
| $R^6$ | Wasserstoff und $R^7$ $C_1$-$C_6$-Alkylcarbonyl bedeuten; |
| $R^5$, $R^6$, $R^7$ | unabhängig voneinander $C_1$-$C_4$-Alkyl bedeuten; |
| $R^2$ | für einen Rest der Formel III |

$-(CH_2)_n-X$ (III)

steht, wobei

| | |
|---|---|
| n | 2 bedeutet und |
| X | für Carboxy, $C_1$-$C_4$-Alkyloxycarbonyl; Benzyloxycarbonyl; Cyclohexylcarbonyl, das ein- oder mehrfach durch $C_1$-$C_6$-Alkyl substituiert sein kann; Carbonyl, das mit einer natürlich vorkommenden $\alpha$-Aminosäure peptidisch verknüpft ist; Aminocarbonyl, wobei beide Amino-Reste zusammen mit dem sie verknüpfenden Stickstoffatom einen fünf- bis sechsgliedrigen Heterozyklus bilden, wobei ein Kohlenstoffatom durch ein Sauerstoff-oder Stickstoffatom ersetzt sein kann; und |
| A | für eine C,C-Einfach- oder eine C,C-Doppelbindung steht, herstellt. |

7. Verfahren nach Anspruch 1, dadurch gekennzeichnet, daß man eine Verbindung der Formel Ia, in welcher A für eine C,C-Doppelbindung steht, herstellt.

8. Verfahren nach Anspruch 1, dadurch gekennzeichnet, daß man eine Verbindung der Formel Ia oder Ib aus der Reihe
5-Aminomethyl-isoxazol-3-propionsäure-benzylester-Hydrochlorid,
5-Aminomethyl-isoxazol-3-propionsäure- ethylester-Hydrochlorid,
5-Aminomethyl-isoxazol-3-propionsäure-(+)-menthylester-Toluol-4-sulfonat,
5-Aminomethyl-isoxazol-3-propionsäure-(-)-menthylester-Toluol-4-sulfonat,
5-Aminomethyl-isoxazol-3-propionsäure-cis-(3,3,5)-trimethylcyclohexylester-Toluol-4-sulfonat,
2-(5-Aminomethyl-isoxazol-3-yl)-ethyl-2-(P-methyl)-phosphinsäure-ethylester-Hydrochlorid,
3-Carboxy-2-isoxazolin-5-yl-carbonsäure-(-)-menthylester-Dicyclohexylammoniumsalz,
5-Trimethylammonio-methyl-isoxazol-3-propionsäure-cis-(3,3,5)-trimethylcyclohexylester-iodid
3-Hydroxyiminomethyl-isoxazol-5-propionsäure-methylester, 3,5-Dicarboxy-2-isoxazolin,
5-Hydroxymethyl-isoxazol-3-propionsäure-Natriumsalz,
5-Aminomethyl-isoxazol-3-propionsäure-methylester-Hydrochlorid,
5-Aminomethyl-isoxazol-3-propionsäure,

5-(1-Amino-1-methyl-ethyl)-isoxazol-3-propionsäure,
5-Benzylaminomethyl-isoxazol-3-propionsäure,
5-Dimethylaminomethyl-isoxazol-3-propionsäure,
2-(5-Aminomethyl-isoxazol-3-yl)-ethyl-2-(P-methyl)-phosphinsäure-Hydrochlorid,
5-Acetamidomethyl-isoxazol-3-propionsäure,
5-Trimethylammoniomethyl-isoxazol-3-propionsäure-(-)-menthylester-Jodid,
5-Trimethylammoniomethyl-isoxazol-3-propionsäure-(+)-menthylester-Jodid,
5-(L-Phenylalanyl-amino)-methyl-isoxazol-3-propionsäure-(+)-menthylester-Hydrochlorid,
5-(L-Phenylalanyl-amino)-methyl-isoxazol-3-propionsäure-(-)-methylester-Hydrochlorid,
5-(L-Phenylalanyl-amino)-methyl-isoxazol-3-propionsäure-methylester-Hydrochlorid,
5-Guanidinomethyl-isoxazol-3-propionsäure,
N-(5-Aminomethyl-isoxazol-3-yl)-propionyl-glycin,
Bis-(3-[2-carboxy]-ethyl-isoxazol-5-yl-methyl)-amin-Diammoniumsalz,
5-Aminomethyl-2-isoxazolin-3-propionsäure-(+)-menthylester-Toluol-4-sulfonat,
5-Aminomethyl-2-isoxazolin-3-propionsäure-(-)-menthylester-Toluol-4-sulfonat,
3-(2-Carboxyethyl)-5-(2-pyridyl)-2-isoxazolin,
5-Trimethylammoniomethyl-isoxazol-3-propionsäureester,
5-Piperidinomethyl-isoxazol-3-propionsäure-Hydrochlorid,
5-Aminomethyl-isoxazol-3-propionsäureamid-Hydrochlorid,
5-(L-Phenylalanyl-amino)-methyl-isoxazol-3-yl-propionylglycin-Trifluoracetat, herstellt.

**9.** Verfahren zur Herstellung eines Arzneimittels, dadurch gekennzeichnet, daß man mindestens einer Verbindung der Formel Ia, Ib oder Ic oder gegebenenfalls einem von deren physiologisch verträglichen Salzen oder in stereoisomerenreiner Form.

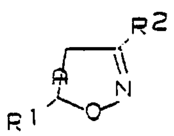 (Ia) 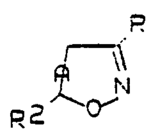 (Ib) 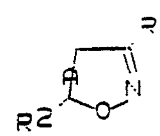 (Ic)

in welcher

$R^1$ für 2-, 3-, 4-Pyridyl oder einen Rest der Formel II

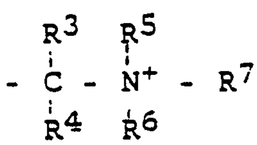 (II)

steht, wobei

$R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_6$-Alkyl; $C_3$-$C_6$-Cycloalkyl; $C_6$-$C_{12}$-Aryl-$C_1$-$C_4$-Alkyl; Carbamimidoyl; $C_1$-$C_6$-Alkylcarbonyl, $C_1$-$C_4$-Alkenylcarbonyl, $C_1$-$C_6$-Alkyloxycarbonyl, $C_6$-$C_{12}$-Aryl-$C_1$-$C_4$-Alkylcarbonyl, $C_6$-$C_{10}$-Aryl-$C_1$-$C_4$-Alkyloxycarbonyl, $C_6$-$C_{10}$-Arylcarbonyl, den Rest einer natürlich vorkommenden $\alpha$-Aminosäure oder $\gamma$-Aminobuttersäure, die durch $C_1$-$C_6$-Alkyl, Hydroxy, Halogen, Amino oder Nitro substituiert sein können; oder

$R^6$ und $R^7$ zusammen mit dem sie verknüpfenden Stickstoffatom einen fünf- bis siebengliedrigen Heterozyklus bilden, wobei ein Kohlenstoffatom durch ein Schwefel-, Sauerstoff- oder Stickstoffatom ersetzt sein kann, bedeuten; oder

$R^5$, $R^6$, $R^7$ unabhängig voneinander $C_1$-$C_4$-Alkyl oder $C_3$-$C_6$-Cycloalkyl bedeuten, oder $R^5$ $C_1$-$C_4$-Alkyl bedeutet und $R^6$ und $R^7$ zusammen mit dem sie verknüpfenden Stickstoffatom einen fünf- bis siebengliedrigen Heterozyklus bilden, oder $R^5$, $R^6$ und $R^7$ zusammen mit dem sie verknüpfenden Stickstoffatom einen sechs- bis zwölfgliedrigen Heterozyklus bilden;

$R^2$ für einen Rest der Formel III

$-(CH_2)_n-X$ (III)

steht, wobei

n 0 oder eine ganze Zahl von 1 bis 4 bedeutet;

X für Hydroxy; $C_1$-$C_4$-Alkyloxy; Carboxy; Halogenformyl; Formyl; Oxyimino; $C_1$-$C_{12}$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden α-Aminosäure, γ-Aminobuttersäure oder einem Dipeptid peptidisch verknüpft ist, oder Aminocarbonyl bedeutet, wobei Amino durch $C_1$-$C_6$-Alkyl mono- oder disubstituiert oder durch Phenyl-$C_1$-$C_6$-Alkyl monosubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann;
oder X für eine Gruppe der Formel IV

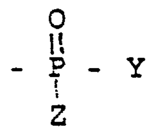

(IV)

steht, wobei

Y und Z unabhängig voneinander Hydroxy, $C_1$-$C_4$-Alkyl oder $C_1$-$C_4$-Alkyloxy bedeuten; und

A für eine C,C-Einfach- oder eine C,C-Doppelbindung steht;

mit den üblichen Trägerstoffen sowie gegebenenfalls Zusatz- und Hilfsstoffen in eine geeignete Darreichungsform bringt.

**10.** Verfahren nach Anspruch 9, dadurch gekennzeichnet, daß man eine Verbindung der Formel Ia oder Ib, in welcher $R^1$ für 2-, 3-, 4-Pyridyl oder für einen Rest der Formel II

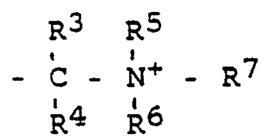

(II)

steht, wobei $R^3$ und $R^4$ unabhängig voneinander Wasserstoff oder $C_1$-$C_4$-Alkyl;

$R^5$ ein freies Elektronenpaar oder Wasserstoff;

$R^6$ und $R^7$ unabhängig voneinander Wasserstoff; $C_1$-$C_4$-Alkyl; Phenyl-$C_1$-$C_2$-Alkyl;

$R^6$ und $R^7$ zusammen mit dem sie verknüpfenden Stickstoffatom einen fünf- bis siebengliedrigen Heterozyklus bilden, wobei ein Kohlenstoffatom durch ein Schwefel-, Sauerstoff- oder Stickstoffatom ersetzt sein kann, bedeuten;

$R^6$ Wasserstoff und $R^7$ Carbamimidoyl; $C_1$-$C_6$-Alkylcarbonyl, $C_1$-$C_4$-Alkenylcarbonyl, $C_1$-$C_6$-Alkyloxycarbonyl, Phenyl-$C_1$-$C_4$-alkylcarbonyl, Benzyloxycarbonyl, Benzoyl, den Rest einer natürlich vorkommenden α-Aminosäure oder γ-Aminobuttersäure, die durch $C_1$-$C_4$-Alkyl, Hydroxy, Halogen, Amino oder Nitro substituiert sein können;

$R^5$, $R^6$, $R^7$ unabhängig voneinander $C_1$-$C_4$-Alkyl oder $C_3$-$C_6$-Cycloalkyl bedeuten;

$R^2$ für einen Rest der Formel III

$- (CH_2)_n - X$ (III)

steht wobei

n 0 oder eine ganze Zahl von 1 bis 3 beduetet;

X für Hydroxy; $C_1$-$C_4$-Alkyloxy; Carboxy; $C_1$-$C_4$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden α-Aminosäure, γ-Aminobuttersäure oder einem Dipeptid peptidisch verknüpft ist; Aminocarbonyl bedeutet, wobei Amino durch $C_1$-$C_4$-Alkyl mono- oder disubstituiert oder durch Phenyl-$C_1$-$C_4$-Alkyl monosubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff-oder Stickstoffatom ersetzt sein kann;
oder X für eine Gruppe der Formel IV

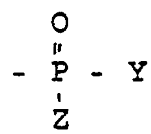 (IV)

steht, wobei

| | |
|---|---|
| Y und Z | unabhängig voneinander Hydroxy, $C_1$-$C_4$-Alkyl oder $C_1$-$C_4$-Alkyloxy bedeuten; und |
| A | für eine C,C-Einfach- oder eine C,C-Doppelbindung steht, verwendet. |

11. Verfahren nach Anspruch 9, dadurch gekennzeichnet, daß man eine Verbindung der Formel Ia, in welcher $R^1$ für 2-Pyridyl oder für einen Rest der Formel II

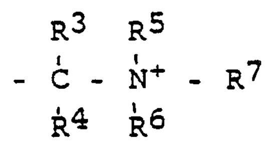 (II)

steht, wobei $R^3$ und $R^4$ unabhängig voneinander Wasserstoff oder $C_1$-$C_4$-Alkyl;

| | |
|---|---|
| $R^5$ | ein freies Elektronenpaar oder Wasserstoff; |
| $R^6$ und $R^7$ | unabhängig voneinander Wasserstoff; $C_1$-$C_4$-Alkyl; Phenyl-$C_1$-$C_2$-Alkyl; |
| $R^6$ und $R^7$ | zusammen mit dem sie verknüpfenden Stickstoffatom einen fünf- bis siebengliedrigen gesättigten Heterozyklus bilden, wobei ein Kohlenstoffatom durch ein Schwefel-, Sauerstoff- oder Stickstoffatom ersetzt sein kann, bedeuten; |
| $R^6$ | Wasserstoff und $R^7$ Carbamimidoyl, $C_1$-$C_6$-Alkylcarbonyl oder den Rest einer natürlich vorkommenden $\alpha$-Aminosäure oder $\gamma$-Aminobuttersäure bedeuten; |
| $R^5$, $R^6$, $R^7$ | unabhängig voneinander $C_1$-$C_4$-Alkyl bedeuten; $R^2$ für einen Rest der Formel III |

$-(CH_2)_n-X$ (III)

steht wobei

| | |
|---|---|
| n | 0, 1 oder 2 bedeutet; |
| X | für Hydroxy; $C_1$-$C_4$-Alkyloxy; Carboxy; Halogenformyl; $C_1$-$C_4$-Alkyloxycarbonyl; Benzyloxycarbonyl oder $C_3$-$C_6$-Cycloalkyloxycarbonyl, die ein- oder mehrfach durch $C_1$-$C_6$-Alkyl substituiert sein können; oder für Carbonyl stehen, das mit einer natürlich vorkommenden $\alpha$-Aminosäure oder $\gamma$-Aminobuttersäure peptidisch verknüpft ist; Aminocarbonyl bedeutet, wobei Amino durch $C_1$-$C_4$-Alkyl mono- oder disubstituiert sein kann, oder beide Amino-Reste zusammen mit dem sie verknüpfenden Stickstoffatom einen fünf- bis siebengliedrigen Heterozyklus bilden, wobei ein Kohlenstoffatom durch ein Sauerstoff- oder Stickstoffatom ersetzt sein kann; |

oder X für eine Gruppe der Formel IV

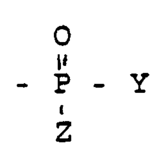 (IV)

steht, wobei

Y und Z unabhängig voneinander Hydroxy, $C_1$-$C_4$-Alkyl oder $C_1$-$C_4$-Alkyloxy bedeuten; und
A für eine C,C-Einfach- oder eine C,C-Doppelbindung steht, verwendet.

**12.** Verfahren nach Anspruch 9, dadurch gekennzeichnet, daß man eine Verbindung der Formel Ia, in welcher $R^1$ für 2-Pyridyl oder für einen Rest der Formel II

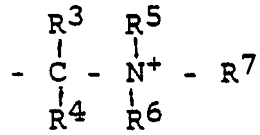

(II)

steht, wobei
$R^3$ und $R^4$ unabhängig voneinander Wasserstoff oder $C_1$-$C_4$-Alkyl;
$R^5$ ein freies Elektronenpaar oder Wasserstoff;
$R^6$ und $R^7$ unabhängig voneinander Wasserstoff; $C_1$-$C_4$-Alkyl; Benzyl;
$R^6$ und $R^7$ zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff-oder Stickstoffatom ersetzt sein kann;
$R^6$ Wasserstoff und $R^7$ Carbamimidoyl; $C_1$-$C_6$-Alkylcarbonyl,oder den Rest einer natürlich vorkommenden α-Aminosäure;
$R^5$, $R^6$, $R^7$ unabhängig voneinander $C_1$-$C_4$-Alkyl bedeuten;
$R^2$ für einen Rest der Formel III

$-(CH_2)_n-X$ (III)

steht, wobei
n 0, 1 oder 2 bedeutet;
X für Hydroxy; Carboxy; $C_1$-$C_4$-Alkyloxycarbonyl; Benzyloxycarbonyl; Cyclohexyloxycarbonyl, das ein-oder mehrfach durch $C_1$-$C_6$-Alkyl substituiert sein kann; Carbonyl, das mit einer natürlich vorkommenden α-Aminosäure peptidisch verknüpft ist; Aminocarbonyl, wobei beide Amino-Reste zusammen mit dem sie verknüpfenden Stickstoffatom einen fünf- bis sechsgliedrigen Heterozyklus bilden, wobei ein Kohlenstoffatom durch ein Sauerstoff- oder Stickstoffatom ersetzt sein kann;
oder X für eine Gruppe der Formel IV

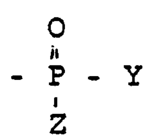

(IV)

steht, wobei
Y Hydroxy oder $C_1$-$C_4$-Alkyloxy und
Z $C_1$-$C_4$-Alkyl bedeuten, und
A für eine C,C-Einfach- oder eine C,C-Doppelbindung steht, verwendet.

**13.** Verfahren nach Anspruch 9, dadurch gekennzeichnet, daß man eine Verbindung der Formel Ib, in welcher $R^1$ für einen Rest der Formel II

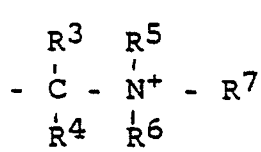

(II)

steht, wobei

$R^3$ und $R^4$ Wasserstoff;
$R^5$ ein freies Elektronenpaar oder Wasserstoff;
$R^6$ und $R^7$ unabhängig voneinander Wasserstoff; $C_1$-$C_4$-Alkyl; Phenyl-$C_1$-$C_2$-Alkyl;
$R^6$ und $R^7$ zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff- oder Stickstoffatom ersetzt sein kann;
$R^6$ Wasserstoff und $R^7$ $C_1$-$C_4$-Acyl; $C_1$-$C_6$-Alkylcarbonyl, Benzoyl oder den Rest einer natürlich vorkommenden $\alpha$-Aminosäure oder $\gamma$-Aminobuttersäure, bedeuten;
$R^5$, $R^6$, $R^7$ unabhängig voneinander $C_1$-$C_4$-Alkyl oder $C_3$-$C_6$-Cycloalkyl bedeuten;
$R^2$ für einen Rest der Formel III

$-(CH_2)_n-X$ (III)

steht, wobei
n 0, 1 oder 2 bedeutet;
X für Carboxy; Halogenformyl; $C_1$-$C_4$-Alkyloxycarbonyl; Benzyloxycarbonyl oder $C_3$-$C_6$-Cycloalkylcarbonyl, die ein- oder mehrfach durch $C_1$-$C_6$-Alkyl substituiert sein können; oder für Carbonyl steht, das mit einer natürlich vorkommenden $\alpha$-Aminosäure oder $\gamma$-Aminobuttersäure peptidisch verknüpft ist; Aminocarbonyl, bedeutet, wobei Amino durch $C_1$-$C_4$-Alkyl mono- oder disubstituiert sein kann, oder beide Amino-Reste zusammen mit dem sie verknüpfenden Stickstoffatom einen fünf- bis sechsgliedrigen Heterozyklus bilden, wobei ein Kohlenstoffatom durch ein Sauerstoff- oder Stickstoffatom ersetzt sein kann; und
A für eine C,C-Einfach- oder eine C,C-Doppelbindung steht, verwendet.

14. Verfahren nach Anspruch 9, dadurch gekennzeichnet, daß man eine Verbindung der Formel Ib, in welcher $R^1$ für einen Rest der Formel II

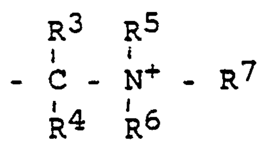 (II)

steht, wobei
$R^3$ und $R^4$ Wasserstoff;
$R^5$ ein freies Elektronenpaar;
$R^6$ und $R^7$ unabhängig voneinander Wasserstoff oder $C_1$-$C_4$-Alkyl;
$R^6$ und $R^7$ zusammen mit dem sie verknüpfenden Stickstoffatom einen fünf- bis sechsgliedrigen gesättigten Heterozyklus bilden, wobei ein Kohlenstoffatom durch ein Schwefel-, Sauerstoff-oder Stickstoffatom ersetzt sein kann;
$R^6$ Wasserstoff und $R^7$ $C_1$-$C_6$-Alkylcarbonyl bedeuten;
$R^5$, $R^6$, $R^7$ unabhängig voneinander $C_1$-$C_4$-Alkyl bedeuten;
$R^2$ für einen Rest der Formel III

$-(CH_2)_n-X$ (III)

steht, wobei
n 2 bedeutet und
X für Carboxy, $C_1$-$C_4$-Alkyloxycarbonyl; Benzyloxycarbonyl; Cyclohexylcarbonyl, das ein- oder mehrfach durch $C_1$-$C_6$-Alkyl substituiert sein kann; Carbonyl, das mit einer natürlich vorkommenden $\alpha$-Aminosäure peptidisch verknüpft ist; Aminocarbonyl, wobei beide Amino-Reste zusammen mit dem sie verknüpfenden Stickstoffatom einen fünf- bis sechsgliedrigen Heterozyklus bilden, wobei ein Kohlenstoffatom durch ein Sauerstoff-oder Stickstoffatom ersetzt sein kann; und
A für eine C,C-Einfach- oder eine C,C-Doppelbindung steht, verwendet.

15. Verfahren nach Anspruch 9, dadurch gekennzeichnet, daß man eine Verbindung der Formel Ia, in

welcher A für eine C,C-Doppelbindung steht, verwendet.

**16.** Verfahren nach Anspruch 9, dadurch gekennzeichnet, daß man eine Verbindung der Formel Ia, Ib oder Ic aus der Reihe
5-Aminomethyl-isoxazol- 3-propionsäure-benzylester-Hydrochlorid,
5-Aminomethyl-isoxazol-3-propionsäure-ethylester-Hydrochlorid,
5-Aminomethyl-isoxazol-3-propionsäure-(+)-menthylester-Toluol-4-sulfonat,
5-Aminomethyl-isoxazol-3-propionsäure-(-)-menthylester-Toluol-4-sulfonat,
5-Aminomethyl-isoxazol-3-propionsäure-cis-(3,3,5)-trimethylcyclohexylester-Toluol-4-sulfonat,
2-(5-Aminomethyl-isoxazol-3-yl)-ethyl-2-(P-methyl)-phosphinsäure-ethylester-Hydrochlorid,
3-Carboxy-2-isoxazolin-5-yl-carbonsäure-(-)-menthylester-Dicyclohexylammoniumsalz,
5-Trimethylammonio-methyl-isoxazol-3-propionsäure-cis-(3,3,5)-trimethylcyclohexylester-iodid
3-Hydroxyiminomethyl-isoxazol-5-propionsäure-methylester, 3,5-Dicarboxy-2-isoxazolin,
5-Hydroxymethyl-isoxazol-3-propionsäure-Natriumsalz,
5-Aminomethyl-isoxazol-3-propionsäure-methylester-Hydrochlorid,
5-Aminomethyl-isoxazol-3-propionsäure,
5-(1-Amino-1-methyl-ethyl)-isoxazol-3-propionsäure,
5-Benzylaminomethyl-isoxazol-3-propionsäure,
5-Dimethylaminomethyl-isoxazol-3-propionsäure,
2-(5-Aminomethyl-isoxazol-3-yl)-ethyl-2-(P-methyl)-phosphinsäure-Hydrochlorid,
5-Acetamidomethyl-isoxazol-3-propionsäure,
5-Trimethylammoniomethyl-isoxazol-3-propionsäure-(-)-menthylester-Jodid,
5-Trimethylammoniomethyl-isoxazol-3-propionsäure-(+)-menthylester-Jodid,
5-(L-Phenylalanyl-amino)-methyl-isoxazol-3-propionsäure-(+)-menthylester-Hydrochlorid,
5-(L-Phenylalanyl-amino)-methyl-isoxazol-3-propionsäure-(-)-menthylester-Hydrochlorid,
5-(L-Phenylalanyl-amino)-methyl-isoxazol-3-propionsäure-methylester-Hydrochlorid,
5-Guanidinomethyl-isoxazol-3-propionsäure,
N-(5-Aminomethyl-isoxazol-3-yl)-propionyl-glycin,
Bis-(3-[2-carboxy]-ethyl-isoxazol-5-yl-methyl)-amin-Diammoniumsalz,
5-Aminomethyl-2-isoxazolin-3-propionsäure-(+)-menthylester-Toluol-4-sulfonat,
5-Aminomethyl-2-isoxazolin-3-propionsäure-(-)-menthylester-Toluol-4-sulfonat,
3-(2-Carboxyethyl)-5-(2-pyridyl)-2-isoxazolin,
5-Trimethylammoniomethyl-isoxazol-3-propionsäureester,
5-Piperidinomethyl-isoxazol-3-propionsäure-Hydrochlorid,
5-Aminomethyl-isoxazol-3-propionsäureamid-Hydrochlorid,
5-(L-Phenylalanyl-amino)-methyl-isoxazol-3-yl-propionylglycin-Trifluoracetat, verwendet.

**17.** Verwendung von mindestens einem Arzneimittel gemäß den Ansprüchen 9 bis 16 zur Prophylaxe oder Behandlung von pathologischen neurodegenerativen Erkrankungen.

**18.** Verwendung von mindestens einer Verbindung der Formel Ia, Ib oder Ic, ihre gegebenenfalls stereosiomere Form und/oder gegebenenfalls ihr physiologisch verträgliches Salz gemäß den Ansprüchen 9 bis 16 zur Anwendung in einem Verfahren zur prophylaktischen und/oder therapeutischen Behandlung von pathologischen neurodegenerativen Erkrankungen des menschlichen und tierischen Körpers.

**19.** Verwendung einer Verbindung der Formel Ia, Ib oder Ic ihre gegebenenfalls stereoisomere Form und/oder gegebenenfalls ihr physiologisch verträgliches Salz zur Herstellung eines Arzneimittels gemäß den Ansprüchen 9 bis 16.

Europäisches Patentamt

# EUROPÄISCHER RECHERCHENBERICHT

Nummer der Anmeldung

EP 91 10 5614

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 313 997 (HOECHST AKTIENGESELLSCHAFT) * Seite 57, Zeile 50 - Seite 58, Zeile 40; Anspruch 1 & DE-A-3736113 * | 1,10 | C 07 D 261/08<br>C 07 D 261/18<br>C 07 D 261/04<br>C 07 F 9/30<br>C 07 F 9/32<br>C 07 F 9/38<br>C 07 F 9/40<br>A 61 K 31/42 |
| A | WO-A-8 809 330 (ZAMBON GROUP S.P.A) * Seiten 1 - 5, Zeile 28 * | 1,10 | |
| A | GB-A-2 163 746 (F.HOFFMANN-LAROCHE & CO AKTIENGESELLSCHAFT) * Seiten 1 - 2, Zeile 58 * | 1,10 | |
| A,D | Journal of Medicinal Chemistry vol. 28, no. 5, 1985, Columbus Ohio Seiten 668 - 672; J.Lauridsen et al.: "Ibotenic Acid Analogues.Synthesis,Molecular Flexibility,and in Vitro Activity of Agonists and Antagonists at Central Glutamic Acid Receptors" * das ganze Dokument * | 1,10 | |
| A | CHEMICAL ABSTRACTS, vol. 112, no. 5, 29 Januar 1990 Columbus, Ohio, USA De Amici Marco et al.: "Synthesis and pharmacological investigation of cholinergic ligands structurally related to muscarone." & Eur.J.Med.Chem.1989,vol.24,no 2,171-7 Seite 568; Spalte 1; ref. no. 35731X * Zusammenfassung * | 1,10 | **RECHERCHIERTE SACHGEBIETE (Int. Cl.5)**<br>C 07 D 261/00<br>A 61 K 31/00<br>C 07 F |

Der vorliegende Recherchenbericht wurde für alle Patentansprüche erstellt

| Recherchenort | Abschlußdatum der Recherche | Prüfer |
|---|---|---|
| Berlin | 18 Juli 91 | KYRIAKAKOU G |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument